(19) Europäisches Patentamt
European Patent Office
Office européen des brevets

(11) EP 2 184 272 A1

(12) **EUROPEAN PATENT APPLICATION**
published in accordance with Art. 153(4) EPC

(43) Date of publication:
**12.05.2010 Bulletin 2010/19**

(21) Application number: **08827984.9**

(22) Date of filing: **19.08.2008**

(51) Int Cl.:
*C07D 209/34* (2006.01) *A61K 31/496* (2006.01)
*A61P 9/10* (2006.01) *A61P 25/02* (2006.01)
*A61P 25/04* (2006.01) *A61P 25/08* (2006.01)
*A61P 25/14* (2006.01) *A61P 25/16* (2006.01)
*A61P 25/28* (2006.01) *A61P 43/00* (2006.01)
*C07D 215/22* (2006.01) *C07D 235/26* (2006.01)
*C07D 263/58* (2006.01) *C07D 401/12* (2006.01)
*C07D 401/14* (2006.01) *C07D 403/12* (2006.01)
*C07D 413/12* (2006.01) *C07D 413/14* (2006.01)
*C07D 487/08* (2006.01) *C07D 498/04* (2006.01)

(86) International application number:
**PCT/JP2008/064731**

(87) International publication number:
**WO 2009/025265 (26.02.2009 Gazette 2009/09)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **21.08.2007 JP 2007214732**

(71) Applicant: **Shionogi&Co., Ltd.**
**Osaka-shi, Osaka 5410045 (JP)**

(72) Inventors:
- **MASUI, Moriyasu**
**Osaka-shi**
**Osaka 553-0002 (JP)**
- **TOMIDA, Minoru**
**Koka-shi**
**Shiga 520-3423 (JP)**
- **KOBAYASHI, Naotake**
**Osaka-shi**
**Osaka 553-0002 (JP)**
- **ANAN, Kousuke**
**Osaka-shi**
**Osaka 553-0002 (JP)**
- **TAZAWA, Aya**
**Osaka-shi**
**Osaka 553-0002 (JP)**
- **HATA, Kayoko**
**Osaka-shi**
**Osaka 553-0002 (JP)**

(74) Representative: **HOFFMANN EITLE**
**Patent- und Rechtsanwälte**
**Arabellastrasse 4**
**D-81925 München (DE)**

(54) **PIPERAZINE DERIVATIVE**

(57) A compound which specifically binds to a receptor of NR1/ NR2B, and is used as a NR2B receptor antagonist is provided.

It has been found out that a piperazine derivative represented by the formula (I) binds specifically to a receptor of NR1/NR2B, and is used as a NR2B receptor antagonist.

A compound represented by:

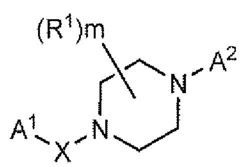

(I)

wherein $R^1$ is each independently C1-C3 alkyl or the like, m is an integer of 0 to 4, X is $-N(R^4)-C(=O)-C(=O)-$, $-N(R^4)-(CR^5R^6)_p-C(=O)-$, $-N(R^4)-C(-O)-(CR^7R^8)_q-$ or $-C(=O)-N(R^4)-(CR^7R^8)_q-$, p and q are each independently an integer of 1 to 3, $R^4$, $R^5$, $R^6$, $R^7$ and $R^8$ are each independently a hydrogen atom or lower alkyl, $A^1$ is ben-

EP 2 184 272 A1

zoxazolinone or the like, and $A^2$ is optionally substituted phenyl or the like, or a pharmaceutically acceptable salt or a solvate thereof.

## Description

[Technical field]

The present invention relates to a nitrogen-containing heterocyclic derivative which exhibits specific antagonism for a glutamic acid receptor of a central nervous cell, particularly, a NR1/NR2B receptor which is one kind of NMDA receptors, preferably, is useful as a drug such as an analgesic and the like having little side effect on motor function (e.g., paresis), and mental symptom (e.g., psychological fragmentation).

[Background Art]

An amino acid such as L-glutamic acid, and L-aspartic acid is important as a neurotransmitter in a central nervous system for activating a nervous cell. However, it is thought that excessive extracellular accumulation of these excitatory amino acids causes various cerebro-neurological diseases such as Parkinson's disease, senile dementia, Huntington's disease, and epilepsia, as well as defect in mental and motor functions as those seen at anoxia, ischemia, hypoglycemic state, and head or spinal cord damage.
It is known that the activity of excitatory amino acids on a central nervous cell acts via a glutamic acid receptor present on a nerve cell, and it is thought that a glutamic acid receptor antagonist is useful as a therapeutic agent for the diseases and symptoms, for example, an anti-epileptic drug, an ischemic brain damage preventing drug, and an anti-Parkinson's disease drug.
The NMDA receptor which is one kind of glutamic acid receptors is composed of two subunits of NR1 and NR2, and there are further four kinds (NR2A, 2B, 2C, 2D) of subfamilies in the NR2 subunit. It is said that the NR1/NR2A receptor is involved exclusively in memory formation and learning acquisition, and the NR1/NR2B receptor is involved in neurodegenerative cell death and transmission of a pain at brain ischemia. Therefore, there is a high possibility that a drug exhibiting high affinity for the NR1/NR2B receptor becomes an effective analgesic having little side effect.
In addition, compounds similar to the present compound are described in Patent Documents 1 to 16 and Non-patent Document 1, but none of compounds related to the present invention are described.
[Patent Document 1]
International Publication WO 02/068409
[Patent Document 2]
International Publication WO 02/080928
[Patent Document 3]
International Publication WO 02/40466
[Patent Document 4]
Japanese Patent Application Laid-Open (JP-A) No.11-147872
[Patent Document 5]
International Publication WO 2003/076420
[Patent Document 6]
International Publication WO 2003/010159
[Patent Document 7]
International Publication WO 2006/010968
[Patent Document 8]
International Publication WO 2006/010964
[Patent Document 9]
International Publication WO 2003/053366
[Patent Document 10]
International Publication WO 2002/051806
[Patent Document 11]
International Publication WO 86/00899
[Patent Document 12]
Switzerland Patent Application Publication CH460016
[Patent Document 13]
Switzerland Patent Application Publication CH460017
[Patent Document 14]
US Patent No.3538089
[Patent Document 15]

JP-A No.56-49363
[Patent Document 16]
JP-ANo.56-49364
[Non-patent Document 1]
Journal of Heterocyclic Chemistry, 1995, vol.32, No.1, pp.1-11

[Disclosure of the invention]

[Problems to be solved by the invention]

A NMDA receptor antagonist which is highly active, more preferably exhibits high affinity for a subtype, particularly, a NR1/NR2B receptor, particularly an analgesic for a cancer pain and the like is provided.

[Means to solved the problems]

The present invention provides the following.

1) A compound represented by the formula (I):

[Chemical formula 1]

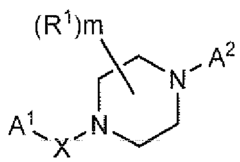

(I)

wherein
$R^1$ is each independently C1-C3 alkyl, halo C1-C3 alkyl, C1-C3 alkoxy, halo C1-C3 alkoxy, hydroxyl, or amino,
two of $R^1$ may be bound to the same carbon atom to form oxo, or
$R^1$ may be bound to different two carbon atoms which are not adjacent to form $-(CH_2)r-$,
m is an integer of 0 to4,
r is an integer of 1 or 2,
X is $-N(R^4)-C(=O)-C(=O)-$, $-N(R^4)-(CR^5R^6)_p-C(=O)-$, $-N(R^4)-C(=O)-(CR^7R^8)_q-$ or $-C(=O)-N(R^4)-(CR^7R^8)_q-$,
p and q are each independently an integer of 1 to 3,
$R^4$, $R^5$, $R^6$, $R^7$ and $R^8$ are each independently a hydrogen atom or a lower alkyl,
$A^1$ is a group represented by the formula:
[Chemical formula 2]

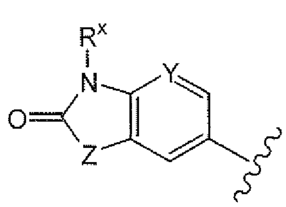

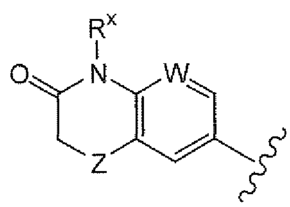

or

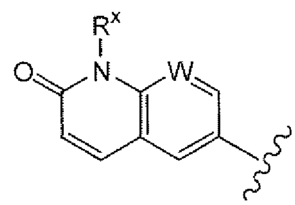

wherein Y and W are each independently CH or N,
Z is an oxygen atom, a sulfur atom, $CH_2$ or $N(-CH_3)$,
$R^X$ is a hydrogen atom, optionally substituted lower alkyl, acyl, lower alkyloxycarbonyl, optionally substituted aralkyloxycarbonyl, lower alkylsulfonyl, arylsulfonyl optionally substituted with lower alkyl, or carbamoyl optionally substituted with

lower alkyl
carbon atoms constituting a ring in the group may be substituted with halogen,
$A^2$ is a group represented by the formula:

[Chemical formula 3]

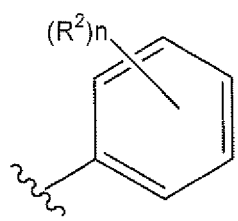

or

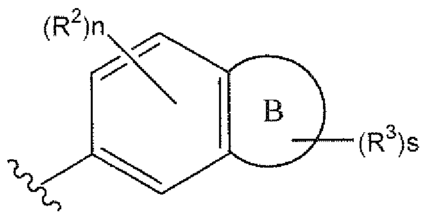

wherein,
ring B is a non-aromatic carbocycle, a non-aromatic heterocycle, an aromatic carbocycle, or an aromatic heterocycle,
$R^2$ and $R^3$ are each independently halogen; cyano; hydroxy; acyl; acylamino; amino optionally substituted with lower alkyl; optionally substituted lower alkyl; lower alkyloxy; lower alkylsulfonyl; aryl optionally substituted with halogen and/or lower alkyl; heteroaryl optionally substituted with halogen and/or lower alkyl; or aralkyl optionally substituted with halogen and/or lower alkyl; or two of $R^3$ may be substituted at the same carbon atom to form oxo,
n and s are each independently an integer of 0 to 3"
provided that when W is CH, X is not $-N(R^4)-C(=O)-(CR^7R^8)_2-$, or a pharmaceutically acceptable salt thereof, or a solvate thereof. 2) The compound according to 1), wherein two of $R^1$ is bound to the same carbon atom to form oxo, or $R^1$ is bound to different two carbon atoms which are not adjacent to form $-CH_2-$ or $-(CH_2)_2-$, or a pharmaceutically acceptable salt thereof, or a solvate thereof.
3) The compound according to 1) or 2), wherein $A^2$ is a group represented by the formula:

[Chemical formula 4]

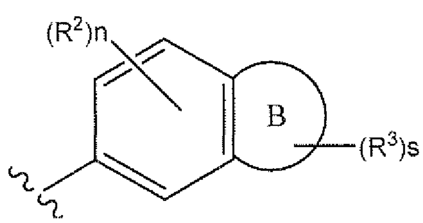

wherein
ring B is a non-aromatic carbocycle, or a non-aromatic heterocycle,
$R^2$, $R^8$, n and s are as defined in 1), or a pharmaceutically acceptable salt thereof, or a solvate thereof.
4) The compound according to 1) or 2), wherein $A^2$ is a group represented by the formula:

[Chemical formula 5]

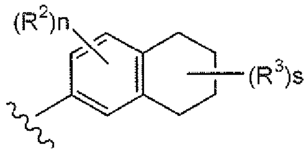

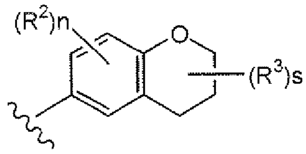

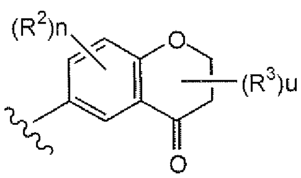

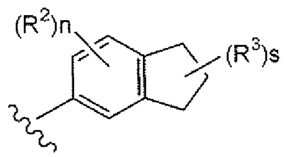

or

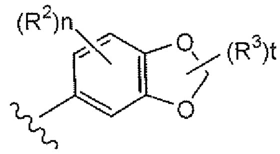

wherein
$R^2$ and $R^3$ are each independently halogen, cyano, hydroxy, acyl, acylamino, amino optionally substituted with lower alkyl, optionally substituted lower alkyl, lower alkyloxy, lower alkylsulfonyl, aryl optionally substituted with halogen and/or lower alkyl, heteroaryl optionally substituted with halogen and/or lower alkyl, or aralkyl optionally substituted with halogen and/or lower alkyl,
n and s are each independently an integer of 0 to 3, t is an integer of 0 to 2, and u is an integer of 0 or 1,
or a pharmaceutically acceptable salt thereof, or a solvate thereof.
5) The compound according to 1), or 2), wherein $A^2$ is a group represented by the formula:
[Chemical formula 6]

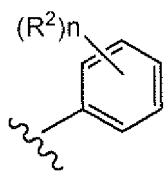

wherein $R^2$ and n are as defined in 1),
or a pharmaceutically acceptable salt thereof, or a solvate thereof.
6) The compound according to any one of 1) to 5), wherein $A^1$ is a group represented by the formula:
[Chemical formula 7]

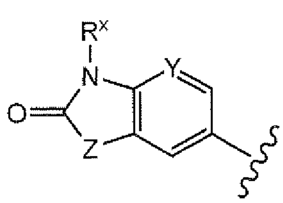

wherein Y, Z and $R^X$ are as defined in 1), and carbon atoms constituting a ring may be substituted with halogen,
or a pharmaceutically acceptable salt thereof, or a solvate thereof.
7) The compound according to any one of 1) to 5), wherein $A^1$ is a group represented by the formula:
[Chemical formula 8]

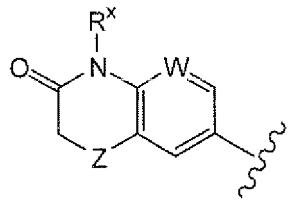

or

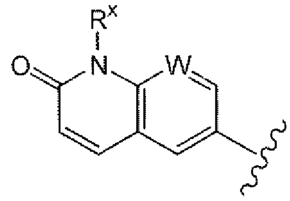

wherein W, Z and $R^X$ are as defined in 1), and carbon atoms constituting a ring in the group may be substituted with halogen,

or a pharmaceutically acceptable salt thereof, or a solvate thereof.

8) The compound according to any one of 1) to 7), wherein Z is an oxygen atom, or a pharmaceutically acceptable salt thereof, or a solvate thereof.

9) The compound according to any one of 1) to 8), wherein both ofY and W are CH, and $R^X$ is a hydrogen atom, or a pharmaceutically acceptable salt thereof, or a solvate thereof.

10) The compound according to any one of 1) to 9), wherein X is -NH-C(=O)-C(-O)-, $-NHCH_2C(=O)-$, $-NH-C(=O)-CH_2-$, -NH-CH(Me)·C(=O)-, -NH-C(=O)-CH(Me)-, $-C(=O)-NH-(CH_2)_2-$ or $-C(=O)-NH-(CH_2)_3-$ wherein Me is methyl, or a pharmaceutically acceptable salt thereof, or a solvate thereof. 11) The compound according to any one of 1), or 3) to 10), wherein m is 0 or 1, or a pharmaceutically acceptable salt thereof, or a solvate thereof.

12) The compound according to any one of 1), or 3) to 11), wherein $R^1$ is methyl, or a pharmaceutically acceptable salt thereof, or a solvate thereof. 13) A pharmaceutical composition containing the compound as defined in any one of 1) to 12), or a pharmaceutically acceptable salt thereof, or a solvate thereof.

14) The pharmaceutical composition according to 13), which has NMDA receptor antagonism.

15) The pharmaceutical composition according to 14), which has NR1/NR2B receptor antagonism.

16) A method of alleviating a pain, or a method of treating migraine, cerebral stroke, head trauma, Alzheimer's disease, Parkinson's disease, tinnitus, epilepsia, Huntington's disease, a motor disorder or alcohol dependency, comprising administering the compound as defined in any one of 1) to 12), or a pharmaceutically acceptable salt, or a solvate thereof.

17) Use of the compound as defined in any one of 1) to 12), for manufacturing an analgesic, or a therapeutic agent for migraine, cerebral stroke, head trauma, Alzheimer's disease, Parkinson's disease, tinnitus, epilepsia, Huntington's disease, a motor disorder or alcohol dependency.

18) The compound as defined in any one of 1) to 12) for use as an analgesic, or in therapy of migraine, cerebral stroke, head trauma, Alzheimer's disease, Parkinson's disease, tinnitus, epilepsia, Huntington's disease, a motor disorder or alcohol dependency.

[Effect of the invention]

The present compound is not only used in therapy of neurodegenezation such as cerebral stroke and brain trauma, but also is useful as an analgesic (e.g., cancer pain analgesic) having little side effect.

[Best mode for carrying out the invention]

Herein, "C1-C3 alkyl" includes straight or branched alkyl of a carbon number of 1 to 3, and examples include methyl, ethyl, isopropyl and n-propyl. Preferably, methyl is exemplified.

Herein, "halogen" includes fluorine, chlorine, bromine, or iodine.

Herein, "halo C1-C3 alkyl" includes the "C1-C3 alkyl" substituted with the halogen at 1 to 6 places, preferably 1 to 3 places. Examples include trifluoromethyl, trichloromethyl, trifluoroethyl, trichloroethyl, difluoromethyl, dichloromethyl, difluoroethyl, dichloroethyl and the like, preferably trifluoromethyl.

Herein, "C1-C3 alkoxy" includes straight or branched alkoxy of a carbon number of 1 to 3, and examples include methyloxy, ethyloxy, isopropyloxy, and n-propyloxy, preferably methyloxy and ethyloxy.

Herein, "halo C1-C3 alkoxy" includes the "C1-C3 alkoxy" substituted with the halogen at 1 tao 6 places, preferably 1 to 3 places. Examples include trifluoromethyloxy, trichloromethyloxy, trifluoroethyloxy, trichloroethyloxy, difluoromethyloxy, dichloromethyloxy, difluoroethyloxy, dichloroethyloxy and the like, preferably trifluoromethyloxy.

Herein, "lower alkyl" used alone or in combination with other term includes straight or branched alkyl of a carbon number of 1 to 8, more preferably a carbon number of 1 to 6, further preferably a carbon number of 1 to 3. Examples include methyl, ethyl, n-propyl, isopropyl, n-butyl, isobutyl, sec-butyl, tert-butyl, n-pentyl, isopentyl, neopentyl, hexyl,

isohexyl, n-heptyl, isoheptyl, n-octyl, isooctyl and the like.

Herein, "aryl" used alone or in combination with other term includes a monocyclic aromatic hydrocarbon group, and a condensed cyclic aromatic hydrocarbon group in which 2 or 3 aromatic rings are condensed. Examples include phenyl, naphthyl, anthryl, phenanthryl and the like, particularly preferably phenyl.

Herein, "non-aromatic carbocycle" includes cycloalkane, cycloalkene and the like.

"Cycloalkane" is a carbocycle of a carbon number of 3 to 10, preferably a carbon number of 4 to 8, more preferably a carbon number of 5 to 7, and examples include cyclopropane, cyclobutane, cyclopentane, cyclohexane, cycloheptane, cyclooctane, cyclononane, cyclodecane and the like.

"Cycloalkene" includes those having one or more double bonds at arbitrary positions in a ring of the cycloalkane, and examples include cyclopropene, cyclobutene, cyclopentene, cyclohexene, cycloheptene, cyclooctene, cycloheptadiene and the like.

Herein, "non-aromatic heterocycle" includes a 5- to 7- membered non-aromatic ring containing one or more heteroatoms selected from the group consisting of N, O and S in a ring, and a ring group in which two or more of them are condensed, and a ring group in which the "ring" is condensed with the "aryl". Examples include pyrrolidine, pyrroline, imidazolidine, imidazoline, pyrazolidine, pyrazoline, piperidine, piperazine, indoline, isoindoline, morpholine, dihydropyridine, tetrahydropyridine, tetrahydroquinoline, tetrahydrofuran, dihydropyrane, tetrahydropyrane, 1,3-benzodioxolane, 1,4-benzodioxane, 1-benzoxolane, oxetane, azetidine, diazetidine, chromane and the like.

Herein, "aromatic carbocycle" includes a 5- to 7- membered aromatic ring comprising carbon atoms, or a ring in which two or more of aromatic rings are condensed. Examples include benzene, pentalene, indene, naphthalene, azulene, fluorene, phenanthrene, anthracene and the like.

Herein, "aromatic heterocycle," includes a 5- to 6- membered aromatic ring comprising one or more heteroatoms selected from the group consisting of N, O and S in a ring, and the aromatic ring may be condensed with cycloalkyl, aryl, a non-aromatic heterocyclic group, or other heteroaryl, and these may be condensed at all the possible positions. Examples include pyrrole, furan, thiophene, imidazole, pyrazole, isothiazole, isoxazole, oxazole, thiazole, pyridine, pyrazine, pyrimidine, pyridazine, tetrazole, oxadiazole, thiadiazole, indolizine, isoindole, indole, indazole, purine, quinolizine, isoquinoline, quinoline, phthalazine, naphthyridme, quinoxaline, quinazoline, cinnoline, pteridine, carbazole, phenanthridine, acridine, dibenzofuran, benzimidazole, benzisoxazole, benzoxazole, benzoxadiazole, benzisothiazole, benzothiazole, benzofuran, benzothiophene, thienopyrimidine and the like.

Herein, "heteroaryl" used alone or in combination with other term includes a 5- to 6- membered aromatic cyclic group including one or more arbitrarily selected oxygen atoms, sulfur atoms or nitrogen atoms in a ring, this may be condensed with cycloalkyl, aryl, a non-aromatic heterocycle, or other heteroaryl, and these can condense at all the possible positions. Examples include pyrrolyl (e.g., 1-pyrrolyl, 2-pyrrolyl, 3-pyrrolyl), furyl (e.g., 2-furyl, 3-furyl), thienyl (e.g., 2-thienyl, 3-thienyl), imidazolyl (e.g., 2-imidazolyl, 4-imidazolyl), pyrazolyl (e.g., 1-pyrazolyl, 3-pyrazolyl), isothiazolyl (e.g., 3-isothiazolyl), isoxazolyl (e.g., 3-isoxazolyl), oxazolyl (e.g., 2-oxazolyl), thiazolyl (e.g., 2-thiazolyl), pyridyl (e.g., 2-pyridyl, 3-pyridyl, 4-pyridyl), pyrazinyl (e.g., 2-pyrazinyl), pyrimidinyl (e.g., 2-pyrimidinyl, 4-pyrimidinyl), pyridazinyl (e.g., 3-pyridazinyl), tetrazolyl (e.g., 1H-tetrazolyl), oxadiazolyl (e.g., 1,3,4-oxadiazolyl), thiadiazolyl (e.g., 1,3,4-thiadiazolyl), indolidinyl (e.g., 2-indolidinyl, 6-indolidinyl), isoindolyl (e.g., 2-isoindolyl), indolyl (e.g., 1-indolyl, 2-indolyl, 3-indolyl, 5-indolyl, 6-indolyl), indazolyl (e.g., 3-indazolyl), purinyl (e.g., 8-purinyl), quinolizinyl (e.g., 2-quinolizinyl), isoquinolyl (e.g., 3-isoquinolyl), quinolyl (e.g., 2-quinolyl, 5-quinolyl), phthalazinyl (e.g., 1-phthalazinyl), naphthyridinyl (e.g., 2-naphthyridinyl), quinoxanyl (e.g., 2-quinoxanyl), quinazolinyl (e.g., 2-quinazolinyl), cinnolinyl (e.g., 3-cinnolinyl), pteridinyl (e.g., 2-pteridinyl), carbazolyl (e.g., 2-carbazolyl, 3-carbazolyl), phenanthridinyl (e.g., 2-phenanthridinyl, 3-phenanthridinyl), acridinyl (e.g., 1-acridinyl, 2-acridinyl), dibenzofuranyl (e.g., 1-dibenzofuranyl, 2-dibenzofuranyl, 3-dibenzofuranyl), benzimidazolyl (e.g., 2-benzimidazolyl), benzisoxazolyl (e.g., 3-benzisoxazolyl), benzoxazolyl (e.g., 2-benzoxazolyl), benzoxadiazolyl (e.g., 4-benzoxadiazolyl), benzisothiazolyl (e.g., 3-benzisothiazolyl), benzothiazolyl (e.g., 2-benzothiazolyl, 5-benzothiazolyl), benzofuryl (e.g., 2-benzofuryl, 3-benzofuryl, 5-benzofuryl), benzothienyl (e.g., 2-benzothienyl), thienopyrimidinyl and the like.

Herein, "acyl" includes alkylcarbonyl in which an alkyl part is the "lower alkyl", and arylcarbonyl in which an aryl part is the "aryl". Examples include formyl, acetyl, propionyl, butyryl, isobutyryl, valeryl, pivaloyl, hexanoyl, acryloyl, propioloyl, methacryloyl, crotonoyl, benzoyl and the like.

Herein, "arylsulfonyl optionally substituted with lower alkyl" is sulfonyl in which an "aryl" part is the "aryl", and aryl may be substituted with one or more of the "lower alkyls" and examples include benzenesulfonyl, toluenesulfonyl and the like.

Herein, "carbamoyl optionally substituted with lower alkyl" includes carbamoyl optionally substituted with one "lower alkyl" described above, and examples include carbamoyl, methylcarbamoyl, ethylcarbamoyl, isopropylcarbamoyl and the like.

Herein, "acylamino" includes amino in which an acyl part is the "acyl", and examples include formylamino, acetylamino, propionylamino, butyrylamino, isobutyrylamino, valerylamino, pivaloylamino, hexanoylamino, acryloylamino, propioloylamino, benzoylamino and the like.

Herein, "amino optionally substituted with lower alkyl" includes amino optionally substituted with one or two of the "lower alkyls", and examples include methylamino, dimethylamino, ethylamino, diethylamino, n-propylamino, di n-propylamino, isopropylamino, n-butylamino, isobutylamino, sec-butylamino, tert-butylamino, n-pentylamino and the like.

Herein, examples of a substituent of "optionally substituted lower alkyl" include halogen, cyano, nitro, lower alkyloxy, aralkyloxy, halo lower alkyloxy, lower alkyloxyimino, halo lower alkyloxyimino, amino optionally substituted with lower alkyl, hydroxyl, hydroxyimino, alkylthio and the like.

Herein, "lower alkyloxy" is alkyloxy in which an alkyl part is the "lower alkyl", and examples include methyloxy, ethyloxy, n-propyloxy, isopropyloxy, n-butyloxy, isobutyloxy, sec-butyloxy, tert-butyloxy, n-pentyloxy, isopentyloxy, neopentyloxy, hexyloxy, isohexyloxy, n-heptyloxy, isoheptyloxy, n-octyloxy, isooctyloxy and the like.

Herein, "lower alkyloxycarbonyl" is carbonyl in which a lower alkyloxy part is substituted with the "lower alkyloxy", and examples include tert-butoxycarbonyl, methoxycarbonyl, ethoxycarbonyl and the like.

Herein, "aralkyl" used alone or in combination with other term is the "lower alkyl" substituted with one or more of the "aryls", and these can replace at all the possible positions. Examples include benzyl, phenylethyl (e.g., 2-phonylethyl etc.), phenylpropyl (e.g., 3-phenylethyl etc.) and the like, preferably benzyl and phenylethyl.

Herein, "aralkyloxy" is aralkyloxy in which an aralkyl part is the "aralkyl", and examples include benzyloxy, phenylethyloxy (e.g., 2-phenylethyl etc.) and the like, preferably benzyloxy.

Herein, "optionally substituted aralkyloxycarbonyl" is substituted carbonyl with the "aralkyloxy", and examples of the substituent of aryl include halogen, cyano, nitro, lower alkyl, halo lower alkyl, lower alkyloxy, halo lower alkyloxy, hydroxy and the like. Preferable examples of "optionally substituted aralkyloxycarbonyl" include benzyloxycarbonyl, p-methoxybenzyloxycarbonyl, p-nitrobenzyloxycarbonyl and the like.

Herein, "lower alkyloxyimino" is alkyloxyimino in which an alkyl part is the "lower alkyl", and examples include methyloxyimino, ethyloxyimino, n-propyloxyimino, isopropyloxyimino, n-butyloxyimino, isobutyloxyimino, sec-butyloxyimino, tert-butyloxyimino and the like.

Herein, the term "halo lower alkyl" used alone or in combination with other term includes the "lower alkyl" substituted with the "halogen" at 1 to 8 places, preferably 1 to 5 places. Examples include trifluoromethyl, trichloromethyl, trifluoroethyl, trichloroethyl, difluoromethyl, dichloromethyl, difluoroethyl, dichloroethyl and the like, preferably trifluoromethyl.

Herein, "halo lower alkyloxyimino" includes the "lower alkyloxyimino" substituted with the "halogen" at 1 to 8 places, preferably 1 to 5 places. Examples include trifluoromethyloxyimino, trifluoroethyloxyimino and the like.

Herein, the term "amino optionally substituted with lower alkyl" is "amino" optionally substituted with the "lower alkyl" at 1 to 2 places and, as a substituent, methyl, ethyl, n-propyl or isopropyl is preferable.

Herein, "lower alkylsulfonyl" is alkylsulfonyl in which an alkyl part is the "lower alkyl", and examples include methylsulfonyl, ethylsulfonyl and the like.

Herein, the term "aryl optionally substituted with halogen and/or lower alkyl" is the "aryl" optionally substituted with the "halogen" and/or the "lower alkyl" at 1 to 3 places and, as a substituent, fluorine, chlorine, methyl, ethyl, n-propyl or isopropyl is preferable.

Herein, the term "heteroaryl optionally substituted with halogen and/or lower alkyl" is the "heteroaryl" optionally substituted with the "halogen" and/or the "lower alkyl" at 1 to 3 places and, as a substituent, fluorine, chlorine, methyl, ethyl, n-propyl or isopropyl is preferable.

Herein, the term "aralkyl optionally substituted with halogen and/or lower alkyl" is "aralkyl" optionally substituted with the "halogen and/or the "lower alkyl" at 1 to 3 places and, as a substituent, fluorine, chlorine, methyl, ethyl, n-propyl or isopropyl is preferable.

The case where "two of $R^1$ is bound to the same carbon atom to form oxo" means:

[Chemical formula 9]

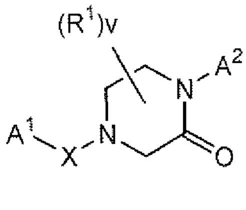
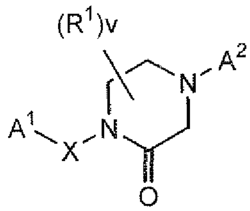
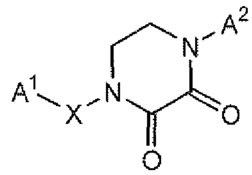

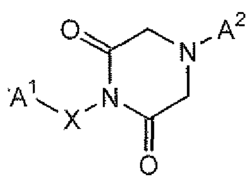
or
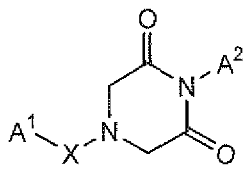

wherein $A^1$, X and $A^2$ are as defined in 1), and v is an integer of 0 to 2.
The case where "$R^1$ is bound to different two carbon atoms which are not adjacent to form $-(CH_2)_r-$" means:
[Chemical formula 10]

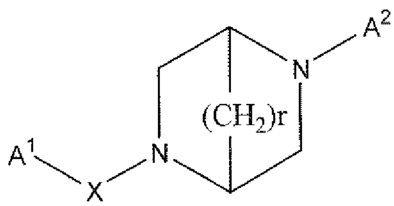
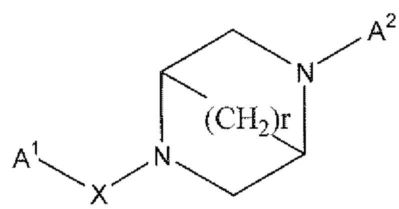

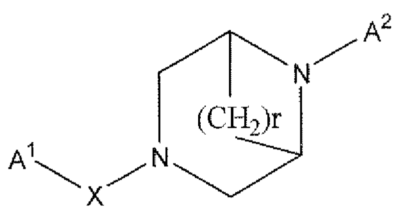
or
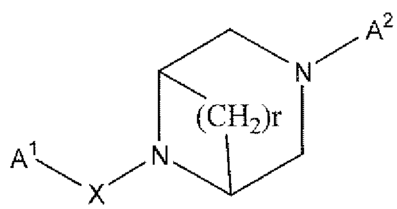

wherein $A^1$, X, $A^2$, and r are as defined in 1).
The present compound (I) is not limited to a specified isomer, but includes all possible isomers and racemates. For example, the present compound includes tautomers as follows.
[Chemical formula 11]

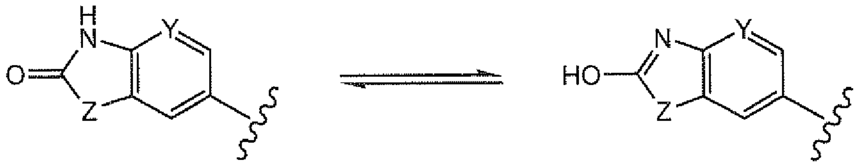

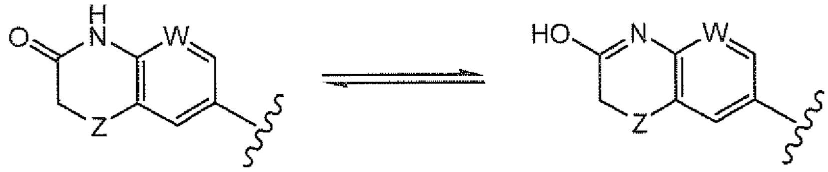

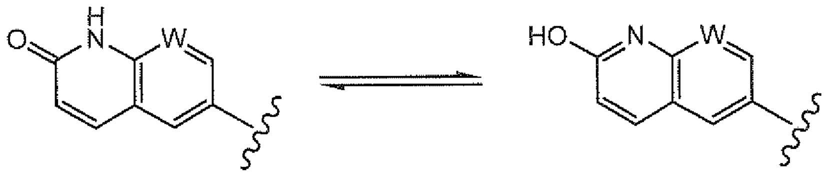

A general method of synthesizing the present compound will be shown below, but is not limited to the present synthesis method.

A method: Synthesis of (I-a) from compound (II)

[Chemical formula 12]

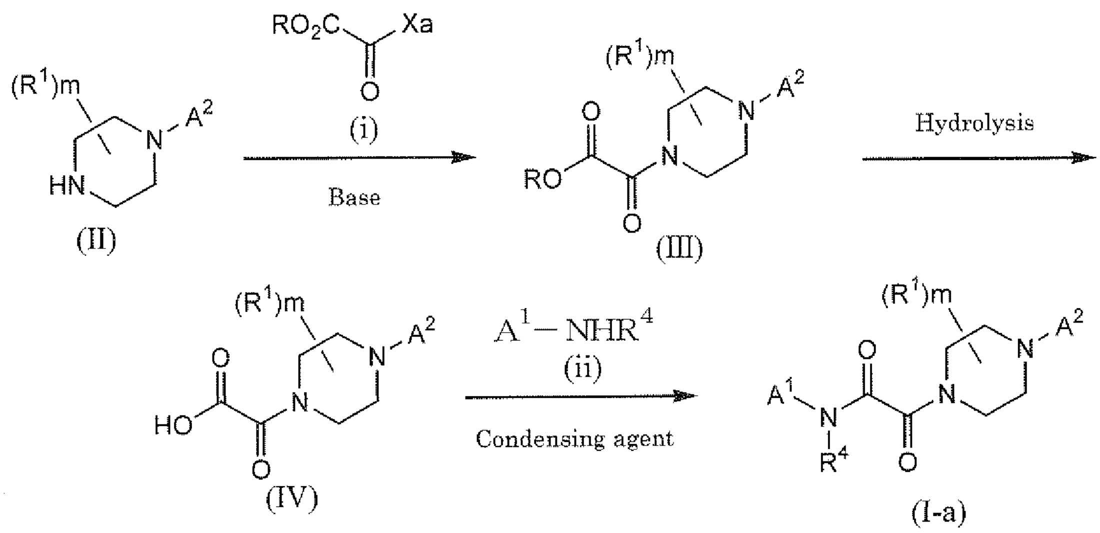


(II) (III) (IV) (I-a)

wherein R is a protecting group of carboxylic acid, $X^a$ is a chlorine atom or a bromine atom, and other symbols are as defined above.

A piperazine derivative represented by the general formula (II) is a commercially available product, or can be synthesized by methods described in Examples described later, as well as methods according to those methods. In addition, a compound represented by the general formula (ii) is a commercially available product, or can be synthesized by the known methods (Bulletin of the Korean Chemical Society, 2004, vol.25, No.9, pp.1326-1330, Acta Facultatis Rerum Naturalium Universtatis Comennianae Chimia, 1985, vol.33, pp.137-146, Bulletin of the Korean Chemical Society, 2005, vol.26, No.11, pp.1757-1760, US Patent No.20050256144), as well as methods according to those methods.

Examples of a protecting group R include methyl, ethyl and the like.

a) Synthesis of (III) from general formula (II)

A compound represented by the general formula (III) can be synthesized by condensing a compound represented by the general formula (II) and a compound represented by the general formula (i) in the presence of a base. The compound represented by the general formula (i) can be used at 1 to 3 mole equivalents relative to the compound represented by the general formula (II).
Examples of the reaction solvent include tetrahydrofuran, diethyl ether, acetonitrile, methylene chloride, chloroform, toluene, N,N-dimethylformamide, 1,3-dimethyl-2-imidazolidinone, N-methyl-2-pyrrolidone, water and the like, and these can be used alone or by mixing them.
Examples of the base include sodium hydroxide, potassium hydroxide, sodium bicarbonate, sodium carbonate, potassium carbonate, sodium hydride, potassium hydride, triethylamine, morpholine, N-methylmorpholine and the like. The base can be used at 1.0 to 5 mole equivalents relative to the compound represented by the general formula (II).
An example of a reaction temperature includes -10 to 50˚C.
An example of a reaction time includes 0.5 to 24 hours.
The resulting compound represented by the general formula (III) can be isolated and purified by the known means (e.g., chromatography, recrystallization and the like).

b) Synthesis of (IV) from general formula (III)

Carboxylic acid represented by the general formula (IV) can be synthesized by hydrolyzing the compound represented by the general formula (III).
Lithium hydroxide, sodium hydroxide, potassium hydroxide or the like can be used at 1.0 to 5 mole equivalents relative to the compound represented by the general formula (III).
Examples of a reaction solvent include methanol, ethanol, propanol, isopropanol, butanol, water and the like, and solvents can be used alone or by mixing them.
An example of a reaction temperature includes 0˚C to a refluxing temperature of a solvent.
An example of a reaction time includes 0.5 to 24 hours.
The resulting compound represented by the general formula (IV) can be isolated and purified by the known means (e.g., chromatography, recrystallization and the like).

c) Synthesis of (I-a) from general formula (IV)

An amide compound represented by the general formula (I-a) can be synthesized by condensing carboxylic acid represented by the general formula (IV) and an amine compound represented by the general formula (ii) in the presence of a condensing agent.
The compound represented by the general formula (ii) can be used at 0.5 to 2 mole equivalents relative to the compound represented by the general formula (IV).
Examples of a reaction solvent include methylene chloride, tetrahydrofuran, N,N-dimethylformamide, N,N-dimethylacetamide, 1,3-dimethyl-2-imidazolidinone, N-methyl-2-pyrrolidone and the like.
Examples of the condensing agent include dicyclohexylcarbodiimide, 1-(3-dimethylaminopropyl)-3-ethylcarbodiimide hydrochloride, N,N'-carbonyldiimidazole, diethylphosphorocyanidate, diphenylphosphoryl azide, propylphosphonic acid anhydride, ethyl chlorocarbonate, isobutyl chlorocarbonate, thionyl chloride, oxalyl chloride and the like, and the condensing agent can be used at 0.5 to 2 mole equivalents relative to the compound represented by the general formula (IV). 1-Hydroxybenzotriazole and the like may be used as a condensation aid at 0.5 to 2 mole equivalents.
Examples of the base include triethylamine, N-methylmorpholine, 4-dimethylaminopyridine and the like, and these can be used alone or by mixing them. Each of them can be used at 0.05 to 2 mole equivalents relative to the compound represented by the general formula (IV).
An example of a reaction temperature includes 0 to 100˚C.
An example of a reaction time includes 0.5 to 72 hours.
In addition, when ethyl chlorocarbonate, isobutyl chlorocarbonate, thionyl chloride, oxalyl chloride or the like is used as the condensing agent, a reaction time can be shortened.
The resulting compound represented by the general formula (I-a) can be isolated and purified by the known means (e.g., chromatography, recrystallization and the like).

B method: Synthesis of (I-b) from compound (II)

[Chemical formula 13]

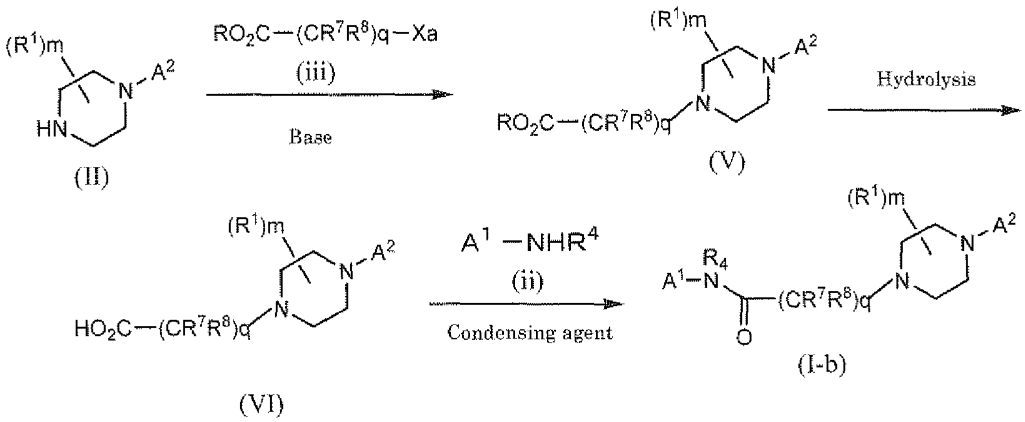


wherein R is a protecting group of carboxylic acid, and each symbol is as defined above.

a) Synthesis of (V) from general formula (II)

A compound represented by the general formula (V) can be synthesized by condensing a compound represented by the general formula (II) and a compound represented by the general formula (iii) in the presence of a base.

The compound represented by the general formula (iii) can be used at 1 to 3 mole equivalents relative to the compound represented by the general formula (II).
Examples of a reaction solvent include tetrahydrofuran, diethyl ether, acetonitrile, methylene chloride, chloroform, toluene, N,N-dimethylformamide, N-methyl-2-pyrrolidone, water and the like, and these can be used alone or by mixing them.

Examples of the base include sodium hydroxide, potassium hydroxide, sodium bicarbonate, sodium carbonate, potassium carbonate, sodium hydride, potassium hydride, triethylamine, morpholine, N-methylmorpholine and the like. The base can be used at 1.0 to 5 mole equivalents relative to the compound represented by the general formula (II). As an additive, sodium iodide or potassium iodide can be used at 0.05 to 2 mole equivalents relative to the compound represented by the general formula (II).

An example of a reaction temperature includes -10 to 50˚C.
An example of a reaction time includes 0.5 to 24 hours.
The resulting compound represented by the general formula (III) can be isolated and purified by the known means (e.g., chromatography, recrystallization and the like).

b) Synthesis of (VI) from general formula (V)

Carboxylic acid represented by the general formula (VI) can be synthesized by hydrolyzing a compound represented by the general formula (V).
Lithium hydroxide, sodium hydroxide, potassium hydroxide or the like can be used at 1.0 to 5 mole equivalents relative to the compound represented by the general formula (V).

Examples of a reaction solvent include methanol, ethanol, propanol, isopropanol, butanol, water and the like, and solvents can be used alone or by mixing them.
An example of a reaction temperature includes 0˚C to a refluxing temperature of a solvent.
An example of a reaction time includes 0.5 to 24 hours.
The resulting compound represented by the general formula (VI) can be isolated and purified by the known means (e.g., chromatography, recrystallization and the like).

c) Synthesis of (I-b) from general formula (VI)

An amide compound represented by the general formula (I-b) by condensing carboxylic acid represented by

the general formula (VI) and an amine compound represented by the general formula (ii) in the presence of a condensing agent.
The compound represented by the general formula (ii) can be used at 0.5 to 2 mole equivalents relative to the compound represented by the general formula (VI).
Examples of a reaction solvent include methylene chloride, tetrahydrofuran, N,N-dimethylformamide, N,N-dimethylacetamide, 1,3-dimethyl-2-imidazolidinone, N-methyl-2-pyrrolidone and the like.
Examples of the condensing agent include dicyclohexylcarbodiimide, 1-(3-dimethylaminopropyl)-3-ethylcarbodiimide hydrochloride, N,N'-carbonyldiimidazole, diethylphosphorocyanidate, diphenylphosphoryl azide, propylphosphonic acid anhydride, ethyl chlorocarbonate, isobutyl chlorocarbonate, thionyl chloride, oxalyl chloride and the like, and the condensing agent can be used at 0.5 to 2 mole equivalents relative to the compound represented by the general formula (VI). 1-Hydroxybenzotriazole and the like can be used as a condensation aid at 0.5 to 2 mole equivalents.
Examples of the base include triethylamine, N-methylmorpholine, 4-dimethylaminopyridine and the like, and these can be used alone or by mixing them. Each of them can be used at 0.05 to 2 mole equivalents relative to the compound represented by the general formula (VI).
An example of a reaction temperature includes 0 to 1004˚C.
An example of a reaction time includes 0.5 to 72 hours.
In addition, when ethyl chlorocarbonate, isobutyl chlorocarbonate, thionyl chloride, oxalyl chloride or the like is used as the condensing agent, a reaction time can be shortened.
The resulting compound represented by the general formula (I-b) can be isolated and purified by the known means (e.g., chromatography, recrystallization and the like).

C method: Synthesis of (I-c) from compound (II)

[Chemical formula 15]

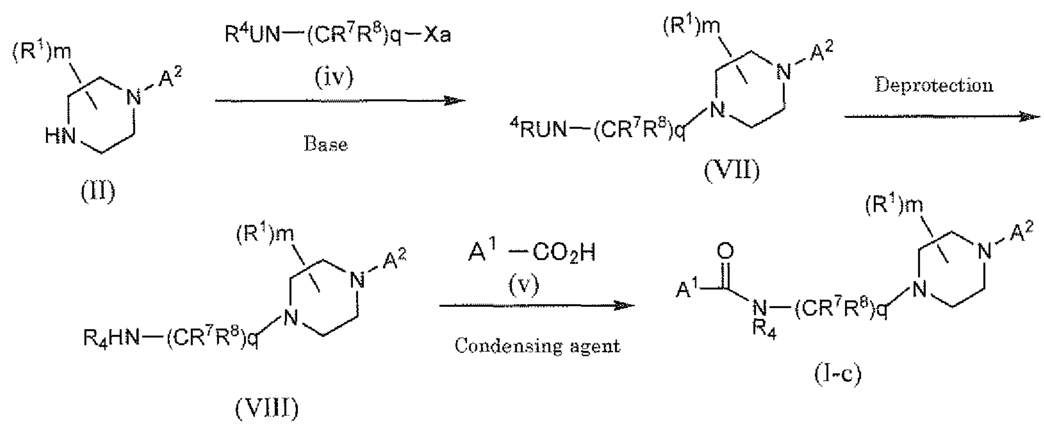

(II) (VII) (VIII) (I-c)

wherein U is a protecting group of amine, and each symbol is as defined above.
Examples of the protecting group U include t-butoxycarbonyl, benzyloxycarbonyl and the like.

a) Synthesis of (VII) from general formula (II)

A compound represented by the general formula (VII) can be synthesized by condensing a compound represented by the general formula (II) and a compound represented by the general formula (iv) in the presence of a base.
The compound represented by the general formula (iv) can be used at 1 to 3 mole equivalents relative to the compound represented by the general formula (II).
Examples of a reaction solvent include tetrahydrofuran, diethyl ether, acetonitrile, methylene chloride, chloroform, toluene, N,N-dimethylformamide, N-methyl-2-pyrrolidone, water and the like, and these can be used alone or by mixing them.
Examples of the base include sodium hydroxide, potassium hydroxide, sodium bicarbonate, sodium carbonate, potassium carbonate, sodium hydride, potassium hydride, triethylamine, morpholine, N-methylmorpholine and the like. The base can be used at 1.0 to 5 mole equivalents relative to the compound represented by the general formula (II). As an additive, sodium iodide or potassium iodide can be used at 0.05 to 2 mole equivalents relative to the compound represented by the general formula (II).
An example of a reaction temperature includes -10 to a refluxing temperature of a solvent.
An example of a reaction time includes 0.5 to 24 hours.
The resulting compound represented by the general formula (VII) can be isolated and purified by the known means (e.g., chromatography, recrystallization and the like).

b) Synthesis of (VIII) from general formula (VII)

Amine represented by the general formula (VIII) can be synthesized by deprotecting a compound represented by the general formula (VII).
Hydrogen chloride, hydrogen bromide, trifluoroacetic acid, formic acid or the like can be used at 1.0 to 10 mole equivalents relative to the compound represented by the general formula (VII).
Examples of the reaction solvent include ethyl acetate, methanol, ethanol, propanol, isopropanol, butanol, acetic acid, water and the like, and these can be used alone or by mixing them.
An example of a reaction temperature includes 0˚C to a refluxing temperature of a solvent.
An example of a reaction time includes 0.5 to 24 hours.
The resulting compound represented by the general formula (VIII) can be isolated and purified by the known means (e.g., chromatography, recrystallization and the like).

c) Synthesis of (I-c) from general formula (VIII)

An amide compound represented by the general formula (I-c) can be synthesized by condensing amine represented by the general formula (VIII) and a compound represented by the general formula (v) in the presence of a condensing agent.
The compound represented by the general formula (v) can be used at 0.5 to 2 mole equivalents relative to the compound represented by the general formula (VIII).
Examples of a reaction solvent include methylene chloride, tetrahydrofuran, N,N-dimethylformamide, N,N-dimethylacetamide, 1,3-dimethyl-2-imidazolidinone, N-methyl-2-pyrrolidone and the like.
Examples of the condensing agent include dicyclohexylcarbodiimide, 1-(3-dimethylaminopropyl)-3-ethylcarbodiimide hydrochloride, N,N'-carbonyldiimidazole, diethylphosphorocyanidate, diphenylphosphoryl azide, propylphosphonic acid anhydride, ethyl chlorocarbonate, isobutyl chlorocarbonate, thionyl chloride, oxalyl chloride and the like, and the condensing agent can be used at 0.5 to 2 mole equivalents relative to the compound represented by the general formula (VIII).
1-Hydroxybenzotriazole and the like can be used as a condensation aid at 0.5 to 2 mole equivalents.
Examples of the base include triethylamine, N-methylmorpholine, 4-dimethylaminopyridine and the like, and these can be used alone or by mixing them. Each of them can be used at 0.05 to 2 mole equivalents relative to the compound represented by the general formula (VIII).
An example of a reaction temperature includes 0 to 100˚C.
An example of a reaction time includes 0.5 to 72 hours.
In addition, when ethyl chlorocarbonate, isobutyl chlorocarbonate, thionyl chloride, oxalyl chloride or the like is used as the condensing agent, a reaction time can be shortened.
The resulting compound represented by the general formula (I-c) can be isolated and purified by the known means (e.g., chromatography, recrystallization and the like).
D method: Synthesis of (I-d) from compound (II)
[Chemical formula 16]

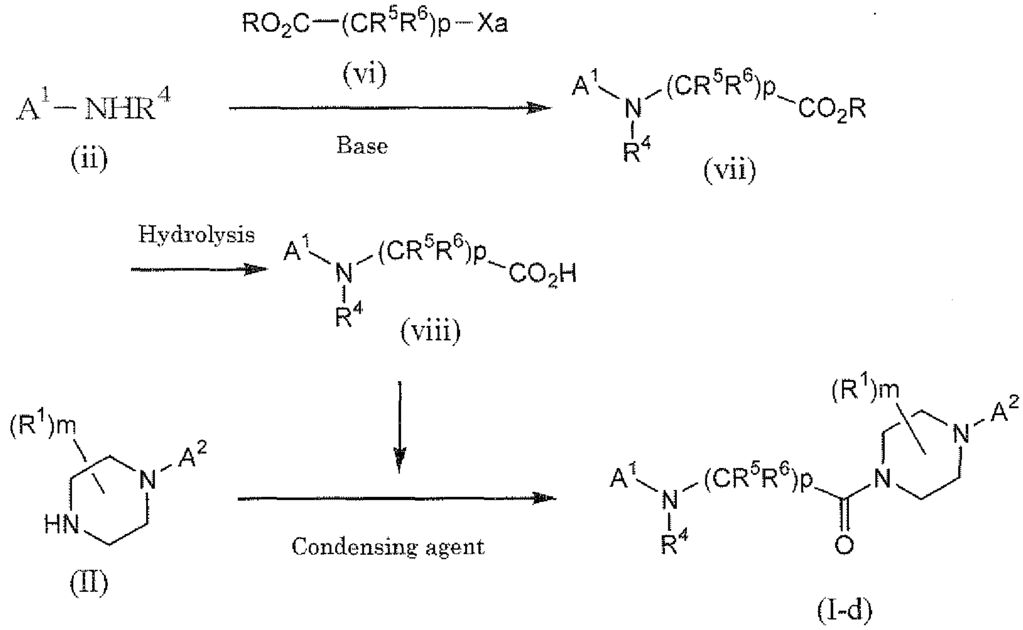


wherein R is a protecting group of carboxylic acid, and each symbol is as defined above.

a) Synthesis of (vii) from general formula (ii)

A compound represented by the general formula (vii) can be synthesized by condensing a compound represented by the general formula (ii) and a compound represented by the general formula (vi) in the presence or absence of a base.
The compound represented by the general formula (vi) can be used at 1 to 3 mole equivalents relative to the compound represented by the general formula (ii).
Examples of a reaction solvent include tetrahydrofuran, diethyl ether, acetonitrile, methylene chloride, chloroform, toluene, N,N-dimethylformamide, N-methyl-2-pyrrolidone, water and the like, and these can be used alone or by mixing them.
Examples of the base include sodium hydroxide, potassium hydroxide, sodium bicarbonate, sodium carbonate, potassium carbonate, sodium hydride, potassium hydride, triethylamine, morpholine, N-methylmorpholine, diisopropylethylamine and the like. The base can be used at 0.1 to 5 mole equivalents relative to the compound represented by the general formula (ii).
An example of a reaction temperature includes -10 to a refluxing temperature of a solvent.
An example of a reaction time includes 0.5 to 24 hours.
The resulting compound represented by the general formula (vii) can be isolated and purified by the known means (e.g., chromatography, recrystallization and the like).

b) Synthesis of (viii) from general formula (vii)

Carboxylic acid represented by the general formula (viii) can be synthesized by hydrolyzing a compound represented by the general formula (vii).
Lithium hydroxide, sodium hydroxide, potassium hydroxide or the like can be used at 1.0 to 5 mole equivalents relative to the compound represented by the general formula (vii).
Examples of a reaction solvent include methanol, ethanol, propanol, isopropanol, butanol, water and the like, and solvents can be used alone or by mixing them.
An example of a reaction temperature includes 0˚C to a refluxing temperature of a solvent.
An example of a reaction time includes 0.5 to 24 hours.
The resulting compound represented by the general formula (viii) can be isolated and purified by the known means (e.g., chromatography, recrystallization and the like).

c) Synthesis of (I-d) from general formula (II)

An amide compound represented by the general formula (I-d) can be synthesized by condensing amine represented by the general formula (II) and carboxylic acid represented by the general formula (viii) obtained above in the presence of a condensing agent.
The compound represented by the general formula (viii) can be used at 0.5 to 2 mole equivalents relative to the compound represented by the general formula (II).
Examples of a reaction solvent include methylene chloride, tetrahydrofuran, N,N-dimethylformamide, N,N-dimethylacetamide, 1,3-dimethyl-2-imidazolidinone, N-methyl-2-pyrrolidone and the like.
Examples of the condensing agent include dicyclohexylcarbodiimide, 1-(3-dimethylaminopropyl)-3-ethylcarbodiimide hydrochloride, N,N'-carbonyldiimidazole, diethylphosphorocyanidate, diphenylphosphoryl azide, propylphosphonic acid anhydride, ethyl chlorocarbonate, isobutyl chlorocarbonate, thionyl chloride, oxalyl chloride and the like, and the condensing agent can be used at 0.5 to 2 mole equivalents relative to the compound represented by the general formula (II). 1-Hydroxybenzotriazole and the like can be used as a condensation aid at 0.5 to 2 mole equivalents.
Examples of the base include triethylamine, N-methylmorpholine, 4-dimethylaminopyridine and the like, and these can be used alone or by mixing them. Each of them can be used at 0.05 to 2 mole equivalents relative to the compound represented by the general formula (II).
An example of a reaction temperature includes 0 to 100˚C.
An example of a reaction time includes 0.5 to 72 hours.
In addition, when ethyl chlorocarbonate, isobutyl chlorocarbonate, thionyl chloride, oxalyl chloride or the like is used as the condensing agent, a reaction time can be shortened.
The resulting compound represented by the general formula (I-d) can be isolated and purified by the known means (e.g., chromatography, recrystallization and the like).
Herein, the "protecting group" can be deprotected under the normally used reaction condition (e.g., the method described in T. W. Green et al., Protective Groups in Organic Synthesis, Second Edition, John Wiley & Sons (1991)).
When the present compound contains an optical isomer, a steric isomer, a positional isomer, or a rotational isomer, they are also included as the present compound, and each can be obtained by a single compound by the known per se synthesis procedure or separation procedure. For example, when an optical isomer is present in the present compound, an optical isomer resolved from the compound is also included in the present compound. The optical isomer can be produced by the known per se method. Specifically, an optical isomer is obtained by optically resolving a mixture of racemates of a final product according to a usual method, or by using an optically active starting material or intermediate.
As an optical resolving method, the known per se method, for example, a separation recrystallization method, a chiral column method, a diastereomer method and the like, described in detail below, is used.

1) Separation recrystallization method

A salt is formed by a racemate and an optically active compound (e.g., (+)-mandelic acid, (-)-mandelic acid, (+)-tartaric acid, (-)-tartaric acid, (+)-1-phenethyl amine, (-)-1-phenethyl amine, cinchonine, (-)-cinchonidine, brucine and the like), and this is separated by a separation recrystallization method to obtain a free optical isomer optionally via a neutralization step.

2) Chiral column method

A method of separation by applying a racemate or a salt thereof to a column for separating an optical isomer (chiral column). For example, in the case of liquid chromatography, a mixture of optical isomers is added to a chiral column such as ENANTIO-OVM (manufactured by Toso Co., Ltd.) or CHIRAL Series manufactured by Daicel Chemical Industries Ltd., this is developed with water, a variety of buffers (e.g., phosphate buffer), or an organic solvent (e.g., ethanol, methanol, isopropanol, acetonitrile, trifluoroacetic acid, diethylamine and the like) alone or as a mixed solution, thereby, optical isomers are separated. In addition, for example, in the case of gas chromatography, separation is performed using a chiral column such as CP-Chirasil-DeX CB (manufactured by GL Science Inc.).

3) Diastereomer method

A method of obtaining an optical isomer by converting a mixture of racemates into a mixture of diastereomers by a chemical reaction with an optically active reagent, converting this into a single substance via a usual separation means (e.g., separation recrystallization, chromatography method and the like), thereafter, severing an optically active reagent site by chemical treatment such as a hydrolysis method. For example, when the present compound has hydroxy or primary or secondary amino in a molecule, the compound and an optically active organic acid (e.g., MTPA [α-methoxy-α-(trifluoromethyl)phenylacetic acid], (-)-menthoxy acetic acid etc.) are subjected to a condensation reaction, thereby, a diastereomer of an ester body or a diastereomer of an amide body can be obtained, respectively. On the other hand, when the present compound has a carboxylic acid group, the compound and optically active amine or an alcohol reagent are subjected to a condensation reaction, a diastereomer of an amide body or a diastereomer of an ester body is obtained, respectively. A separated diastereomer is converted into an optical isomer of the original compound by being subjected to an acid hydrolysis or base hydrolysis reaction.

As a salt of the present compound, a pharmaceutically acceptable salt can be used, and examples of a basic addition salt include alkali metal salts such as a sodium salt, a potassium salt and the like; alkaline earth metal salts such as a calcium salt, a magnesium salt and the like; an ammonium salt; a trimethylamine salt, a triethylamine salt; aliphatic amine salts such as a dicyclohexylamine salt, an ethanolamine salt, a diethanolamine salt, a triethanolamine salt, a procaine salt and the like; aralkylamine salts such as a N,N-dibenzylethylenediamine salt and the like; heterocyclic aromatic amine salts such as a pyridine salt, a picoline salt, a quinoline salt, an isoquinoline salt and the like; quaternary ammonium salts such as a tetramethylammonium salt, a tetraethylammonium salt, a benzyltrimethylammonium salt, a benzyltriethylammonium salt, a benzyltributylammonium salt, a methyltrioctylammonium salt, a tetrabutylammonium salt and the like; an arginine salt; basic amino acid salts such as a lysine salt and the like.

Examples of an acid addition salt include inorganic acid salts such as hydrochloride, sulfate, nitrate, phosphate, carbonate, bicarbonate, perchlorate and the like; organic acid salts such as oxalate, acetate, propionate, lactate, maleate, fumarate, tartarate, malate, citrate, ascorbate and the like; sulfonates such as methanesulfonate, isethionate, benzenesulfonate, p-toluenesulfonate and the like; acidic amino acids salts such as aspartate, glutamate and the like.

The present compound (I) may be a solvate of water, acetonitrile, acetone, ethyl acetate, methanol, ethanol or the like. And, the number of solvation of a solvate of the present compound can usually vary depending on a synthesis method, a purification method or a crystallization condition and, for example, is in a range of 0.5 to 5 molecules per one molecule of a compound.

Among the present compound (I), the following compounds are particularly preferable.

In the formula (I),

$R^1$ is methyl, ethyl, trifluoromethyl or trifluoroethyl, or $R^1$ may be bound to the same carbon atom to form oxo, and m is an integer of 0 to 2,

1) a compound in which $A^1$ is benzoxazolinone, methylbenzimidazolinone, methyldihydroimidazopyridinone or dihydropyridoxazole-one (hereinafter, $A^1$ is al),
2) a compound in which $A^1$ is dihydroquinolinone, quinoline-one, dihydronaphthyridine-one or oxodihydronaphthyridine (hereinafter, A is a2),
3) a compound in which X is -NH-C(=O)-C(=O)-, $-NHCH_2C$ (=O)-, $-NH(CH_2)_2C(=O)-$, $-NH-C(=O)-CH_2-$, -NH-CH (Me)-C(=C)-, -NH-C(=O)-CH(Me)-, $-C(-O)-NH-(CH_2)_2-$ or $-C(=O)-NH-(CH_2)_3-$ wherein Me is methyl (hereinafter, X is x1),
4) a compound in which X is -NH-C(=O)-C(=O)-, $NHCH_2C(=O)-$, $-NH-C(=O)-CH_2-$ or $-C(=O)-NH-(CH_2)_2-$ (hereinafter, X is x2),
5) a compound in which $A^2$ is phenyl optionally substituted with one or more groups selected from halogen, cyano, hydroxy, acyl, acylamino, amino optionally substituted with lower alkyl, optionally substituted lower alkyl, lower alkyloxy, lower alkylsulfonyl, aryl optionally substituted with halogen and/or lower alkyl, heteroaryl optionally substituted with halogen and/or lower alkyl, or aralkyl optionally substituted with halogen and/or lower alkyl (hereinafter, $A^2$ is b1),
6) a compound in which $A^2$ is phenyl optionally substituted with one or more groups selected from halogen, C1-C3 alkyl, C1-C3 alkoxy, C1-C3 alkyl substituted with halogen, C1-C3 alkoxy substituted with halogen or C1-C3 alkyl substituted with C1-C3 alkoxy (hereinafter, $A^2$ is b2),
7) a compound in which $A^2$ is phenyl in which a meta-position and a para-position may be substituted with 2 or 3 groups selected from halogen, C1-C3 alkyl, C1-C3 alkoxy, C1-C3 alkyl substituted with halogen, C1-C3 alkoxy substituted with halogen or C1-C3 alkyl substituted with C1-C3 alkoxy, respectively (hereinafter, $A^2$ is b3),
8) a compound in which a combination of $A^1$, X, and $A^2$ ($A^1$, X, $A^2$) is as follows,
($A^1$, X, $A^2$) = (a1, x1, b1), (a1, x1, b2), (a1, x1, b3), (a1, x2, b1), (a1, x2, b2), (a1, x2, b3), (a2, x1, b1), (a2, x1, b2), (a2, x1, b3), (a2, x2, b1), (a2, x2, b2), (a2, x2, b3)
or a pharmaceutically acceptable salt, or a solvate thereof.

A compound in which a combination of $A^1$, X, $A^2$ ($A^1$, X, $X^2$) is as follows in the following formula (1) to (13), a pharmaceutically acceptable salt or a solvate thereof is also a preferable aspect of the present invention.

[Chemical formula 17]

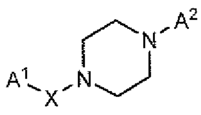
(1)

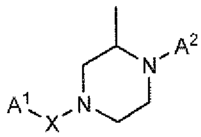
(2)

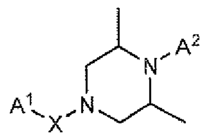
(3)

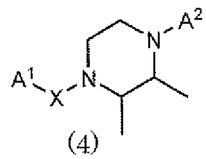
(4)

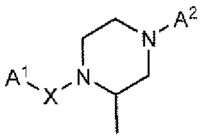
(5)

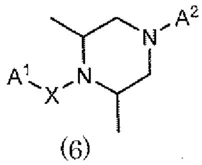
(6)

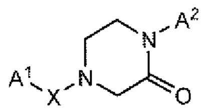
(7)

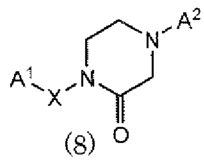
(8)

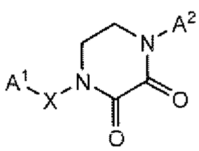
(9)

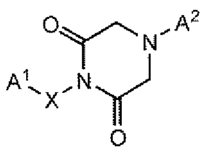
(10)

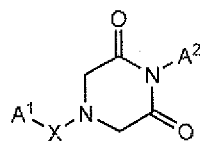
(11)

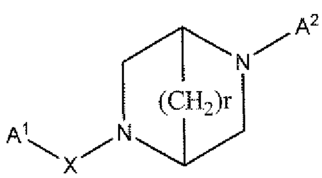
(12)

or

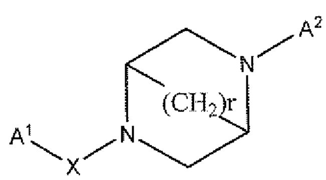
(13)

[Table 1]

| A1 | | A1 | |
|---|---|---|---|
| a1 | 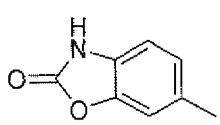 | a10 | 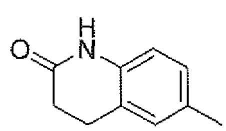 |
| a2 | 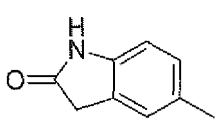 | a11 | 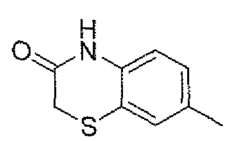 |

(continued)

| A1 | | A1 | |
|---|---|---|---|
| a3 | 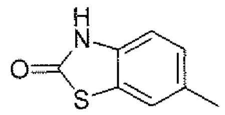 | a12 | 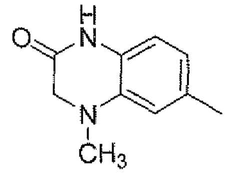 |
| a4 | 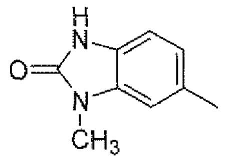 | a13 | 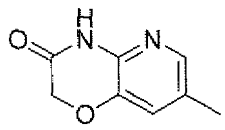 |
| a5 | 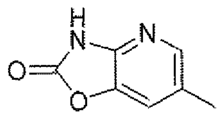 | a14 | 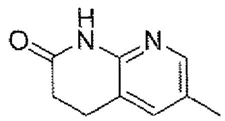 |
| a6 | 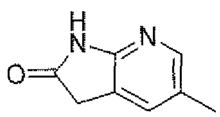 | a15 | 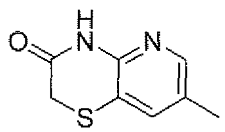 |
| a7 | 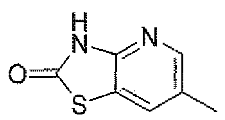 | a16 | 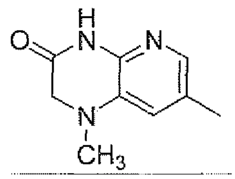 |
| a8 | 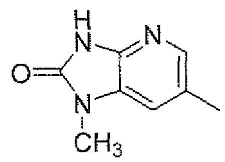 | a17 | 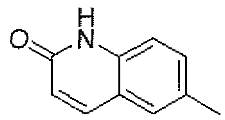 |
| a9 | 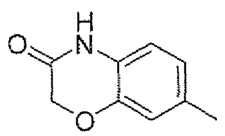 | a18 | 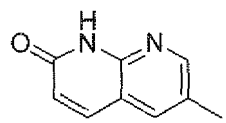 |

[Table 2]

| X | |
|---|---|
| X1 | $NH-CH_2-C(=O)$ |
| X2 | $NH-CH_2CH_2-C(O)$ |
| X3 | $NH-CH(CH_3)-C(=O)$ |
| X4 | NH-C(=O)-C(=O) |
| X5 | $NH-C(=O)-CH_2$ |
| X6 | $C(=O)-NH-CH_2CH_2$ |
| X7 | $CH_2-NH-C(=O)-C(=O)$ |

[Table 3]

| A2 | | A2 | | A2 | |
|---|---|---|---|---|---|
| b1 | 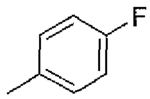 | b11 | 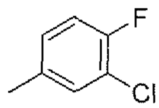 | b21 | 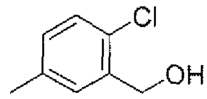 |
| b2 | 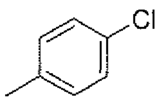 | b12 | 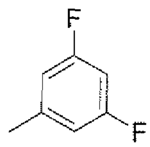 | b22 | 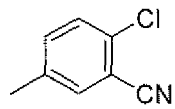 |
| b3 | 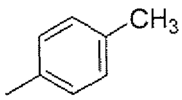 | b13 | 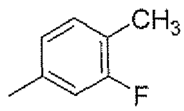 | b23 | 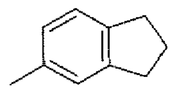 |
| b4 | 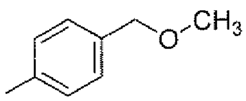 | b14 | 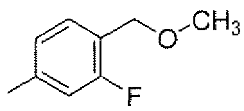 | b24 | 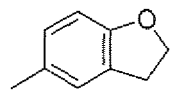 |
| b5 | 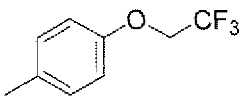 | b15 | 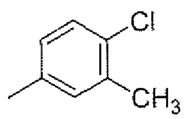 | b25 | 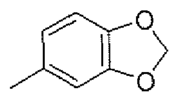 |
| b6 | 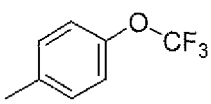 | b16 | 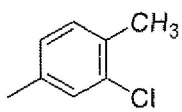 | b26 | 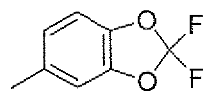 |
| b7 | 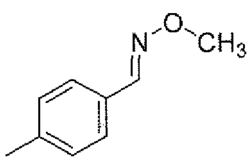 | b17 | 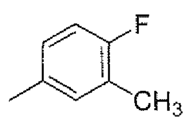 | b27 | 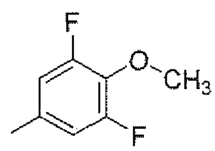 |
| b8 | 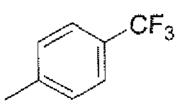 | b18 | 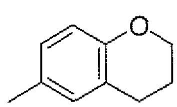 | b28 | 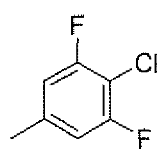 |
| b9 | 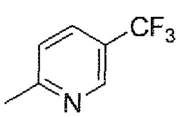 | b19 | 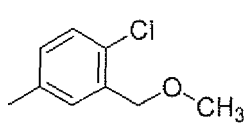 | | |
| b10 | 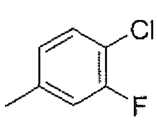 | b20 | 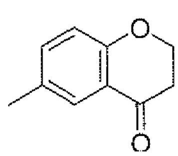 | | |

(A1,X,A2)= (a1,X1,b1),(a1,X1,b2),(a1,X1,b3),(a1,X1,b4),(a1,X1,b1),(a1,X1,b6),(a1,X1,b7),( a1,X1,b8),(a1, X1,b9),(a1,X1,b10),(a1,X1,b11),(a1,X1,b12),(a1,X1,b13),(a1,X1, b14),(a1,X1,b15),(a1,X1,b16),(a1,X1,b17),(a1,X1, b18),(a1,X1,b19),(a1,X1,b20) ,(a1,X1,b21),(a1,X1,b22),(a1,X1,b23),(a1,X1,b24),(a1,X1,b25),(a1,X1,b26),(a1, X1, b21),(a1,X1,b28),(a1,X1,b1),(a1,X2,b2),(a1,X2,b3),(a1,X2,b1),(a1,X1,b1),(a 1,X2,b6),(a1,X2,b7),(a1,X2,b8),(a1,X2, b9), (a1,X2,b10), (a1,X2,b11), (a1,X2,b12) , (al, X1,b13), (al, X1,b14), (a1,X2,b15), (a1,X2,b16), (al, X1,b17), (a1,X2,b18), (a1, X2,b19),(a1,X2,b20),(a1,X2,b21),(a1,X2,b22),(a1,X2,b23),(a1,X2,b24),(a1,X2,b 25),(a1,X2,b26),(a1,X2,b27),(a1, X2,b28),(a1,X3,b1),(a1,X3,b2),(a1,X3,b3),(a1, X3,b4),(a1,X3,b5),(a1,X3,b6),(a1,X3,b7),(a1,X3,b8),(a1,X3,b9),(a1,X3, b10),(a1 ,X3,b11),(a1,X3,b12),(a1,X3,b13),(a1,X3,b14),(a1,X3,b15),(a1,X3,b16),(a1,X3, b17),(a1,X3,b18),(a1,X3, b19),(a1,X3,b20),(a1,X3,b21),(a1,X3,b22),(a1,X3,b23) ,(a1,X3,b24),(a1,X3,b25),(a1,X3,b26),(a1,X3,b27),(a1,X3, b28),(a1,X4,b1),(a1,X 4,b2),(a1,X4,b3),(a1,X4,b4),(a1,X4,b5),(a1,X4,b6),(a1,X4,b7),(a1,X4,b8),(a1,X 4,b9),(a1,X4, b10), (a1,X4,b11), (a1,X4,b12), (a1,X4,b13) (a1,X4,b14), (a1,X4,b15 ), (a1,X4,b16), (a1,X4,b17), (a1,X4,b18), (a1,X4,b19), (a1,X4,b20), (a1,X4,b21), (a1 , X4,b22), (a1,X4,b23), (a1,X4,b24), (a1,X4,b25), (a1,X4,b26), (a1,X4,b27), (a1,X4, b28), (a1,X5,b1), (a1,X5,b2), (a1,X5,b3), (a1,X5,b4), (a1,X5,b5), (a1,X5,b6), (a1,X5 , b7), (a1,X5,b8), (a1,X5,b9), (a1,X5,b10), (a1,X5,b11), (a1,X5,b12), (a1,X5,b13), (a 1,X5,b14), (a1,X5,b15), (a1,X5,b16), (a1,X5,b17), (a1,X5,b18), (a1,X5,b19), (a1,X5 , b20), (a1,X5,b21), (a1,X5,b22), (a1,X5,b23), (a1,X5,b24), (a1,X5,b25), (a1,X5,b26 ), (a1,X5,b27), (a1,X5,b28), (al, X6,b1), (a1,X6,b2), (a1,X6,b3), (a1,X6,b4), (a1,X6, b5), (a1,X6,b6), (a1,X6,b7), (a1,X6,b8), (a1,X6,b9), (a1,X6,b10), (a1,X6,b11), (a1,X 6,b12), (a1,X6,b13), (a1,X6,b14), (a1,X6,b15), (a1,X6,b16), (al, X6,b17), (al, X6,b1 8), (a1,X6,b19), (a1,X6,b20), (a1,X6,b21), (a1,X6,b22), (a1,X6,b23), (a1,X6,b24), (a 1,X6,b25), (a1,X6,b26), (a1,X6,b27), (a1,X6,b28), (a1,X7,b1), (a1,X7,b2), (a1,X7,b 3), (a1,X7,b4), (a1,X7,b5), (a1,X7,b6), (a1,X7,b7), (a1,X7,b8), (a1,X7,b9), (a1,X7,b 10), (a1,X7,b11), (a1,X7,b12), (a1,X7,b13), (a1,X7,b14), (a1,X7,b15), (a1,X7,b16), ( a1,X7,b17), (a1,X7,b18), (a1,X7,b19) (a1,X7,b20), (a1,X7,b21), (a1,X7,b22), (a1,X 7,b23), (a1,X7,b24), (a1,X7,b25), (a1,X7,b26), (a1,X7,b27), (a1,X7,b28), (a2,X1,b1), (a2,X1,b2), (a2,X1,b3), (a2,X1,b4), (a2,X1,b5), (a2,X1,b6), (a2,X1,b7), ( a2,X1,b8), (a2,X1,b9), (a2,X1,b10), (a2,X1,b11), (a2,X1,b12), (a2,X1,b13), (a2,X1, b14), (a2,X1,b15), (a2,X1,b16), (a2,X1,b17), (a2,X1,b18), (a2,X1,b19), (a2,X1,b20) , (a2,X1,b21), (a2,X1,b22), (a2,X1,b23), (a2,X1,b24), (a2,X1,b25), (a2,X1,b26), (a2, X1,b27), (a2,X1,b28), (a2,X2,b1), (a2,X2,b2), (a2,X2,b3), (a2,X2,b4), (a2,X2,b5), (a 2,X2,b6), (a2,X2,b7), (a2,X2,b8), (a2,X2,b9), (a2,X2,b10), (a2,X2,b11), (a2,X2,b12) , (a2,X2,b13), (a2,X2,b14), (a2,X2,b15), (a2,X2,b16), (a2,X2,b17), (a2,X2,b18), (a2, X2,b19), (a2,X2,b20), (a2,X2,b21), (a2,X2,b22), (a2,X2,b23), (a2,X2,b24), (a2,X2,b 25), (a2,X2,b26), (a2,X2,b27), (a2,X2,b28), (a2,X3,b1), (a2,X3,b2), (a2,X3,b3), (a2, X3,b4), (a2,X3,b5), (a2,X3,b6), (a2,X3,b7), (a2,X3,b8), (a2,X3,b9), (a2,X3,b10), (a2 , X3,b11), (a2,X3,b12), (a2,X3,b13), (a2,X3,b14), (a2,X3,b15), (a2,X3,b16), (a2,X3, b17), (a2,X3,b18), (a2,X3,b19), (a2,X3,b20), (a2,X3,b21), (a2,X3,b22), (a2,X3,b23) , (a2,X3,b24), (a2,X3,b25), (a2,X3,b26), (a2,X3,b27), (a2,X3,b28), (a2,X4,b1), (a2,X 4,b2), (a2,X4,b3), (a2,X4,b4), (a2,X4,b5), (a2,X4,b6), (a2,X4,b7), (a2,X4,b8), (a2,X 4,b9), (a2,X4,b10), (a2,X4,b11), (a2,X4,b12), (a2,X4,b13), (a2,X4,b14) /a2,X4,b15 ), (a2,X4,b16), (a2,X4,b17), (a2,X4,b18), (a2,X4,b19), (a2,X4,b20), (a2,X4,b21), (a2 , X4,b22), (a2,X4,b23), (a2,X4,b24), (a2,X4,b25), (a2,X4,b26), (a2,X4,b27), (a2,X4, b28), (a2,X5,b1), (a2,X5,b2), (a2,X5,b3), (a2,X5,b4), (a2,X5,b5), (a2,X5,b6), (a2,X5 , b7), (a2,X5,b8), (a2,X5,b9), (a2,X5,b10), (a2,X5,b11), (a2,X5,b12), (a2,X5,b13), (a 2,X5,b14), (a2,X5,b15), (a2,X5,b16), (a2,X5,b17), (a2,X5,b18), (a2,X5,b19), (a2,X5 , b20), (a2,X5,b21), (a2,X5,b22), (a2,X5,b23), (a2,X5,b24), (a2,X5,b25), (a2,X5,b26 ), (a2,X5,b27), (a2,X5,b28), (a2,X6,b1), (a2,X6,b2), (a2,X6,b3), (a2,X6,b4), (a2,X6, b5), (a2,X6,b6), (a2,X6,b7) (a2,X6,b8), (a2,X6,b9), (a2,X6,b10) (a2,X6,b11), (a2,X 6,b12), (a2,X6,b13), (a2,X6,b14), (a2,X6,b15), (a2,X6,b16), (a2,X6,b17), (a2,X6,b1 8), (a2,X6,b19), (a2,X6,b20), (a2,X6,b21), (a2,X6,b22), (a2,X6,b23), (a2,X6,b24), (a 2,X6,b25), (a2,X6,b26), (a2,X6,b27), (a2,X6,b28), (a2,X7,b1), (a2,X7,b2), (a2,X7,b 3), (a2,X7,b4), (a2,X7,b5), (a2,X7,b6), (a2,X7,b7), (a2,X7,b8), (a2,X7,b9), (a2,X7,b 10), (a2,X7,b11), (a2,X7,b12), (a2,X7,b13), (a2,X7,b14), (a2,X7,b15), (a2,X7,b16), ( a2,X7,b17), (a2,X7,b18), (a2,X7,b19), (a2,X7,b20), (a2,X7,b21), (a2,X7,b22), (a2,X 7,b23), (a2,X7,b24), (a2,X7,b25), (a2,X7,b26), (a2,X7,b27), (a2,X7,b28), (a3,X1,b1), (a3,X1,b2), (a3,X1,b3), (a3,X1,b4), (a3,X1,b5), (a3,X1,b6), (a3,X1,b7), ( a3,X1,b8), (a3,X1,b9), (a3,X1,b10), (a3,X1,b11), (a3,X1,b12), (a3,X1,b13), (a3,X1, b14), (a3,X1,b15), (a3,X1,b16), (a3,X1,b17), (a3,X1,b18), (a3,X1,b19), (a3,X1,b20) , (a3,X1,b21), (a3,Xl, b22), (a3,X1,b23), (a3,X1,b24), (a3,X1,b25), (a3,X1,b26), (a3, X1,b27), (a3,X1,b28), (a3,X2,b1), (a3,X2,b2), (a3,X2,b3), (a3,X2,b4), (a3,X2,b5), (a 3,X2,b6), (a3,X2,b7), (a3,X2,b8), (a3,X2,b9), (a3,X2,b10), (a3,X2,b11), (a3,X2,b12) , (a3,X2,b13), (a3,X2,b14), (a3,X2,b15), (a3,X2,b16), (a3,X2,b17), (a3,X2,b1l8), (a3, X2,b19), (a3,X2,b20), (a3,X2,b21), (a3,X2,b22), (a3,X2,b23), (a3,X2,b24), (a3,X2,b 25), (a3,X2,b26), (a3,X2,b27), (a3,X2,b28), (a3,X3,b1), (a3,X3,b2), (a3,X3,b3), (a3, X3,b4), (a3,X3,b5), (a3,X3,b6), (a3,X3,b7), (a3,X3,b8), (a3,X3,b9), (a3,X3,b10), (a3 , X3,b11), (a3,X3,b12), (a3,X3,b13), (a3,X3,b14), (a3,X3,b15), (a3,X3,b16), (a3,X3, b17), (a3,X3,b18), (a3,X3,b19), (a3,X3,b20), (a3,X3,b21), (a3,X3,b22), (a3,X3,b23) , (a3,X3,b24), (a3,X3,b25), (a3,X3,b26), (a3,X3,b27), (a3,X3,b28), (a3,X4,b1), (a3,X 4,b2), (a3,X4,b3), (a3,X4,b4), (a3,X4,b5), (a3,X4,b6), (a3,X4,b7), (a3,X4,b8), (a3,X 4,b9), (a3,X4,b10), (a3,X4,b11), (a3,X4,b12), (a3,X4,b13), (a3,X4,b14), (a3,X4,b15 ), (a3,X4,b16), (a3,X4,b17), (a3,X4,b18), (a3,X4,b19), (a3,X4,b20), (a3,X4,b21), (a3 , X4,b22), (a3,X4,b23), (a3,X4,b24), (a3,X4,b25), (a3,X4,b26), (a3,X4,b27), (a3,X4, b28), (a3,X5,b1), (a3,X1,b2), (a3,X5,b3), (a3,X5,b4), (a3,X5,b5), (a3,X5,b6), (a3,X5 , b7), (a3,X5,b8), (a3,X5,b9), (a3,X5,b10), (a3,X5,b11), (a3,X5,b12), (a3,X5,b13), (a 3,X5,b14), (a3,X5,b15), (a3,X5,b16), (a3,X5,b17), (a3,X5,b18), (a3,X5,b19), (a3,X5 , b20), (a3,X5,b21), (a3,X5,b22), (a3,X5,b23), (a3,X5,b24), (a3,X1,b25), (a3,X5,b26 ), (a3,X5,b27), (a3,X5,b28), (a3,X6,b1), (a3,X6,b2), (a3,X6,b3), (a3,X6,b4), (a3,X6, b5), (a3,X6,b6), (a3,X6,b7), (a3,X6,b8), (a3,X6,b9),(a3,X6,b10), (a3,X6,b11), (a3,X 6,b12), (a3,X6,b13), (a3,X6,b14), (a3,X6,b15), (a3,X6,b16), (a3,X6,b17), (a3,X6,b1 8), (a3,X6,b19), (a3,X6,b20), (a3,X6,b21), (a3,X6,b22), (a3,X6,b23), (a3,X6,b24), (a 3,X6,b25), (a3,X6,b26), (a3,X6,b27), (a3,X6,b28), (a3,X7,b1), (a3,X7,b2), (a3,X7,b 3), (a3,X7,b4), (a3,X7,b5), (a3,X7,b6), (a3,X7,b7), (a3,X7,b8), (a3,X7,b9), (a3,X7,b 10), (a3,X7,b11), (a3,X7,b12), (a3,X7,b13), (a3,X7,b14), (a3,X7,b15), (a3,X7,b16), ( a3,X7,b17), (a3,X7,b18), (a3,X7,b19), (a3,X7,b20), (a3,X7,b21), (a3,X7,b22), (a3,X 7,b23), (a3,X7,b24), (a3,X7,b25), (a3,X7,b26), (a3,X7,b27), (a3,X7,b28), (a4,X1,b1), (a4,X1,b2), (a4,X1,b3), (a4,X1,b4), (a4,X1,b5), (a4,X1,b6), (a4,X1,b7), ( a4,X1,b8), (a4,X1,b9), (a4,X1,b10), (a4,X1,b11), (a4,X1,b12), (a4,X1,b13), (a4,X1, b14), (a4,X1,b15), (a4,X1,b16), (a4,X1,b17), (a4,X1,b18), (a4,X1,b19), (a4,X1,b20) , (a4,X1,b21), (a4,X1,b22), (a4,X1,b23), (a4,X1,b24), (a4,X1,b25), (a4,X1,b26), (a4, X1,b27), (a4,X1,b28), (a4,X2,b1), (a4,X2,b2), (a4,X2,b3), (a4,X2,b4), (a4,X2,b5), (a 4,X2,b6), (a4,X2,b7), (a4,X2,b8), (a4,X2,b9), (a4,X2,b10), (a4,X2,b11), (a4,X2,b12) , (a4,X2,b13), (a4,X2,b14), (a4,X2,b15), (a4,X2,b16), (a4,X2,b17), (a4,X2,b18), (a4, X2,b19), (a4,X2,b20), (a4,X2,b21), (a4,X2,b22), (a4,X2,b23), (a4,X2,b24), (a4,X2,b 25), (a4,X2,b26), (a4,X2,b27), (a4,X2,b28), (a4,X3,b1), (a4,X3,b2), (a4,X3,b3), (a4, X3,b4), (a4,X3,b5), (a4,X3,b6), (a4,X3,b7), (a4,X3,b8), (a4,X3,b9), (a4,X3,b10), (a4 , X3,b11), (a4,X3,b12), (a4,X3,b13), (a4,X3,b14), (a4,X3,b15), (a4,X3,b16), (a4,X3, b17), (a4,X3,b18), (a4,X3,b19), (a4,X3,b20), (a4,X3,b21), (a4,X3,b22), (a4,X3,b23) , (a4,X3,b24), (a4,X3,b25), (a4,X3,b26), (a4,X3,b27), (a4,X3,b28), (a4,X4,b1), (a4,X 4,b2), (a4,X4,b3), (a4,X4,b4), (a4,X4,b5), (a4,X4,b6), (a4,X4,b7), (a4,X4,b8), (a4,X 4,b9), (a4,X4,b10), (a4,X4,b11), (a4,X4,b12), (a4,X4,b13), (a4,X4,b14), (a4,X4,b15 ), (a4,X4,b16), (a4,X4,b17), (a4,X4,b18), (a4,X4,b19), (a4,X4,b20), (a4,X4,b21), (a4 , X4,b22), (a4,X4,b23), (a4,X4,b24), (a4,X4,b25), (a4,X4,b26), (a4,X4,b27), (a4,X4, b28), (a4,X5,b1), (a4,X5,b2), (a4,X5,b3), (a4,X5,b4), (a4,X5,b5), (a4,X5,b6), (a4,X5 , b7), (a4,X5,b8), (a4,X5,b9), (a4,X5,b10), (a4,X5,b11), (a4,X5,b12), (a4,X5,b13), (a 4,X5,b14), (a4,X5,b15), (a4,X5,b16), (a4,X5,b17), (a4,X5,b18), (a4,X5,b19), (a4,X5 , b20), (a4,X5,b21), (a4,X5,b22), (a4,X5,b23), (a4,X5,b24), (a4,X5,b25), (a4,X5,b26 ), (a4,X5,b27), (a4,X5,b28), (a4,X6,b1), (a4,X6,b2), (a4,X6,b3), (a4,X6,b4), (a4,X6, b5), (a4,X6,b6), (a4,X6,b7), (a4,X6,b8), (a4,X6,b9), (a4,X6,b10), (a4,X6,b11), (a4,X 6,b12), (a4,X6,b13), (a4,X6,b14), (a4,X6,b15), (a4,X6,b16), (a4,X6,b17), (a4,X6,b1 8), (a4,X6,b19), (a4,X6,b20), (a4,X6,b21), (a4,X6,b22), (a4,X6,b23), (a4,X6,b24), (a 4,X6,b25), (a4,X6,b26), (a4,X6,b27), (a4,X6,b28), (a4,X7,b1), (a4,X7,b2), (a4,X7,b 3), (a4,X7,b4), (a4,X7,b5), (a4,X7,b6), (a4,X7,b7), (a4,X7,b8), (a4,X7,b9), (a4,X7,b 10), (a4,X7,b11), (a4,X7,b12), (a4,X7,b13), (a4,X7,b14), (a4,X7,b15), (a4,X7,b16), ( a4,X7,b17), (a4,X7,b18), (a4,X7,b19), (a4,X7,b20), (a4,X7,b21), (a4,X7,b22), (a4,X 7,b23), (a4,X7,b24), (a4,X7,b25), (a4,X7,b26), (a4,X7,b27), (a4,X7,b28), (a5,X1,b1), (a5,X1,b2), (a5,X1,b3), (a5,X1,b4), (a5,X1,b5), (a5,X1,b6), (a5,X1,b7), ( a5,X1,b8), (a5,X1,b9), (a5,X1,b10), (a5,X1,b11), (a5,X1,b12), (a5,X1,b13), (a5,X1, b14), (a5,X1,b15), (a5,X1,b16), (a5,X1,b17), (a5,X1,b18), (a5,X1,b19), (a5,X1,b20) , (a5,X1,b21), (a5,X1,b22), (a5,X1,b23), (a5,X1,b24), (a5,X1,b25), (a5,Xl, b26), (a5, X1,b27), (a5,X1,b28), (a5,X2,b1), (a5,X2,b2), (a5,X2,b3), (a5,X2,b4), (a5,X2,b5), (a 5,X2,b6), (a5,X2,b7), (a5,X2,b8), (a5,X2,b9), (a5,X2,b10), (a5,X2,b11), (a5,X2,b12) , (a5,X2,b13), (a5,X2,b14), (a5,X2,b15), (a5,X2,b16), (a5,X2,b17), (a5,X2,b18), (a5, X2,b19), (a5,X2,b20), (a5,X2,b21), (a5,X2,b22), (a5,X2,b23), (a5,X2,b24), (a5,X2,b 25), (a5,X2,b26), (a5,X2,b27), (a5,X2,b28), (a5,X3,b1), (a5,X3,b2), (a5,X3,b3), (a5, X3,b4), (a5,X3,b5), (a5,X3,b6), (a5,X3,b7), (a5,X3,b8), (a5,X3,b9), (a5,X3,b10), (a5 , X3,b11), (a5,X3,b12), (a5,X3,b13), (a5,X3,b14), (a5,X3,b15), (a5,X3,b16), (a5,X3, b17), (a5,X3,b18), (a5,X3,b19), (a5,X3,b20), (a5,X3,b21), (a5,X3,b22), (a5,X3,b23) , (a5,X3,b24), (a5,X3,b25), (a5,X3,b26), (a5,X3,b27), (a5,X3,b28), (a5,X4,b1), (a5,X 4,b2), (a5,X4,b3), (a5,X4,b4), (a5,X4,b5), (a5,X4,b6), (a5,X4,b7), (a5,X4,b8), (a5,X 4,b9), (a5,X4,b10), (a5,X4,b11), (a5,X4,b12), (a5,X4,b13), (a5,X4,b14), (a5,X4,b15 ), (a5,X4,b16), (a5,X4,b17), (a5,X4,b18), (a5,X4,b19), (ai, X4,b20), (a5,X4,b21), (a5 , X4,b22), (a5,X4,b23), (a5,X4,b24), (a5,X4,b25), (a5,X4,b26), (a5,X4,b27), (a5,X4, b28), (a5,X5,b1), (a5,X5,b2), (a3,X5,b3), (a5,X5,b4), (a5,X1,b5), (a1,X5,b6), (a5,X5 , b7), (a1,X5,b8), (a5,X5,b9), (a5,X5,b10), (a1,X5,b11), (a5,X5,b12), (a5,X5,b13), (a 5,X5,b14), (a5,X5,b15), (a5,X5,b16), (a5,X5,b17), (a5,X5,b18), (a5,X5,b19), (a5,X5 , b20), (a5,X5,b21), (a5,X5,b22), (a5,X5,b23), (a5,X5,b24), (a5,X5,b25), (a5,X5,b26 ), (a5,X5,b27), (a5,X5,b28), (a5,X6,b1), (a5,X6,b2), (a5,X6,b3), (a5,X6,b4), (a5,X6, b5), (a5,X6,b6), (a5,X6,b7), (a5,X6,b8), (a5,X6,b9), (a5,X6,b10), (a5,X6,b11), (a5,X 6,b12), (a5,X6,b13), (a5,X6,b14), (a5,X6,b15), (a5,X6,b16), (a5,X6,b17), (a5,X6,b1 8), (a5,X6,b19), (a5,X6,b20), (a5,X6,b21), (a5,X6,b22), (a5,X6,b23), (a5,X6,b24), (a 5,X6,b25), (a5,X6,b26), (a5,X6,b27), (a5,X6,b28), (a5,X7,b1), (a5,X7,b2), (a5,X7,b 3), (a5,X7,b4), (a5,X7,b5), (a5,X7,b6), (a5,X7,b7), (a5,X7,b8), (a5,X7,b9), (a5,X7,b 10), (a5,X7,b11), (a5,X7,b12), (a5,X7,b13), (a5,X7,b14), (a5,X7,b15), (a5,X7,b16), ( a5,X7,b17), (a5,X7,b18), (a5,X7,b19), (a5,X7,b20), (a5,X7,b21), (a5,X7,b22), (a5,X 7,b23), (a5,X7,b24), (a5,X7,b25), (a5,X7,b26), (a5,X7,b27), (a5,X7,b28), (a6,X1,b1), (a6,X1,b2), (a6,X1,b3), (a6,X1,b4), (a6,X1,b5), (a6,X1,b6), (a6,X1,b7), ( a6,X1,b8), (a6,X1,b9), (a6,X1,b10), (a6,X1,b11), (a6,X1,b12), (a6,X1,b13), (a6,X1, b14), (a6,X1,b15), (a6,X1,b16), (a6,X1,b17), (a6,X1,b18), (a6,X1,b19), (a6,X1,b20) , (a6,X1,b21), (a6,X1,b22), (a6,X1,b23), (a6,X1,b24), (a6,X1,b25), (a6,X1,b26), (a6, X1,b27), (a6,X1,b28), (a6,X2,b1), (a6,X2,b2), (a6,X2,b3), (a6,X2,b4), (a6,X2,b5), (a 6,X2,b6), (a6,X2,b7), (a6,X2,b8), (a6,X2,b9), (a6,X2,b10), (a6,X2,b11), (a6,X2,b12) , (a6,X2,b13), (a6,X2,b14), (a6,X2,b15), (a6,X2,b16), (a6,X2,b17), (a6,X2,b18), (a6, X2,b19), (a6,X2,b20), (a6,X2,b21), (a6,X2,b22), (a6,X2,b23), (a6,X2,b24), (a6,X2,b 25), (a6,X2,b26), (a6,X2,b27), (a6,X2,b28), (a6,X3,b1), (a6,X3,b2), (a6,X3,b3), (a6, X3,b4), (a6,X3,b5), (a6,X3,b6), (a6,X3,b7), (a6,X3,b8), (a6,X3,b9), (a6,X3,b10), (a6 , X3,b11), (a6,X3,b12), (a6,X3,b13), (a6,X3,b14), (a6,X3,b15), (a6,X3,b16), (a6,X3, b17), (a6,X3,b18), (a6,X3,b19), (a6,X3,b20), (a6,X3,b21), (a6,X3,b22), (a6,X3,b23) , (a6,X3,b24), (a6,X3,b25), (a6,X3,b26), (a6,X3,b27), (a6,X3,b28), (a6,X4,b1), (a6,X 4,b2), (a6,X4,b3), (a6,X4,b4), (a6,X4,b5), (a6,X4,b6), (a6,X4,b7), (a6,X4,b8), (a6,X 4,b9), (a6,X4,b10), (a6,X4,b11), (a6,X4,b12), (a6,X4,b13), (a6,X4,b14), (a6,X4,b15 ), (a6,X4,b16), (a6,X4,b17), (a6,X4,b18),(a6,X4,b19), (a6,X4,b20), (a6,X4,b21), (a6 , X4,b22), (a6,X4,b23), (a6,X4,b24), (a6,X4,b25), (a6,X4,b26), (a6,X4,b27), (a6,X4, b28), (a6,X5,b1), (a6,X5,b2), (a6,X5,b3), (a6,X5,b4), (a6,X5,b5), (a6,X5,b6), (a6,X5 , b7), (a6,X5,b8), (a6,X5,b9), (a6,X5,b10), (a6,X5,b11), (a6,X5,b12), (a6,X5,b13), (a 6,X5,b14), (a6,X5,b15), (a6,X5,b16), (a6,X5,b17), (a6,X5,b18), (a6,X5,b19), (a6,X5 , b20), (a6,X5,b21), (a6,X5,b22), (a6,X5,b23), (a6,X5,b24), (a6,X5,b25), (a6,X5,b26 ), (a6,X5,b27), (a6,X5,b28), (a6,X6,b1), (a6,X6,b2), (a6,X6,b3), (a6,X6,b4), (a6,X6, b5), (a6,X6,b6), (a6,X6,b7), (a6,X6,b8), (a6,X6,b9), (a6,X6,b10), (a6,X6,b11), (a6,X 6,b12), (a6,X6,b13), (a6,X6,b14), (a6,X6,b15), (a6,X6,b16), (a6,X6,b17), (a6,X6,b1 8), (a6,X6,b19), (a6,X6,b20), (a6,X6,b21), (a6,X6,b22), (a6,X6,b23), (a6,X6,b24), (a 6,X6,b25), (a6,X6,b26), (a6,X6,b27), (a6,X6,b28), (a6,X7,b), (a6,X7,b2), (a6,X7,b 3), (a6,X7,b4), (a6,X7,b5), (a6,X7,b6), (a6,X7,b7), (a6,X7,b8), (a6,X7,b9), (a6,X7,b 10), (a6,X7,b11), (a6,X7,b12), (a6,X7,b13), (a6,X7,b14), (a6,X7,b10), (a6,X7,b16), ( a6,X7,b17), (a6,X7,b18), (a6,X7,b19), (a6,X7,b20), (a6,X7,b21), (a6,X7,b22), (a6,X 7,b23), (a6,X7,b24), (a6,X7,b25), (a6,X7,b26), (a6,X7,b27), (a6,X7,b28), (a7,X1,b1), (a7,X1,b2), (a7,X1,b3), (a7,X1,b4), (a7,X1,b5), (a7,X1,b6), (a7,X1,b7), ( a7,X1,b8), (a7,X1,b9), (a7,X1,b10), (a7,X1,b11), (a7,X1,b12), (a7,X1,b13), (a7,X1, b14), (a7,X1,b15), (a7,X1,b16), (a7,X1,b17), (a7,X1,b18), (a7,X1,b19), (a7,X1,b20) , (a7,X1,b21), (a7,X1,b22), (a7,X1,b23), (a7,X1,b24), (a7,X1,b25), (a7,X1,b26), (a7, X1,b27), (a7,X1,b28), (a7,X2,b1), (a7,X2,b2), (a7,X2,b3), (a7,X2,b4), (a7,X2,b5), (a 7,X2,b6), (a7,X2,b7), (a7,X2,b8), (a7,X2,b9), (a7,X2,b10), (a7,X2,b11), (a7,X2,b12) , (a7,X2,b13), (a7,X2,b14), (a7,X2,b15), (a7,X2,b16), (a7,X2,b17), (a7,X2,b18), (a7, X2,b19), (a7,X2,b20), (a7,X2,b21), (a7,X2,b22), (a7,X2,b23), (a7,X2,b24), (a7,X2,b 25), (a7,X2,b26), (a7,X2,b27), (a7,X2,b28), (a7,X3,b1), (a7,X3,b2), (a7,X3,b3), (a7, X3,b4), (a7,X3,b5), (a7,X3,b6), (a7,X3,b7), (a7,X3,b8), (a7,X3,b9), (a7,X3,b10), (a7 , X3,b11), (a7,X3,b12), (a7,X3,b13), (a7,X3,b14), (a7,X3,b15), (a7,X3,b16), (a7,X3, b17), (a7,X3,b18), (a7,X3,b19), (a7,X3,b20), (a7,X3,b21), (a7,X3,b22), (a7,X3,b23) , (a7,X3,b24), (a7,X3,b25), (a7,X3,b26), (a7,X3,b27), (a7,X3,b28), (a7,X4,b1), (a7,X 4,b2), (a7,X4,b3), (a7,X4,b4), (a7,X4,b5), (a7,X4,b6), (a7,X4,b7), (a7,X4,b8), (a7,X 4,b9), (a7,X4,b10), (a7,X4,b11), (a7,X4,b12), (a7,X4,b13), (a7,X4,b14), (a7,X4,b15 ), (a7,X4,b16), (a7,X4,b17), (a7,X4,b18), (a7,X4,b19), (a7,X4,b20), (a7,X4,b21), (a7 , X4,b22), (a7,X4,b23), (a7,X4,b24), (a7,X4,b25), (a7,X4,b26), (a7,X4,b27), (a7,X4, b28), (a7,X5,b1), (a7,X5; b2), (a7,X5,b3), (a7,X5,b4), (a7,X5,b5), (a7,X5,b6), (a7,X5 , b7), (a7,X5,b8), (a7,X5,b9), (a7,X5,b10), (a7,X5,b11), (a7,X5,b12), (a7,X5,b13), (a 7,X5,b14), (a7,X5,b15), (a7,X5,b16), (a7,X5,b17), (a7,X5,b18), (a7,X5,b19), (a7,X5 , b20), (a7,X5,b21), (a7,X5,b22), (a7,X5,b23), (a7,X5,b24), (a7,X5,b25) (a7,X5,b26 ), (a7,X5,b27), (a7,X5,b28), (a7,X6,b1), (a7,X6,b2), (a7,X6,b3), (a7,X6,b4), (a7,X6, b5), (a7,X6,b6), (a7,X6,b7), (a7,X6,b8), (a7,X6,b9), (a7,X6,b10), (a7,X6,b11), (a7,X 6,b12), (a7,X6,b13), (a7,X6,b14), (a7,X6,b15), (a7,X6,b16), (a7,X6,b17), (a7,X6,b1 8), (a7,X6,b19), (a7,X6,b20), (a7,X6,b21), (a7,X6,b22), (a7,X6,b23), (a7,X6,b24), (a 7,X6,b25), (a7,X6,b26), (a7,X6,b27), (a7,X6,b28), (a7,X7,b1), (a7,X7,b2), (a7,X7,b 3), (a7,X7,b4), (a7,X7,b5), (a7,X7,b6), (a7,X7,b7), (a7,X7,b8), (a7,X7,b9), (a7,X7,b 10), (a7,X7,b11), (a7,X7,b12), (a7,X7,b13), (a7,X7,b14), (a7,X7,b15), (a7,X7,b16), ( a7,X7,b17), (a7,X7,b18), (a7,X7,b19), (a7,X7,b20), (a7,X7,b21), (a7,X7,b22), (a7,X 7,b23), (a7,X7,b24), (a7,X7,b25), (a7,X7,b26), (a7,X7,b27), (a7,X7,b28), (a8,X1,b1), (a8,X1,b2), (a8,X1,b3), (a8,X1,b4), (a8,X1,b5), (a8,X1,b6), (a8,X1,b7), ( a8,X1,b8), (a8,X1,b9), (a8,X1,b10), (a8,X1,b11), (a8,X1,b12), (a8,X1,b13), (a8,X1, b14), (a8,X1,b15), (a8,X1,b16), (a8,X1,b17), (a8,X1,b18), (a8,X1,b19), (a8,X1,b20) , (a8,X1,b21), (a8,X1,b22), (a8,X1,b23), (a8,X1,b24), (a8,X1,b25), (a8,X1,b26), (a8, X1,b27), (a8,X1,b28), (a8,X2,b1), (a8,X2,b2), (a8,X2,b3), (a8,X2,b4), (a8,X2,b5), (a 8,X2,b6), (a8,X2,b7), (a8,X2,b8), (a8,X2,b9), (a8,X2,b10), (a8,X2,b11), (a8,X2,b12) , (a8,X2,b13), (a8,X2,b14), (a8,X2,b15), (a8,X2,b16), (a8,X2,b17), (a8,X2,b18), (a8, X2,b19), (a8,X2,b20), (a8,X2,b21), (a8,X2,b22), (a8,X2,b23), (a8,X2,b24), (a8,X2,b 25), (a8,X2,b26), (a8,X2,b27), (a8,X2,b28), (a8,X3,b1), (a8,X3,b2), (a8,X3,b3), (a8, X3,b4), (a8,X3,b5), (a8,X3,b6), (a8,X3,b7), (a8,X3,b8), (a8,X3,b9), (a8,X3,b10), (a8 , X3,b11), (a8,X3,b12), (a8,X3,b13), (a8,X3,b14), (a8,X3,b15), (a8,X3,b16), (a8,X3, b17), (a8,X3,b18), (a8,X3,b19), (a8,X3,b20), (a8,X3,b21), (a8,X3,b22), (a8,X3,b23) , (a8,X3,b24), (a8,X3,b25), (a8,X3,b26), (a8,X3,b27), (a8,X3,b28), (a8,X4,b1), (a8,X 4,b2), (a8,X4,b3), (a8,X4,b4), (a8,X4,b5), (a8,X4,b6), (a8,X4,b7), (a8,X4,b8), (a8,X 4,b9), (a8,X4,b10), (a8,X4,b11), (a8,X4,b12), (a8,X4,b13), (a8,X4,b14), (a8,X4,b15 ), (a8,X4,b16), (a8,X4,b17), (a8,X4,b18), (a8,X4,b19), (a8,X4,b20), (a8,X4,b21), (a8 , X4,b22), (a8,X4,b23), (a8,X4,b24), (a8,X4,b25), (a8,X4,b26), (a8,X4,b27), (a8,X4, b28), (a8,X5,b1), (a8,X5,b2), (a8,X5,b3), (a8,X5,b4), (a8,X5,b5), (a8,X5,b6), (a8,X5 , b7), (a8,X5,b8), (a8,X5,b9), (a8,X5,b10), (a8,X5,b11), (a8,X5,b12), (a8,X5,b13), (a 8,X5,b14), (a8,X5,b15), (a8,X5,b16), (a8,X5,b17), (a8,X5,b18), (a8,X5,b19), (a8,X5 , b20), (a8,X5,b21), (a8,X5,b22), (a8,X5,b23), (a8,X5,b24), (a8,X5,b25), (a8,X5,b26 ), (a8,X5,b27), (a8,X5,b28), (a8,X6,b1), (a8,X6,b2), (a8,X6,b3), (a8,X6,b4), (a8,X6, b5), (a8,X6,b6), (a8,X6,b7), (a8,X6,b8), (a8,X6,b9), (a8,X6,b10), (a8,X6,b11), (a8,X 6,b12), (a8,X6,b13), (a8,X6,b14), (a8,X6,b15), (a8,X6,b16), (a8,X6,b17), (a8,X6,b1 8), (a8,X6,b19), (a8,X6,b20), (a8,X6,b21), (a8,X6,b22), (a8,X6,b23), (a8,X6,b24), (a 8,X6,b25), (a8,X6,b26), (a8,X6,b27), (a8,X6,b28), (a8,X7,b1), (a8,X7,b2), (a8,X7,b 3), (a8,X7,b4), (a8,X7,b5), (a8,X7,b6), (a8,X7,b7), (a8,X7,b8), (a8,X7,b9), (a8,X7,b 10), (a8,X7,b11), (a8,X7,b12), (a8,X7,b13), (a8,X7,b14), (a8,X7,b15), (a8,X7,b16), ( a8,X7,b17), (a8,X7,b18), (a8,X7,b19), (a8,X7,b20), (a8,X7,b21), (a8,X7,b22), (a8,X 7,b23), (a8,X7,b24), (a8,X7,b25), (a8,X7,b26), (a8,X7,b27), (a8,X7,b28), (a9,X1,b1), (a9,X1,b2), (a9,X1,b3), (a9,X1,b4), (a9,X1,b5), (a9,X1,b6), (a9,X1,b7), ( a9,X1,b8), (a9,X1,b9), (a9,X1,b10), (a9,X1,b11), (a9,X1,b12), (a9,X1,b13), (a9,X1, b14), (a9,X1,b15), (a9,X1,b16), (a9,X1,b17), (a9,X1,b18), (a9,X1,b19), (a9,X1,b20) , (a9,X1,b21), (a9,X1,b22), (a9,X1,b23), (a9,X1,b24), (a9,X1,b25), (a9,X1,b26), (a9, X1,b27), (a9,X1,b28), (a9,X2,b1), (a9,X2,b2), (a9,X2,b3), (a9,X2,b4), (a9,X2,b5), (a 9,X2,b6), (a9,X2,b7), (a9,X2,b8), (a9,X2,b9), (a9,X2,b10), (a9,X2,b11), (a9,X2,b12) , (a9,X2,b13), (a9,X2,b14), (a9,X2,b15), (a9,X2,b16), (a9,X2,b17), (a9,X2,b18),(a9, X2,b19), (a9,X2,b20), (a9,X2,b21), (a9,X2,b22), (a9,X2,b23), (a9,X2,b24), (a9,X2,b 25), (a9,X2,b26), (a9,X2,b27), (a9,X2,b28), (a9,X3,b1), (a9,X3,b2), (a9,X3,b3), (a9, X3,b4), (a9,X3,b5), (a9,X3,b6), (a9,X3,b7), (a9,X3,b8), (a9,X3,b9), (a9,X3,b10), (a9 , X3,b11), (a9,X3,b12), (a9,X3,b13), (a9,X3,b14), (a9,X3,b15), (a9,X3,b16), (a9,X3, b17), (a9,X3,b18), (a9,X3,b19), (a9,X3,b20), (a9,X3,b21), (a9,X3,b22), (a9,X3,b23) , (a9,X3,b24), (a9,X3,b25), (a9,X3,b26), (a9,X3,b27), (a9,X3,b28), (a9,X4,b1), (a9,X 4,b2), (a9,X4,b3), (a9,X4,b4), (a9,X4,b5), (a9,X4,b6), (a9,X4,b7), (a9,X4,b8), (a9,X 4,b9), (a9,X4,b10), (a9,X4,b11), (a9,X4,b12), (a9,X4,b13), (a9,X4,b14), (a9,X4,b15 ), (a9,X4,b16), (a9,X4,b17), (a9,X4,b18), (a9,X4,b19), (a9,X4,b20), (a9,X4,b21), (a9 , X4,b22), (a9,X4,b23), (a9,X4,b24), (a9,X4,b25), (a9,X4,b26), (a9,X4,b27), (a9,X4, b28), (a9,X5,b1), (a9,X5,b2), (a9,X5,b3), (a9,X5,b4), (a9,X5,b5), (a9,X5,b6), (a9,X5 , b7), (a9,X5,b8), (a9,X5,b9), (a9,X5,b10), (a9,X5,b11), (a9,X5,b12), (a9,X5,b13), (a 9,X5,b14), (a9,X5,b15), (a9,X5,b16), (a9,X5,b17), (a9,X5,b18), (a9,X5,b19), (a9,X5 , b20), (a9,X5,b21), (a9,X5,b22), (a9,X5,b23), (a9,X5,b24), (a9,X5,b25), (a9,X5,b26 ), (a9,X5,b27), (a9,X5,b28), (a9,X6,b1), (a9,X6,b2), (a9,X6,b3), (a9,X6,b4), (a9,X6, b5), (a9,X6,b6), (a9,X6,b7), (a9,X6,b8), (a9,X6,b9), (a9,X6,b10), (a9,X6,b11), (a9,X 6,b12), (a9,X6,b13), (a9,X6,b14), (a9,X6,b15), (a9,X6,b16), (a9,X6,b17), (a9,X6,b1 8), (a9,X6,b19), (a9,X6,b20), (a9,X6,b21), (a9,X6,b22), (a9,X6,b23), (a9,X6,b24), (a 9,X6,b25), (a9,X6,b26), (a9,X6,b27), (a9,X6,b28), (a9,X7,b1), (a9,X7,b2), (a9,X7,b 3), (a9,X7,b4), (a9,X7,b5), (a9,X7,b6), (a9,X7,b7), (a9,X7,b8), (a9,X7,b9), (a9,X7,b 10), (a9,X7,b11), (a9,X7,b12), (a9,X7,b13), (a9,X7,b14), (a9,X7,b15), (a9,X7,b16), ( a9,X7,b17), (a9,X7,b18), (a9,X7,b19), (a9,X7,b20), (a9,X7,b21), (a9,X7,b22), (a9,X 7,b23), (a9,X7,b24), (a9,X7,b25), (a9,X7,b26), (a9,X7,b27), (a9,X7,b28), (a10,X1,b1), (a10,X1,b2), (a10,X1,b3), (a10,X1,b4), (a10,X1,b5), (a10,X1,b6), (a10 , X1,b7), (a10,X1,b8), (a10,X1,b9), (a10,X1,b10), (a10,X1,b11), (a10,X1,b12), (a10, X1,b13), (a10,X1,b14), (a10,X1,b15), (a10,X1,b16), (a10,X1,b17), (a10,X1,b18), (a 10,X1,b19), (a10,X1,b20), (a10,X1,b21), (a10,X1,b22), (a10,X1,b23), (a10,X1,b24) , (a10,X1,b25), (a10,X1,b26), (a10,X1,b27), (a10,X1,b28), (a10,X2,b1), (a10,X2,b2 ), (a10,X2,b3), (a10,X2,b4), (a10,X2,b5), (a10,X2,b6), (a10,X2,b7), (a10,X2,b8), (a1 0,X2,b9), (a10,X2,b10), (a10,X2,b11), (a10,X2,b12), (a10,X2,b13), (a10,X2,b14), (a 10,X2,b15), (a10,X2,b16), (a10,X2,b17), (a10,X2,b18), (a10,X2,b19), (a10,X2,b20) , (a10,X2,b21), (a10,X2,b22), (a10,X2,b23), (a10,X2,b24), (a10,X2,b25), (a10,X2,b 26), (a10,X2,b27), (a10,X2,b28), (a10,X3,b1), (a10,X3,b2), (a10,X3,b3), (a10,X3,b 4), (a10,X3,b5), (a10,X3,b6), (a10,X3,b7), (a10,X3,b8), (a10,X3,b9), (a10,X3,b10), ( a10,X3,b11), (a10,X3,b12), (a10,X3,b13), (a10,X3,b14), (a10,X3,b15), (a10,X3,b1 6), (a10,X3,b17), (a10,X3,b18), (a10,X3,b19), (a10,X3,b20), (a10,X3,b21), (a10,X3 , b22), (a10,X3,b23), (a10,X3,b24), (a10,X3,b25), (a10,X3,b26), (a10,X3,b27), (a10, X3,b28), (a10,X4,b1), (a10,X4,b2), (a10,X4,b3), (a10,X4,b4), (a10,X4,b5), (a10,X4, b6), (a10,X4,b7), (a10,X4,b8), (a10,X4,b9), (a10,X4,b10), (a10,X4,b11), (a10,X4,b1 2), (a10,X4,b13), (a10,X4,b14), (a10,X4,b15), (a10,X4,b16), (a10,X4,b17), (a10,X4 , b18), (a10,X4,b19), (a10,X4,b20), (a10,X4,b21), (a10,X4,b22), (a10,X4,b23), (a10, X4,b24), (a10,X4,b25), (a10,X4,b26), (a10,X4,b27), (a10,X4,b28), (a10,X5,b1), (a1 0,X5,b2), (a10,X5,b3), (a10,X5,b4), (a10,X5,b5), (a10,X5,b6), (a10,X5,b7), (a10,X5 , b8), (a10,X5,b9), (a10,X5,b10), (a10,X5,b11), (a10,X5,b12), (a10,X5,b13), (a10,X5 , b14), (a10,X5,b15), (a10,X5,b16), (a10,X5,b17), (a10,X5,b18), (a10,X5,b19), (a10, X5,b20), (a10,X5,b21), (a10,X5,b22), (a10,X5,b23), (a10,X5,b24), (a10,X5,b25), (a 10,X5,b26), (a10,X5,b27), (a10,X5,b28), (a10,X6,b1), (a10,X6,b2), (a10,X6,b3), (a 10,X6,b4), (a10,X6,b5), (a10,X6,b6), (a10,X6,b7), (a10,X6,b8), (a10,X6,b9), (a10,X 6,b10), (a10,X6,b11), (a10,X6,b12), (a10,X6,b13), (a10,X6,b14), (a10,X6,b15), (a1 0,X6,b18), (a10,X6,b17), (a10,X6,b18), (a10,X6,b19), (a10,X6,b20), (a10,X6,b21), (a10,X6,b22), (a10,X6,b23), (a10,X6,b24), (a10,X6,b25), (a10,X6,b26), (a10,X6,b2 7), (a10,X6,b28), (a10,X7,b1), (a10,X7,b2), (a10,X7,b3), (a10,X7,b4), (a10,X7,b5), ( a10,X7,b6), (a10,X7,b7), (a10,X7,b8), (a10,X7,b9), (a10,X7,b10), (a10,X7,b11), (a1 0,X7,b12), (a10,X7,b13), (a10,X7,b14), (a10,X7,b15), (a10,X7,b16), (a10,X7,b17), (a10,X7,b18), (a10,X7,b19), (a10,X7,b20), (a10,X7,b21), (a10,X7,b22), (a10,X7,b2 3), (a10,X7,b24), (a10,X7,b25), (a10,X7,b26), (a10,X7,b27), (a10,X7,b28), (a11,X1,b1), (a11,X1,b2), (a11,X1,b3), (a11,X1,b4), (a11,X1,b5), (a11,X1,b6), (a11, X1,b7), (a11,X1,b8), (a11,X1,b9), (a11,X1,b10), (a11,X1,b11), (a11,X1,b12), (a11,X 1,b13), (a11,X1,b14), (a11,X1,b15), (a11,X1,b16), (a11,X1,b17), (a11,X1,b1a), (a11 , X1,b19), (a11,X1,b20), (a11,X1,b21), (a11,X1,b22), (a11,X1,b23), (a11,X1,b24), (a 11,X1,b25), (a11,X1,b26), (a11,X1,b27), (a11,X1,b28), (a11,X2,b1), (a11,X2,b2), (a 11,X2,b8), (a11,X2,b4), (a11,X2,b5), (a11,X2,b6), (a11,X2,b7), (a1,X2,b8), (a11,X 2,b9), (a11,X2,b10), (a11,X2,b11), (a11,X2,b12), (a11,X2,b13), (a11,X2,b14), (a11, X2,b15), (a11,X2,b16), (a11,X2,b17), (a11,X2,b18), (a11,X2,b19), (a11,X2,b20), (a1 1,X2,b21), (a11,X2,b22), (a11,X2,b23), (a11,X2,b24), (a11,X2,b25), (a11,X2,b26), ( a11,X2,b27), (a11,X2,b28), (a11,X3,b1), (a11,X3,b2), (a11,X3,b3), (a11,X3,b4), (a1 1,X3,b5), (a11,X3,b6), (a11,X3,b7), (a11,X3,b8), (a11,X3,b9), (a11,X3,b10), (a11,X 3,b11), (a11,X3,b12), (a11,X3,b13), (a11,X3,b14), (a11,X3,b15), (a11,X3,b16), (a11 , X3,b17), (a11,X3,b18), (a11,X3,b19), (a11,X3,b20), (a11,X3,b21), (a11,X3,b22), (a 11,X3,b23), (a11,X3,b24), (a11,X3,b25), (a11,X3,b26), (a11,X3,b27), (a11,X3,b28), (a11,X4,b1), (a11,X4,b2), (a11,X4,b3), (a11,X4,b4), (a11,X4,b5), (a11,X4,b6), (a11, X4,b7), (a11,X4,b8), (a11,X4,b9), (a11,X4,b10), (a11,X4,b11), (a11,X4,b12), (a11,X 4,b13), (a11,X4,b14), (a11,X4,b15), (a11,X4,b16), (a11,X4,b17), (a11,X4,b18), (a11 , X4,b19), (a11,X4,b20), (a11,X4,b21), (a11,X4,b22), (a11,X4,b23), (a11,X4,b24), (a 11,X4,b25), (a11,X4,b26), (a11,X4,b27), (a11,X4,b28), (a11,X5,b1), (a11,X5,b2), (a 11,X5,b3), (a11,X5,b4), (a11,X5,b5), (a11,X5,b6), (a11,X5,b7), (a11,X5,b8), (a11,X 5,b9), (a11,X5,b10), (a11,X5,b11), (a11,X5,b12), (a11,X5,b13), (a11,X5,b14), (a11, X5,b15), (a11,X5,b16), (a11,X5,b17), (a11,X5,b18), (a11,X5,b19), (a11,X5,b20), (a1 1,X5,b21), (a11,X5,b22), (a11,X5,b23), (a11,X5,b24), (a11,X5,b25), (a11,X1,b26), ( a11,X5,b27), (a11,X5,b28), (a11,X6,b1), (a11,X6,b2), (a11,X6,b3), (a11,X6,b4), (a1 1,X6,b5), (a11,X6,b6),(a11,X6,b7), (a11,X6,b8), (a11,X6,b9), (a11,X6,b10), (a11,X 6,b11), (a11,X6,b12), (a11,X6,b13), (a11,X6,b14), (a11,X6,b15), (a11,X6,b16), (a11 , X6,b17), (a11,X6,b18), (a11,X6,b19), (a11,X6,b20), (a11,X6,b21), (a11,X6,b22), (a 11,X6,b23), (a11,X6,b24), (a11,X6,b25), (a11,X6,b26), (a11,X6,b27), (a11,X6,b28), (a11,X7,b1), (a11,X7,b2), (a11,X7,b3), (a11,X7,b4), (a11,X7,b5), (a11,X7,b6), (a11, X7,b7), (a11,X7,b8), (a11,X7,b9), (a11,X7,b10), (a11,X7,b11), (a11,X7,b12), (a11,X 7,b13), (a11,X7,b14), (a11,X7,b15), (a11,X7,b16), (a11,X7,b17), (a11,X7,b18), (a11 , X7,b19), (a11,X7,b20), (a11,X7,b21), (a11,X7,b22), (a11,X7,b23), (a11,X7,b24), (a 11,X7,b25), (a11,X7,b26), (a11,X7,b27), (a11,X7,b28), (a12,X1,b1), (a12,X1,b2), (a12,X1,b3), (a12,X1,b4), (a12,X1,b5), (a12,X1,b6), (a12 , X1,b7), (a12,X1,b8), (a12,X1,b9), (a12,X1,b10), (a12,X1,b11), (a12,X1,b12), (a12, X1,b13), (a12,X1,b14), (a12,X1,b15), (a12,X1,b16), (a12,X1,b17), (a12,X1,b18), (a 12,X1,b19), (a12,X1,b20), (a12,X1,b21), (a12,X1,b22), (a12,X1,b23), (a12,X1,b24) , (a12,X1,b25), (a12,X1,b26), (a12,X1,b27), (a12,X1,b28), (a12,X2,b1), (a12,X2,b2 ), (a12,X2,b3), (a12,X2,b4), (a12,X2,b5), (a12,X2,b6), (a12,X2,b7), (a12,X2,b8), (a1 2,X2,b9), (a12,X2,b10), (a12,X2,b11), (a12,X2,b12), (a12,X2,b13), (a12,X2,b14), (a 12,X2,b15), (a12,X2,b1b), (a12,X2,b17), (a12,X2,b18), (a12,X2,b19), (a12,X2,b20) , (a12,X2,b21), (a12,X2,b22), (a12,X2,b23), (a12,X2,b24), (a12,X2,b25), (a12,X2,b 26), (a12,X2,b27), (a12,X2,b28), (a12,X3,b1), (a12,X3,b2), (a12,X3,b3), (a12,X3,b 4), (a12,X3,b5), (a12,X3,b6), (a12,X3,b7), (a12,X3,b8), (a12,X3,b9), (a12,X3,b10), ( a12,X3,b11), (a12,X3,b12), (a12,X3,b13), (a12,X3,b14), (a12,X3,b15), (a12,X3,b1 6), (a12,X3,b17), (a12,X3,b18), (a12,X3,b19), (a12,X3,b20), (a12,X3,b21), (a12,X3 , b22), (a12,X3,b23), (a12,X3,b24), (a12,X3,b25), (a12,X3,b26), (a12,X3,b27), (a12, X3,b28), (a12,X4,b1), (a12,X4,b2), (a12,X4,b3), (a12,X4,b4), (a12,X4,b5), (a12,X4, b6), (a12,X4,b7), (a12,X4,b8), (a12,X4,b9), (a12,X4,b10), (a12,X4,b11), (a12,X4,b1 2), (a12,X4,b13), (a12,X4,b14), (a12,X4,b15), (a12,X4,b16), (a12,X4,b17), (a12,X4 , b18), (a12,X4,b19), (a12,X4,b20), (a12,X4,b21), (a12,X4,b22), (a12,X4,b23), (a12, X4,b24), (a12,X4,b25), (a12,X4,b26), (a12,X4,b27), (a12,X4,b28), (a12,X5,b1), (a1 2,X5,b2), (a12,X5,b3), (a12,X5,b4), (a12,X5,b5), (a12,X5,b6), (a12,X5,b7), (a12,X5 , b8), (a12,X5,b9), (a12,X5,b10), (a12,X5,b11), (a12,X5,b12), (a12,X5,b13), (a12,X5 , b14), (a12,X5,b15), (a12,X5,b16), (a12,X5,b17), (a12,X5,b18), (a12,X5,b19), (a12, X5,b20), (a12,X5,b21), (a12,X5,b22), (a12,X5,b23), (a12,X5,b24), (a12,X5,b25), (a 12,X5,b26), (a12,X5,b27), (a12,X5,b28), (a12,X6,b1), (a12,X6,b2), (a12,X6,b3), (a 12,X6,b4), (a12,X6,b5), (a12,X6,b6), (a12,X6,b7), (a12,X6,b8), (a12,X6,b9), (a12,X 6,b10), (a12,X6,b11), (a12,X6,b12), (a12,X6,b13), (a12,X6,b14), (a12,X6,b15), (a1 2,X6,b16), (a12,X6,b17), (a12,X6,b18), (a12,X6,b19), (a12,X6,b20), (a12,X6,b21), (a12,X6,b22), (a12,X6,b23), (a12,X6,b24), (a12,X6,b25), (a12,X6,b26), (a12,X6,b2 7), (a12,X6,b28), (a12,X7,b1), (a12,X7,b2), (a12,X7,b3), (a12,X7,b4), (a12,X7,b5), ( a12,X7,b6), (a12,X7,b7), (a12,X7,b8), (a12,X7,b9), (a12,X7,b10), (a12,X7,b11), (a1 2,X7,b12), (a12,X7,b13), (a12,X7,b14), (a12,X7,b15), (a12,X7,b16), (a12,X7,b17), (a12,X7,b18), (a12,X7,b19), (a12,X7,b20), (a12,X7,b21), (a12,X7,b22), (a12,X7,b2 3), (a12,X7,b24), (a12,X7,b25), (a12,X7,b26), (a12,X7,b27), (a12,X7,b28), (a13,X1,b1), (a13,x1,b2), (a13,X1,b3), (a13,X1,b4), (a13,X1,b5), (a13,X1,b6), (a13 , X1,b7), (a13,X1,b8), (a13,X1,b9), (a13,X1,b10), (a13,X1,b11), (a13,X1,b12), (a13, X1,b13), (a13,X1,b14), (a13,X1,b15), (a13,X1,b16), (a13,X1,b17), (a13,X1,b18), (a 13,X1,b19), (a13,X1,b20), (a13,X1,b21), (a13,X1,b22), (a13,X1,b23), (a13,X1,b24) , (a13,X1,b25), (a13,X1,b26), (a13,X1,b27), (a13,X1,b28), (a13,X2,b1), (a13,X2,b2 ), (a13,X2,b3), (a13,X2,b4), (a13,X2,b5), (a13,X2,b6), (a13,X2,b7), (a13,X2,b8), (a1 3,X2,b9), (a13,X2,b10), (a13,X2,b11), (a13,X2,b12), (a13,X2,b13), (a13,X2,b14), (a 13,X2,b15), (a13,X2,b16), (a13,X2,b17), (a13,X2,b18), (a13,X2,b19), (a13,X2,b20) , (a13,X2,b21), (a13,X2,b22), (a13,X2,b23), (a13,X2,b24), (a13,X2,b25), (a13,X2,b 26), (a13,X2,b27), (a13,X2,b28), (a13,X3,b1), (a13,X3,b2), (a13,X3,b3), (a13,X3,b 4), (a13,X3,b5), (a13,X3,b6), (a13,X3,b7), (a13,X3,b8), (a13,X3,b9), (a13,X3,b10), ( a13,X3,b11), (a13,X3,b12), (a13,X3,b13), (a13,X3,b14), (a13,X3,b15), (a13,X3,b1 6), (a13,X3,b17), (a13,X3,b18), (a13,X3,b19), (a13,X3,b20), (a13,X3,b21), (a13,X3 , b22), (a13,X3,b23), (a13,X3,b24), (a13,X3,b25), (a13,X3,b26), (a13,X3,b27), (a13, X3,b28), (a13,X4,b1), (a13,X4,b2), (a13,X4,b3), (a13,X4,b4), (a13,X4,b5), (a13,X4, b6), (a13,X4,b7), (a13,X4,b8), (a13,X4,b9), (a13,X4,b10), (a13,X4,b11), (a13,X4,b1 2), (a13,X4,b13), (a13,X4,b14), (a13,X4,b15), (a13,X4,b16), (a13,X4,b17), (a13,X4 , b18), (a13,X4,b19), (a13,X4,b20), (a13,X4,b21), (a13,X4,b22), (a13,X4,b23), (a13, X4,b24), (a13,X4,b25), (a13,X4,b26), (a13,X4,b27), (13,X4,b28), (a13,X5,b1), (a1 3,X5,b2), (a13,X5,b3), (a13,X5,b4), (a13,X5,b5), (a13,X5,b6), (a13,X5,b7), (a13,X5 , b8), (a13,X5,b9), (a13,X5,b10), (a13,X5,b11), (a13,X5,b12), (a13,X5,b13), (a13,X5 , b14), (a13,X5,b15), (a13,X5,b16), (a13,X5,b17), (a13,X5,b18), (a13,X5,b19), (a13, X5,b20), (a13,X5,b21), (a13,X5,b22), (a13,X5,b23), (a13,X5,b24), (a13,X5,b25), (a 13,X5,b26), (a13,X5,b27), (a13,X5,b28), (a13,X6,b1), (a13,X6,b2), (a13,X6,b3), (a 13,X6,b4), (a13,X6,b5), (a13,X6,b6), (a13,X6,b7), (a13,X6,b8), (a13,X6,b9), (a13,X 6,b10), (a13,X6,b11), (a13,X6,b12), (a13,X6,b13), (a13,X6,b14), (a13,X6,b10), (a1 3,X6,b16), (a13,X6,b17), (a13,X6,b18), (a13,X6,b19), (a13,X6,b20), (a13,X6,b21), (a13,X6,b22), (a13,X6,b23), (a13,X6,b24), (a13,X6,b25), (a13,X6,b26), (a13,X6,b2 7), (a13,X6,b28), (a13,X7,b1), (a13,X7,b2), (a13,X7,b3), (a13,X7,b4), (a13,X7,b5), ( a13,X7,b6), (a13,X7,b7), (a13,X7,b8), (a13,X7,b9), (a13,X7,b10), (a13,X7,b11), (a1 3,X7,b12), (a13,X7,b13), (a13,X7,b14), (a13,X7,b15), (a13,X7,b16), (a13,X7,b17), (a13,X7,b18), (a13,X7,b19), (a13,X7,b20), (a13,X7,b21), (a13,X7,b22), (a13,X7,b2 3), (a13,X7,b24), (a13,X7,b25), (a13,X7,b26), (a13,X7,b27), (a13,X7,b28), (a14,X1,b1), (a14,X1,b2), (a14,X1,b3), (a14,X1,b4), (a14,X1,b5), (a14,X1,b6), (a14 , X1,b7), (a14,X1,b8), (a14,X1,b9), (a14,X1,b10), (a14,X1,b11), (a14,X1,b12), (a14, X1,b13), (a14,X1,b14), (a14,X1,b15), (a14,X1,b16), (a14,X1,b17), (a14,X1,b18), (a 14,X1,b19), (a14,X1,b20), (a14,X1,b21), (a14,X1,b22), (a14,X1,b23), (a14,X1,b24) , (a14,X1,b25), (a14,X1,b26), (a14,X1,b27), (a14,X1,b28), (a14,X2,b1), (a14,X2,b2 ), (a14,X2,b3), (a14,X2,b4),(a14,X2,b5), (a14,X2,b6), (a14,X2,b7), (a14,X2,b8), (a1 4,X2,b9), (a14,X2,b10), (a14,X2,b11), (a14,X2,b12), (a14,X2,b13), (a14,X2,b14), (a 14,X2,b15), (a14,X2,b16), (a14,X2,b17), (a14,X2,b18), (a14,X2,b19), (a14,X2,b20) , (a14,X2,b21), (a14,X2,b22), (a14,X2,b23), (a14,X2,b24), (a14,X2,b25), (a14,X2,b 26), (a14,X2,b27), (a14,X2,b28), (a14,X3,b1), (a14,X3,b2), (a14,X3,b3), (a14,X3,b 4), (a14,X3,b5), (a14,X3,b6), (a14,X3,b7), (a14,X3,b8), (a14,X3,b9), (a14,X3,b10), ( a14,X3,b11), (a14,X3,b12), (a14,X3,b13), (a14,X3,b14), (a14,X3,b15), (a14,X3,b1 6), (a14,X3,b17), (a14,X3,b18), (a14,X3,b19), (a14,X3,b20), (a14,X3,b21), (a14,X3 , b22), (a14,X3,b23), (a14,X3,b24), (a14,X3,b25), (a14,X3,b26), (a14,X3,b27), (a14, X3,b28), (a14,X4,b1), (a14,X4,b2), (a14,X4,b3), (a14,X4,b4), (a14,X4,b5), (a14,X4, b6), (a14,X4,b7), (a14,X4,b8), (a14,X4,b9), (a14,X4,b10), (a14,X4,b11), (a14,X4,b1 2), (a14,X4,b13), (a14,X4,b14), (a14,X4,b15) (a14,X4,b16), (a14,X4,b17), (a14,X4 , b18), (a14,X4,b19), (a14,X4,b20), (a14,X4,b21), (a14,X4,b22), (a14,X4,b23), (a14, X4,b24), (a1,X4,b25), (a14,X4,b26), (a14,X4,b27), (a14,X4,b28), (a14,X5,b1), (a1 4,X5,b2), (a14,X5,b3), (a14,X5,b4), (a14,X5,b5), (a14,X5,b6), (a14,X5,b7), (a14,X5 , b8), (a14,X5,b9), (a14,X5,b10), (a14,X5,b11), (a14,X5,b12), (a14,X5,b13), (a14,X5 , b14), (a14,X5,b15), (a14,X5,b16), (a14,X5,b17) (a14,X5,b18), (a14,X5,b19), (a14, X5,b20), (a14,X5,b21), (a14,X5,b22), (a14,X5,b23), (a14,X5,b24), (a14,X5,b25), (a 14,X5,b26), (a14,X5,b27), (a14,X5,b28), (a14,X6,b1), (a14,X6,b2), (a14,X6,b3), (a 14,X6,b4), (a14,X6,b5), (a14,X6,b6), (a14,X6,b7), (a14,X6,b8), (a14,X6,b9), (a14,X 6,b10), (a14,X6,b11), (a14,X6,b12), (a14,X6,b13), (a14,X6,b14), (a14,X6,b15), (a1 4,X6,b16), (a14,X6,b17), (a14,X6,b18), (a14,X6,b19), (a14,X6,b20), (a14,X6,b21), (a14,X6,b22), (a14,X6,b23), (a14,X6,b24), (a14,X6,b25), (a14,X6,b26), (a14,X6,b2 7), (a14,X6,b28), (a14,X7,b1), (a14,X7,b2), (a14,X7,b3), (a14,X7,b4), (a14,X7,b5), ( a14,X7,b6), (a14,X7,b7), (a14,X7,b8), (a14,X7,b9), (a14,X7,b10), (a14,X7,b11), (a1 4,X7,b12), (a14,X7,b13), (a14,X7,b14), (a14,X7,b15), (a14,X7,b16), (a14,X7,b17), (a14,X7,b18), (a14,X7,b19), (a14,X7,b20), (a14,X7,b21), (a14,X7,b22), (a14,X7,b2 3), (a14,X7,b24), (a14,X7,b25), (a14,X7,b26), (a14,X7,b27), (a14,X7,b28), (a15,X1,b1), (a15,X1,b2), (a15,X1,b3), (a15,X1,b4), (a15,X1,b5), (a15,X1,b6), (a15 , X1,b7), (a15,X1,b8), (a15,X1,b9), (a15,X1,b10), (a15,X1,b11), (a15,X1,b12), (a15, X1,b13), (a15,X1,b14), (a15,X1,b15), (a15,X1,b16), (a15,X1,b17), (a15,X1,b18), (a 15,X1,b19), (a15,X1,b20), (a15,X1,b21), (a15,X1,b22), (a15,X1,b23), (a15,X1,b24) , (a15,X1,b25), (a15,X1,b26), (a15,X1,b27), (a15,X1,b28), (a15,X2,b1), (a15,X2,b2 ), (a15,X2,b3), (b15,X2,b4), (a15,X2,b5), (a15,X2,b6), (a15,X2,b7), (a15,X2,b8), (a1 5,X2,b9), (a15,X2,b10), (a15,X2,b11), (a15,X2,b12), (a15,X2,b13), (a15,X2,b14), (a 15,X2,b15), (a15,X2,b16), (a15,X2,b17), (a15,X2,b18), (a15,X2,b19), (a15,X2,b20) , (a15,X2,b21), (a15,X2,b22), (a15,X2,b23), (a15,X2,b24), (a15,X2,b25), (a15,X2,b 26), (a15,X2,b27), (a15,X2,b28), (a15,X3,b1), (a15,X3,b2), (a15,X3,b3), (a15,X3,b 4), (a15,X3,b5), (a15,X3,b6), (a15,X3,b7), (a15,X3,b8), (a15,X3,b9), (a15,X3,b10), ( a15,X3,b11), (a15,X3,b12), (a15,X3,b13), (a15,X3,b14), (a15,X3,b15), (a15,X3,b1 6) (a15,X3,b17), (a15,X3,b18), (a15,X3,b19), (a15,X3,b20), (a15,X3,b21), (a15,X3 , b22), (a15,X3,b23), (a15,X3,b24), (a15,X3,b25), (a15,X3,b26), (a15,X3,b27), (a15, X3,b28), (a15,X4,b1), (a15,X4,b2), (a15,X4,b3), (a15,X4,b4), (a15,X4,b5), (a15,X4, b6), (a15,X4,b7), (a15,X4,b8), (a15,X4,b9), (a15,X4,b10), (a15,X4,b11), (a15,X4,b1 2), (a15,X4,b13), (a15,X4,b14), (a15,X4,b15), (a15,X4,b16), (a15,X4,b17), (a15,X4 , b18), (a15,X4,b19), (a15,X4,b20), (a15,X4,b21), (a15,X4,b22), (a15,X4,b23), (a15, X4,b24), (a15,X4,b25), (a15,X4,b26), (a15,X4,b27), (a15,X4,b28), (a15,X5,b1), (a1 5,X5,b2), (a15,X5,b3), (a15,X5,b4), (a15,X5,b5), (a15,X5,b6), (a15,X5,b1), (a15,X5 , b8), (a15,X5,b9), (a15,X5,b10), (a15,X5,b11), (a15,X5,b12), (a15,X5,b13), (a15,X5 , b14), (a15,X5,b15), (a15,X5,b16), (a15,X5,b17), (a15,X5,b18), (a15,X5,b19), (a15, X5,b20), (a15,X5,b21), (a15,X5,b22), (a15,X5,b23), (a15,X5,b24), (a15,X5,b25), (a 15,X5,b26), (a15,X5,b28), (a15,X5,b28), (a15,X6,b1), (a15,X6,b2), (a15,X6,b3), (a 15,X6,b4), (a15,X6,b5), (a15,X6,b6), (a15,X6,b7), (a15,X6,b8), (a15,X6,b9), (a15,X 6,b10), (a15,X6,b11), (a15,X6,b12), (a15,X6,b13), (a15,X6,b14), (a15,X6,b15), (a1 5,X6,b16), (a15,X6,b17), (a15,X6,b18), (a15,X6,b19), (a15,X6,b20), (a15,X6,b21), (a15,X6,b22), (a15,X6,b23), (a15,X6,b24), (a15,X6,b25), (a15,X6,b26), (a15,X6,b2 7), (a15,X6,b28), (a15,X7,b1), (a15,X7,b2), (a15,X7,b3), (a15,X7,b4), (a15,X7,b5), ( a15,X7,b6), (a15,X7,b7), (a15,X7,b8), (a15,X7,b9), (a15,X7,b10), (a15,X7,b11), (a1 5,X7,b12), (a15,X7,b13), (a15,X7,b14), (a15,X7,b15), (a15,X7,b16), (a15,X7,b17), (a15,X7,b18), (a15,X7,b19), (a15,X7,b20), (a15,X7,b21), (a15,X7,b22), (a15,X7,b2 3), (a15,X7,b24), (a15,X7,b25), (a15,X7,b26), (a15,X7,b27), (a15,X7,b28), (a16,X1,b1), (a16,X1,b2), (a16,X1,b3), (a16,X1,b4), (a16,X1,b5), (a16,X1,b6), (a16 , X1,b7), (a16,X1,b8), (a16,X1,b9), (a16,X1,b10), (a16,X1,b11), (a16,X1,b12), (a16, X1,b13), (a16,X1,b14), (a16,X1,b15), (a16,X1,b16), (a16,X1,b17), (a16,X1,b18), (a 16,X1,b19), (a16,X1,b20), (a16,X1,b21), (a16,X1,b22), (a16,X1,b23), (a16,X1,b24) , (a16,X1,b25), (a16,X1,b26), (a16,X1,b27), (a16,X1,b28), (a16,X2,b1), (a16,X2,b2 ), (a16,X2,b3), (a16,X2,b4), (a16,X2,b5), (a16,X2,b6), (a16,X2,b7), (a16,X2,b8), (a1 6,X2,b9), (a16,X2,b10), (a16,X2,b11), (a16,X2,b12), (a16,X2,b13), (a16,X2,b14), (a 16,X2,b15), (a16,X2,b16), (a16,X2,b17), (a16,X2,b18), (a16,X2,b19), (a16,X2,b20) , (a16,X2,b21), (a16,X2,b22), (a16,X2,b23), (a16,X2,b24), (a16,X2,b25), (a16,X2,b 26), (a16,X2,b27), (a16,X2,b28), (a16,X3,b1), (a16,X3,b2), (a16,X3,b3), (a16,X3,b 4), (a16,X3,b5), (a16,X3,b6), (a16,X3,b7), (a16,X3,b8), (a16,X3,b9), (a16,X3,b10), ( a16,X3,b11), (a16,X3,b12), (a16,X3,b13), (a16,X3,b14), (a16,X3,b15), (a16,X3,b1 6), (a16,X3,b17), (a16,X3,b18), (a16,X3,b19), (a16,X3,b20), (a16,X3,b21), (a16,X3 , b22), (a16,X3,b23), (a16,X3,b24), (a16,X3,b25), (a16,X3,b26), (a16,X3,b27), (a16, X3,b28), (a16,X4,b1), (a16,X4,b2), (a16,X4,b3), (a16,X4,b4), (a16,X4,b5), (a16,X4, b6), (a16,X4,b7), (a16,X4,b8), (a16,X4,b9), (a16,X4,b10), (a16,X4,b11), (a16,X4,b1 2), (a16,X4,b13), (a16,X4,b14), (a16,X4,b15), (a16,X4,b16), (a16,X4,b17), (a16,X4 , b18), (a16,X4,b19), (a16,X4,b20), (a16,X4,b21), (a16,X4,b22), (a16,X4,b23), (a16, X4,b24), (a16,X4,b25), (a16,X4,b26), (a16,X4,b27), (a16,X4,b28), (a16,X5,b1), (a1 6,X5,b2),(a16,X5,b3), (a16,X5,b4), (a16,X5,b5), (a16,X5,b6), (a16,X5,b7), (a16,X5 , b8), (a16,X5,b9), (a16,X5,b10), (a16,X5,b11), (a16,X5,b12), (a16,X5,b13), (a16,X5 , b14), (a16,X5,b15), (a16,X5,b16), (a16,X5,b17), (a16,X5,b18), (a16,X5,b19), (a16, X5,b20), (a16,X5,b21), (a16,X5,b22), (a16,X5,b23), (a16,X5,b24), (a16,X5,b25), (a 16,X5,b26), (a16,X5,b27), (a16,X5,b28), (a16,X6,b1), (a16,X6,b2), (a16,X6,b8), (a 16,X6,b4), (a16,X6,b5), (a16,X6,b6), (a16,X6,b7), (a16,X6,b8), (a16,X6,b9), (a16,X 6,b10), (a16,X6,b11), (a16,X6,b12), (a16,X6,b13), (a16,X6,b14), (a16,X6,b15), (a1 6,X6,b16), (a16,X6,b17), (a16,X6,b18), (a16,X6,b19), (a16,X6,b20), (a16,X6,b21), (a16,X6,b22), (a16,X6,b23), (a16,X6,b24), (a16,X6,b25), (a16,X6,b26), (a16,X6,b2 7), (a16,X6,b28), (a16,X7,b1), (a16,X7,b2), (a16,X7,b3), (a16,X7,b4), (a16,X7,b5), ( a16,X7,b6), (a16,X7,b7), (a16,X7,b8), (a16,X7,b9), (a16,X7,b10), (a16,X7,b11), (a1 6,X7,b12), (a16,X7,b13), (a16,X7,b14), (a16,X7,b15), (a16,X7,b16), (a16,X7,b17), (a16,X7,b18), (a16,X7,b19), (a16,X7,b20), (a16,X7,b21), (a16,X7,b22), (a16,X7,b2 3), (a16,X7,b24), (a16,X7,b25), (a16,X7,b26), (a16,X7,b27), (a16,X7,b28), (a17,X1,b1), (a17,X1,b2), (a17,X1,b3), (a17,X1,b4), (a17,X1,b5), (a17,X1,b6), (a17 , X1,b7), (a17,X1,b8), (a17,X1,b9), (a17,X1,b10), (a17,X1,b11), (a17,X1,b12), (a17, X1,b13), (a17,X1,b14), (a17,X1,b15), (a17,X1,b16), (a17,X1,b17), (a17,X1,b18), (a 17,X1,b19), (a17,X1,b20), (a17,X1,b21) (a17,X1,b22), (a217,X1,b23), (a17,X1,b24) , (a17,X1,b25), (a17,X1,b26), (a17,X1,b27), (a17,X1,b28), (a17,X2,b1), (b17,X2,b2 ), (a17,X2,b3), (a17,X2,b4), (a17,X2,b5), (a17,X2,b6), (a17,X2,b7), (a17,X2,b8), (a1 7,X2,b9), (a17,X2,b10), (a17,X2,b11), (a17,X2,b12), (a17,X2,b13), (a17,X2,b14), (a 17,X2,b15), (a17,X2,b16), (a17,X2,b17), (a17,X2,b18), (a17,X2,b19), (a17,X2,b20) , (a17,X2,b21), (a17,X2,b22), (a17,X2,b23), (a17,X2,b24), (a17,X2,b25), (a17,X2,b 26), (a17,X2,b27), (a17,X2,b28), (a17,X3,b1), (a17,X3,b2), (a17,X3,b3), (a17,X3,b 4), (a17,X3,b5), (a17,X3,b6), (a17,X3,b7), (a17,X3,b8), (a17,X3,b9), (a17,X3,b10), ( a17,X3,b11), (a17,X3,b12), (a17,X3,b13), (a17,X3,b14), (a17,X3,b15), (a17,X3,b1 6), (a17,X3,b17), (a17,X3,b18), (a17,X3,b9), (a17,X3,b20), (a17,X3,b21), (a17,X3 , b22), (a17,X3,b23), (a17,X3,b24), (a17,X3,b25), (a17,X3,b26), (a17,X3,b27), (a17, X3,b28), (a17,X4,b1), (a17,X4,b2), (a17,X4,b3), (a17,X4,b4), (a17,X4,b5), (a17,X4, b6), (a17,X4,b7), (a17,X4,b8), (a17,X4,b9), (a17,X4,b10), (a17,X4,b11), (a17,X4,b1 2), (a17,X4,b13), (a17,X4,b14), (a17,X4,b15), (a17,X4,b16), (a17,X4,b17), (a17,X4 , b18), (a17,X4,b19), (a17,X4,b20), (a17,X4,b21), (a17,X4,b22), (a17,X4,b23), (a17, X4,b24), (a17,X4,b25), (a17,X4,b26), (a17,X4,b27), (a17,X4,b28), (a17,X5,b1), (a1 7,X5,b2), (a17,X5,b3), (a17,X5,b4), (a17,X5,b5), (a17,X5,b6), (a17,X5,b7), (a17,X5 , b8), (a17,X5,b9), (a17,X5,b10), (a17,X5,b11), (a17,X5,b12), (a17,X5,b13), (a17,X5 , b14), (a17,X5,b15), (a17,X5,b16), (a17,X5,b17) (a17,X5,b18), (a17,X5,b19), (a17, X5,b20), (a17,X5,b21), (a17,X5,b22), (a17,X5,b23), (a17,X5,b24), (a17,X5,b20), (a 17,X5,b26), (a17,X5,b27), (a17,X5,b28), (a17,X6,b1), (a17,X6,b2), (a17,X6,b3), (a 17,X6,b4), (a17,X6,b5), (a17,X6,b6), (a17,X6,b7), (a17,X6,b8), (a17,X6,b9), (a17,X 6,b10), (a17,X6,b11), (a17,X6,b12), (a17,X6,b13), (a17,X6,b14), (a17,X6,b15), (a1 7,X6,b16), (a17,X6,b17), (a17,X6,b18), (a17,X6,b19), (a17,X6,b20), (a17,X6,b21), (a17,X6,b22), (a17,X6,b23), (a17,X6,b24), (a17,X6,b25), (a17,X6,b26), (a17,X6,b2 7), (a17,X6,b28), (a17,X7,b1), (a17,X7,b2), (a17,X7,b3), (a17,X7,b4), (a17,X7,b5), ( a17,X7,b6), (a17,X7,b7), (a17,X7,b8), (a17,X7,b9), (a17,X7,b10), (a17,X7,b11), (a1 7,X7,b12), (a17,X7,b13), (a17,X7,b14), (a17,X7,b15), (a17,X7,b16), (a17,X7,b17), (a17,X7,b18), (a17,X7,b19), (a17,X7,b20), (a17,X7,b21), (a17,X7,b22), (a17,X7,b2 3), (a17,X7,b24), (a17,X7,b25), (a17,X7,b26), (a17,X7,b27), (a17,X7,b28), (a18,X1,b1), (a18,X1,b2), (a18,X1,b3), (a18,X1,b4), (a18,X1,b5), (a18,X1,b6), (a18 , X1,b7), (a18,X1,b8), (a18,X1,b9), (a18,X1,b10), (a18,X1,b11) (a18,X1,b12), (a18, X1,b13), (a18,X1,b14), (a18,X1,b16), (a18,X1,b17), (a18,X1,b18), (a18,X1,b18), (a 18,X1,b1), (a18,X1,b20), (a18,X1,b21), (a18,X1,b22), (a18,X1,b23), (a18,X1,b24) , (a18,X1,b25), (a18,X1,b26), (a18,X1,b27), (a18,X1,b28), (a18,X2,b1), (a18,X2,b2 ), (a18,X2,b3), (a18,X2,b4), (a18,X2,b5), (a18,X2,b6), (a18,X2,b7), (a18,X2,b8), (a1 8,X2,b9), (a18,X2,b10), (a18,X2,b11), (a18,X2,b12), (a18,X2,b13), (a18,X2,b14), (a 18,X2,b15), (a18,X2,b16), (a18,X2,b17), (a18,X2,b18), (a18,X2,b19), (a18,X2,b20) , (a18,X2,b21), (a18,X2,b22), (a18,X2,b23), (a18,X2,b24), (a18,X2,b25), (a18,X2,b 26), (a18,X2,b27), (a18,X2,b28), (a18,X3,b1), (a18,X3,b2), (a18,X3,b3), (a18,X3,b 4), (a18,X3,b5), (a18,X3,b6), (a18,X3,b7), (a18,X3,b8), (a18,X3,b9), (a18,X3,b10), ( a18,X3,b11), (a18,X3,b12), (a18,X3,b13), (a18,X3,b14), (a18,X3,b15), (a18,X3,b1 6), (a18,X3,b17), (a18,X3,b18), (a18,X3,b19), (a18,X3,b21), (a18,X3,b21), (a18,X3 , b22), (a18,X3,b23), (a18,X3,b24), (a18,X3,b25), (a18,X3,b26), (a18,X3,b27), (a18, X3,b28), (a18,X4,b1), (a18,X4,b2), (a18,X4,b3), (a18,X4,b4) (a18,X4,b5), (a18,X4, b6), (a18,X4,b7), (a18,X4,b8), (a18,X4,b9), (a18,X4,b10), (a18,X4,b11), (a18,X4,b 2), (a18,X4,b13), (a18,X4,b14), (a18,X4,b15), (a18,X4,b16), (a18,X4,b17), (a18,X4 , b18), (a18,X4,b19), (a18,X4,b20), (a18,X4,b21), (a18,X4,b22), (a18,X4,b23), (a18, X4,b24), (a18,X4,b25), (a18,X4,b26), (a18,X4,b27), (a18,X4,b28), (a18,X5,b1), (a1 8,X5,b2), (a18,X5,b3), (a18,X5,b4), (a18,X5,b5), (a18,X5,b6), (a18,X5,b7), (a18,X5 , b8), (a18,X5,b9), (a18,X5,b10), (a18,X5,b11), (a18,X5,b12), (a18,X5,b13), (a18,X5 , b14), (a18,X5,b15), (a18,X5,b16), (a18,X5,b17), (a18,X5,b18), (a18,X5,b19), (a18, X5,b20), (a18,X5,b21), (a18,X5,b22), (a18,X5,b23), (a18,X5,b24), (a18,X5,b25), (a 18,X5,b26), (a18,X5,b27), (a18,X5,b28), (a18,X6,b1), (a18,X6,b2), (a18,X6,b3), (a 18,X6,b4), (a18,X6,b5), (a18,X6,b6), (a18,X6,b7), (a18,X6,b8), (a18,X6,b9), (a18,X 6,b10), (a18,X6,b11), (a18; X6,b12), (a18,X6,b13), (a18,X6,b14), (a18,X6,b15), (a1 8,X6,b16), (a18,X6,b17), (a18,X6,b18), (a18,X6,b19), (a18,X6,b20), (a18,X6,b21), (a18,X6,b22), (a18,X6,b23), (a18,X6,b24), (a18,X6,b25), (a18,X6,b26), (a18,X6,b2 7), (a18,X6,b28), (a18,X7,b1), (a18,X7,b2), (a18,X7,b3), (a18,X7,b4), (a18,X7,b5), ( a18,X7,b6), (a18,X7,b7), (a18,X7,b8), (a18,X7,b9), (a18,X7,b10), (a18,X7,b11), (a1 8,X7,b12), (a18,X7,b13), (a18,X7,b14), (a18,X7,b15), (a18,X7,b16), (a18,X7,b17), (a18,X7,b18), (a18,X7,b19), (a18,X7,b20), (a18,X7,b21), (a18,X7,b22), (a18,X7,b2 3), (a18,X7,b24), (a18,X7,b25), (a18,X7,b26), (a18,X7,b27),(a18,X7,b28)

Since the compound represented by the formula (I) has high affinity for a NMDA receptor, particularly a NR1/NR2B receptor, and has high subtype selectivity and selectivity for other receptor, it can be a drug whose side effect (e.g., influence on motor function) is alleviated. In addition, the compound has also advantages that stability is high, oral absorbability is high, good bioavailability is exhibited, clearance is low, brain transferring property is high, a half life is long, a non-protein binding rate is high, drug efficacy maintenance is high, and/or hepatic enzyme inhibitory activity is low.

The compound represented by the formula (I) can be orally or parenterally administered to an animal including a human, as a drug, particularly, a preventive / a therapeutic agent for various central neurological diseases (e.g., cerebral stroke, brain infarct, brain trauma, chronic neurodegeneration disease) caused by a NMDA receptor, particularly, a NR1/NR2B receptor, or an analgesic for a cancer pain or the like. As a dosage form, granules, tablets, capsules, injectables and the like are exemplified. Upon formulation into preparations, optionally, various additives, for example, excipients, disintegrating agents, binders, lubricants, stabilizers, colorants, and coating agents can be used. A dose is different depending on an age, a weight and symptom of a subject, and an administration method, and is not particularly limited, but usually in the case of oral administration, the dose is about 1 mg to about 5000 mg and, in the case of parenteral administration, the dose is about 0.1 mg to about 1000 mg per adult a day.

The present invention will be explained in more detail below by way of Examples, but the present invention is not limited by these Examples at all. A melting point described in a text is an unadjusted value. In addition, $^1$H-NMR was measured using tetramethylsilane as an internal standard in a solvent of heavy chloroform ($CDCl_3$) or heavy dimethyl sulfoxide (DMSO-$d_6$). A $\delta$ value is indicated in ppm, and a coupling constant (J) is indicated in Hz. In data, s means singlet, d means doublet, t means triplet, q means quartet, m means multiplet, br means broad, and brs means broad singlet.

Each abbreviation has the following mean.
Me: methyl,
Et: ethyl,
$Bu^t$: tert-butyl,
THF: tetrahydrofuran
DMF: N,N-dimethylformamide
NMP: 1-methyl-2-pyrrolidinone
HOBt: 1-hyclroxybenzotriazole
DMAP: 4-dimethylaminopyridine
EDC: 1-(3-dimethylaminopropyl)-3-ethylcarbodiimide hydrochloride
DEPC: diethylphosphorocyanidate
BINAP: 2,2'-bis(diphenylphosphino)-1,1'-binaphthyl
Xantphos: 9,9-dimethyl-4,5-bis(di-tert-butylphosphino)xanthene
$Pd_2(dba)_3$: bis(dihenzylideneacetone)palladium(O)

[Example 1]

Synthesis of compound (I-40)

a) Synthesis compound 3

[Chemical formula 18]

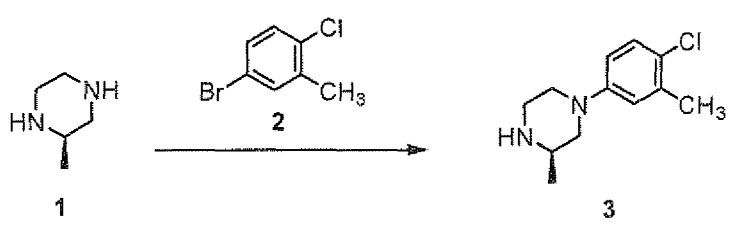

Under the nitrogen atmosphere, toluene (7.5 ml) was added to a commercially available compound 2 (1.50 g, 7.30 mmol), a commercially available compound 1 (1.50 g, 7-30 mmol), and sodium tert-butoxide (842 mg, 8.76 mmol) were added, and the system was degassed, and replaced with nitrogen. Xantphos (127 mg, 0.22 mmol) and $Pd_2(dba)_3$ (67 mg, 0.67 mmol) were added to react them at 100˚C for 1 hour. Toluene (8 ml) was added to dilute the reaction, and this was filtered using Celite. The filtrate was concentrated under reduced pressure, and the resulting residue was purified by silica gel column chromatography (chloroform-methanol) to obtain a compound 3 (1.27 g, yield 77%).
$^1$H-NMR ($CDCl_3$ / TMS) δppm: 1.13 (d, J = 6.2Hz, 3H), 1.57 (s, 1H), 2.28-2.37 (m, 1H), 2.35 (s, 3H), 2.67 (dt, J = 3.5, 11.6Hz, 1H), 2.92-3.15 (m, 3H), 3.46 (d, J = 11.6Hz, 2H), 6.69 (dd, J = 8.9, 2.7Hz, 1H), 6.78 (d, J = 2.7Hz, 1H), 7.19 (d, J = 8.9Hz, 1H).

b) Synthesis of compound 4

[Chemical formula 19]

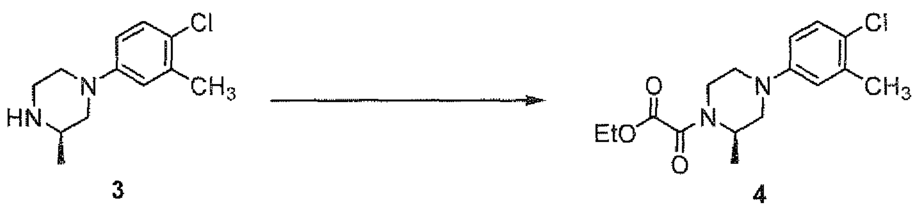

3

4

A compound 3 (900 mg, 4.00 mmol) was dissolved in toluene (9 ml), water (9 ml) and potassium carbonate (664 mg, 4.81 mmol) were added, and the mixture was cooled in an ice bath. Ethyl chloroglyoxylate (0.582 ml, 5.21 mmol) was added dropwise, and the mixture was stirred at 0˚C for 1 hour. Water was added, this was extracted with ethyl acetate, and the organic layer was washed with a saturated aqueous sodium chloride solution, and dried with anhydrous sodium sulfate. A solvent was distilled off under reduced pressure, and the resulting residue was purified by silica gel column chromatography (hexane-ethyl acetate) to obtain a compound 4 (1.27 g, yield 98%).
$^1$H-NMR ($CDCl_3$ / TMS) δppm: 1.39 (t, J = 7.4Hz, 3H), 1.40 and 1.49 (each d, J = 6.9Hz, 3H), 2.34 (s, 3H), 2.69-4.82 (m, 9H), 6.67 (d, J = 9.1Hz, 1H), 6.75 (s, 1H), 7.21 (d, J = 9.1Hz, 1H).

c) Synthesis of compound 5

[Chemical formula 20]

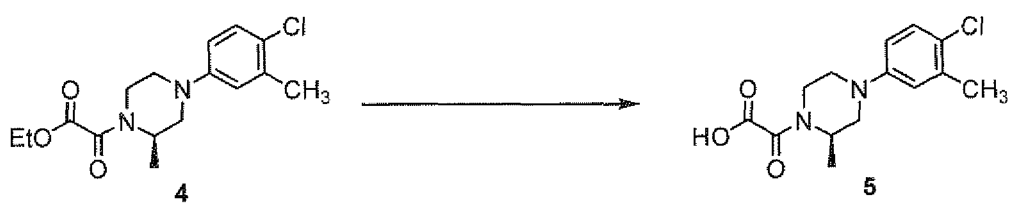

4

5

A compound 4 (1.16 g, 3.57 mmol) was dissolved in ethanol (11.6 ml), and the solution was cooled in an ice bath. 2 mol/L sodium hydroxide (3.57 ml, 7.14 mmol) was added, the mixture was stirred at 0˚C for 30 minutes, 2 mol/L hydrochloric acid (3.57 ml, 7.14 mmol) was added. A saturated aqueous sodium chloride solution was added, this was extracted with ethyl acetate, and the organic layer was dried with anhydrous sodium sulfate. A solvent was distilled off under reduced pressure to obtain a compound 5 (1.09 g, yield 100%).
$^1$H-NMR (DMSO-$d_6$ / TMS) δppm: 1.23 and 1.33 (each d, J = 6.7Hz, 3H), 2.27 (s, 3H), 2.50-4.57 (m, 7H), 6.76-6.82 (m, 1H), 6.93 (m, 1H), 7.22 (d, J = 8.7Hz, 1H).

d) Synthesis of compound (I-40)

[Chemical formula 21]

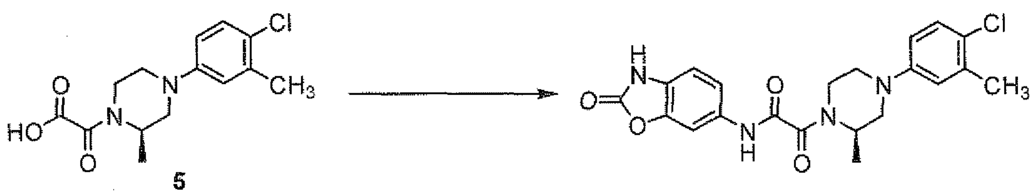

5

I-40

To a compound 5 (500 mg, 1.68 mmol) were added DMF (5 ml), 6-amino-3H-benzoxazole-2-one (253 mg, 1.68 mmol), HOBt (251 mg, 1.85 mmol), DMAP (20.6 mg, 0.17 mmol), and EDC (355 mg, 1.85 mmol), and the mixture was stirred at room temperature for 7 hours. Water was added, and the precipitated crystal was filtered, washed with water, and then dried. The resulting crystal was purified by silica gel column chromatography (chloroform-methanol), and recrystallized from methanol-water to obtain a compound (I-40) (665 mg, yield 92%).
mp 123-124˚C
$^1$H-NMR (DMSO-$d_6$ / TMS) δppm: 1.29 and 1.37 (each d, J = 6.7Hz, 3H), 2.27 (s, 3H), 2.43-4.61 (m, 7H), 6.75-6.82 (m, 1H), 6.94 (m, 1H), 7.07 (d, J = 8.7Hz, 1H), 7.22 (d, J = 8.7Hz, 1H), 7.34-7.39 (m, 1H), 7.70 (s, 1H), 10.84 and 10.88 (each s, 1H), 11.55 (br, 1H).

[Example 2]

Synthesis of compound (I-41)

a) Synthesis of compound 7

[Chemical formula 22]

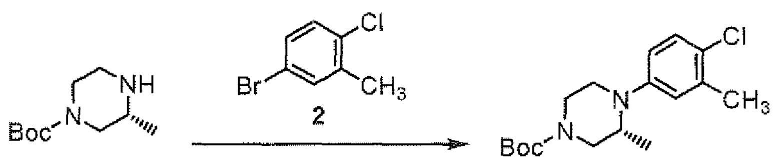

6

7

Under the nitrogen atmosphere, toluene (7.5 ml) was added to a compound 2 (1.50 g, 7.30 mmol), a commercially available compound 6 (1.46 g, 7.30 mmol), and sodium tert-butoxide (1.05 g, 10.9 mmol) were added, and the system was degassed, and replaced with nitrogen. BINAP (68.2 mg, 0.11 mmol), and palladium acetate (16.4 mg, 0.07 mmol) were added to react them at 80˚C for 1 hour. After cooling, a solvent was concentrated under reduced pressure, and the resulting residue was purified by silica gel column chromatography (hexane-ethyl acetate) to obtain a compound 7 (1.26 g, yield 53%).
$^1$H-NMR ($CDCl_3$ / TMS) δppm: 0.97 (d, J = 6.4Hz, 3H), 1.48 (s, 9H), 2.33 (s, 3H), 3.00-3.40 (m, 4H), 3.68-4.05 (m, 3H), 6.67 (dd, J = 8.9, 2.7Hz, 1H), 6.75 (d, J = 2.7Hz, 1H), 7.20 (d, J = 8.9Hz, 1H).

b) Synthesis of compound 8

[Chemical formula 23]

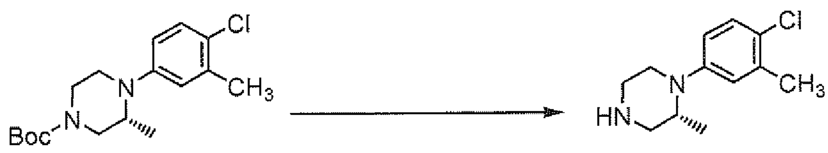

7 8

Under the nitrogen atmosphere, a compound 7 (1.25 g, 3.85 mmol) was dissolved in methanol (6.25 ml), 4 mol/L hydrogen chloride-ethyl acetate (3.85 ml, 15.4 mmol) was added, and the mixture was stirred at 60˚C for 50 minutes. A solvent was distilled off under reduced pressure, and ethyl acetate was added to the residue to crystallize. A crystal was filtered, washed with hexane-ethyl acetate, and then dried to obtain a compound 8 dihydrochloride (1.07 g, yield 93%).
$^{1}$H-NMR (DMSO-$d_6$ / TMS) δppm: 1.06 (d, J = 6.7Hz, 3H), 2.29 (s, 3H), 3.01-4.10 (m, 8H), 6.91 (d, J = 8.7Hz, 1H), 7.05 (s, 1H), 7.30 (d, J = 8.7Hz, 1H), 9.26 (brs, 1H), 9.72 (brs, 1H).

c) Synthesis of compound 9

[Chemical formula 24]

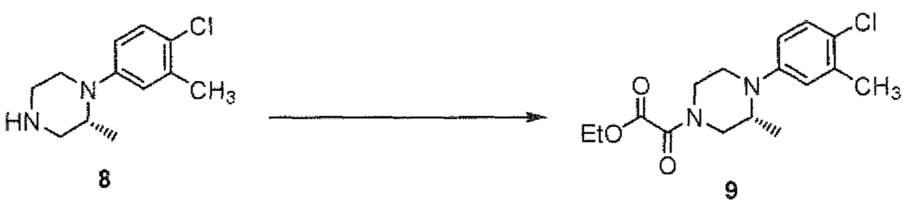

8 9

A compound 8 dihydrochloride (800 mg, 2.69 mmol) was dissolved in toluene (8 ml), water (8 ml) and potassium carbonate (1.11 g, 8.06 mmol) were added, the mixture was cooled in an ice bath, ethyl chloroglyoxylate (0.39 ml, 3.49 mmol) was added dropwise, and the mixture was stirred at 0˚C for 1 hour. Water was added, this was extracted with ethyl acetate, and the organic layer was dried with anhydrous sodium sulfate. A solvent was distilled off under reduced pressure, and the resulting residue was purified by silica gel column chromatography (hexane-ethyl acetate) to obtain a compound 9 (811 mg, yield 93%).
$^{1}$H-NMR ($CDCl_3$ / TMS) δppm: 0.99 and 1.00 (each d, J = 6.5Hz, 3H), 1.38 and 1.39 (each t, J = 7.2Hz, 3H), 2.34 (s, 3H), 3.02-4.41 (m, 9H), 6.66-6.72 (m, 1H), 6.76-6.80 (m, 1H), 7.22 (d, J = 8.7Hz, 1H).

d) Synthesis of compound 10

[Chemical formula 25]

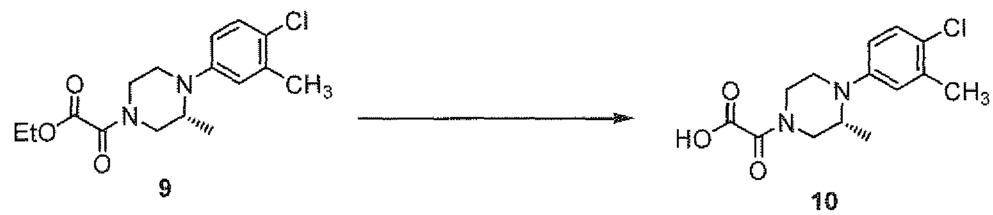

A compound 9 (700 mg, 2.16 mmol) was dissolved in ethanol (7 ml), and the solution was cooled in an ice bath. After 2 mol/L sodium hydroxide (2.16 ml, 4.31 mmol) was added and the mixture was stirred at 0°C for 30 minutes, 2 mol/L hydrochloric acid (2.16 ml, 4.31 mmol) was added. This was extracted with ethyl acetate, the organic layer was dried with anhydrous sodium sulfate, and a solvent was then distilled off under reduced pressure to obtain a compound 10 (660 mg, yield 100%).
$^1$H-NMR (DMSO-de / TMS) δppm: 0.86 and 0.89 (each d, J = 6.5Hz, 3H), 2.27 (s, 3H), 2.88-4.20 (m, 7H), 6.73-6.79 (m, 1H), 6.89-6.92 (m, 1H), 7.22 (d, J = 8.7Hz, 1H).

e) Synthesis of compound (I-41)

[Chemical formula 26]

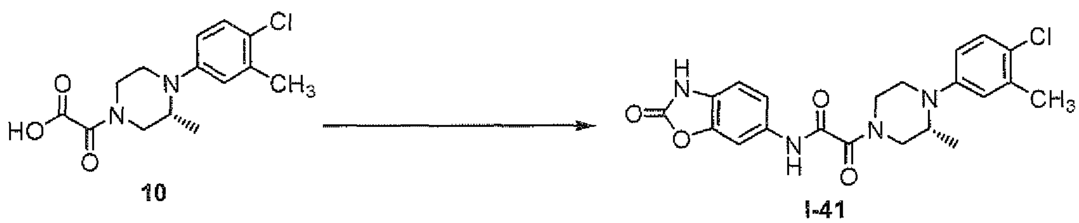

To a compound 10 (500 mg, 1.68 mmol) were added DMF (5 ml), 6-amino-3H-henzoxazole-2-one (253 mg, 1.68 mmol), HOBt (251 mg, 1.85 mmol), DMAP (20.6 mg, 0.17 mmol), and EDC (355 mg, 1.85 mmol), and the mixture was stirred at room temperature for 1.5 hours. Water was added, and the precipitated crystal was filtered, washed with water, and then dried. The resulting crystal was recrystallized from methanol-water to obtain a compound (I-41) (622 mg, yield 86%).
mp 125-127°C
$^1$H-NMR (DMSO-$d_6$ / TMS) δppm: 0.93 (d, J = 6.4Hz, 3H), 2.27 (s, 3H), 2.94-4.30 (m, 7H), 6.74-6.80 (m, 1H), 6.91 (m, 1H), 7.05-7.10 (m, 1H), 7.22 (d, J = 8.7Hz, 1H), 7.34-7.42 (m, 1H), 7.71 (dd, J = 7.7, 1.8Hz, 1H), 10.88 (d, J = 5.4Hz, 1H), 11.60 (br, 1H).

[Example 3]

Synthesis of compound (I-60)

a) Synthesis of compound 11

[Chemical formula 27]

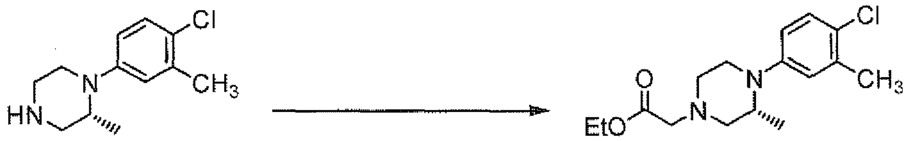

8

11

A compound 8 dihydrochloride (500 mg, 1.68 mmol) was dissolved in DMF (3 ml), potassium carbonate (697 mg, 5.04 mmol), and ethyl bromoacetate (0.24 ml, 2.18 mmol) were added, and the mixture was stirred at room temperature for 1.5 hours. Water was added, this was extracted with ethyl acetate, and the organic layer was washed with water, and a saturated aqueous sodium chloride solution. After drying with anhydrous sodium sulfate, a solvent was distilled off under reduced pressure, and the resulting residue was purified by silica gel column chromatography (hexane-ethyl acetate) to obtain a compound 11 (486 mg, yield 93%). $^1$H-NMR ($CDCl_3$ / TMS) δppm: 1.07 (d, J = 6.5Hz, 3H), 1.29 (t, J = 7.0Hz, 3H), 2.32 (s, 3H), 2.52-2.60 (m, 1H), 2.66 (d, J = 3.7Hz, 2H), 2,81-2.87 (m, 1H), 3.12-3.31 (m, 4H), 3.70-3.80 (m, 1H), 4.20 (dq, J = 3.7, 7.0Hz, 2H), 6.70 (dd, J = 8.6, 2.9Hz, 1H), 6.78 (d, J = 2.9Hz, 1H), 7.19 (d, J = 8.6Hz, 1H). b) Synthesis of compound (I-60)

[Chemical formula 28]

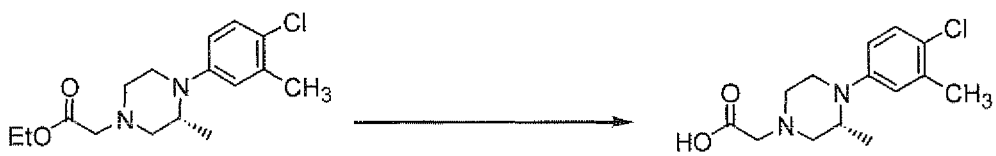

11

12

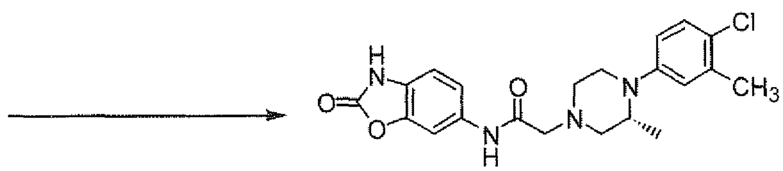

I-60

b-1) Synthesis of compound 12

A compound 11 (400 mg, 1.29 mmol) was dissolved in ethanol (2 ml), 2 mol/L sodium hydroxide (1.29 ml, 2.57 mmol) was added, and the mixture stirred at room temperature for 45 minutes. After 2 mol/L hydrochloric acid (1.29 ml, 2.57 mmol) was added, a solvent was distilled off under reduced pressure to obtain a crude compound 12. This was used as it was in a reaction of a next step.

b-2) Synthesis of compound (I-60)

To the crude compound 12 were added DMF (4 ml), 6-amino-3H-benzoxazole-2-one (193 mg, 1.29 mmol), HOBt (191 mg, 1.41 mmol), DMAP (15.7 mg, 0.13 mmol), and EDC (271 mg, 1.41 mmol), and the mixture was stirred at room temperature for 18.5 hours. A saturated aqueous sodium bicarbonate solution (4 ml) and water (20 ml) were added, and the precipitated crystal was filtered, washed with water, and then dried. The resulting crystal war recrystallized from methanol-water to obtain a compound (I-60) (462 mg, yield from compound 11 87%). mp 169-171˚C
$^1$H-NMR (DMSO-$d_6$ / TMS) δppm: 1.07 (d, J = 6.4Hz, 3H), 2.26 (s, 3H), 2.30-3.37 (m, 8H), 3.97-4.01 (m, 1H), 6.87 (d, J = 2.0Hz, 1H), 7.03 (d, J = 8.4Hz, 1H), 7.19 (d, J = 8.9Hz, 1H), 7.30 (dd, J = 8.4, 2.0Hz, 1H), 7.72 (s, 1H), 9.73 (s, 1H), 11.54 (brs, 1H).

[Example 4]

Synthesis of compound (I-63)

a) Synthesis of compound 14

[Chemical formula 29]

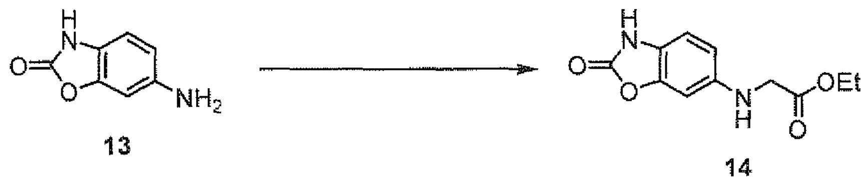

13 14

A commercially available compound 13 (901 mg, 6.00 mmol), and ethyl bromoacetate (613 mg, 5.00 mmol) were dissolved in NMP (5 ml), and the mixture was stirred at 120˚C for 1.5 hours. Diisopropylethylamine (646 mg, 5.00 mmol) was added, and the mixture was further stirred at 1204˚C for 1 hour. After allowing to cool to room temperature, a saturated aqueous sodium bicarbonate solution was added, this was extracted with ethyl acetate, and the organic layer was washed with a saturated aqueous sodium chloride solution. After drying with anhydrous magnesium sulfate, a solvent was distilled off under reduced pressure, and the resulting residue was purified by silica gel column chromatography (chloroform-methanol) to obtain a compound 14 (770 mg, yield 65%).
$^1$H-NMR ($CDCl_3$ / TMS) δppm: 1.31 (t, J = 7.1Hz, 3H), 3.88 (s, 2H), 4.25 (q, J = 7.1Hz, 2H), 4.30 (brs, 1H), 6.41 (dd, J = 8.1, 2.0Hz, 1H), 6.52 (d, J = 2.0Hz, 1H), 6.86 (d, J = 8.1Hz, 1H), 8.49 (brs, 1H).

b) Synthesis of compound 15

[Chemical formula 30]

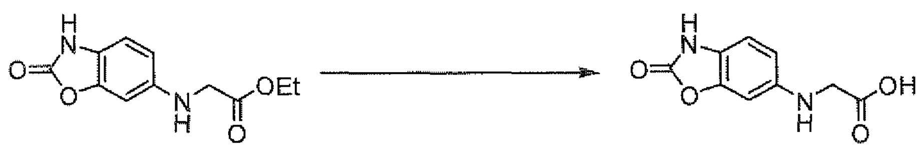

14 15

A compound 14 (1.37 g, 5.80 mmol) was dissolved in methanol (15 ml), and the solution was cooled in an ice bath. 2 mol/L sodium hydroxide (6.40 ml, 12.76 mmol) was added, and the mixture was stirred at room temperature for 1 hour. A solvent was distilled off under reduced pressure, water, 2 mol/L hydrochloric acid (1.29 ml, 2.57 mmol) were added, and the precipitated solid was filtered and dried to obtain a compound 15 (750 mg, yield 62%).
$^1$H-NMR (DMSO-$d_6$ / TMS) δppm: 3.78 (s, 2H), 6.36 (dd, J = 8.1, 2.0Hz, 1H), 6.55 (d, J = 2.0Hz, 1H), 6.81 (d, J = 8.1Hz, 1H), 11.10 (brs, 1H).

c) Synthesis of compound (I-63)

[Chemical formula 31]

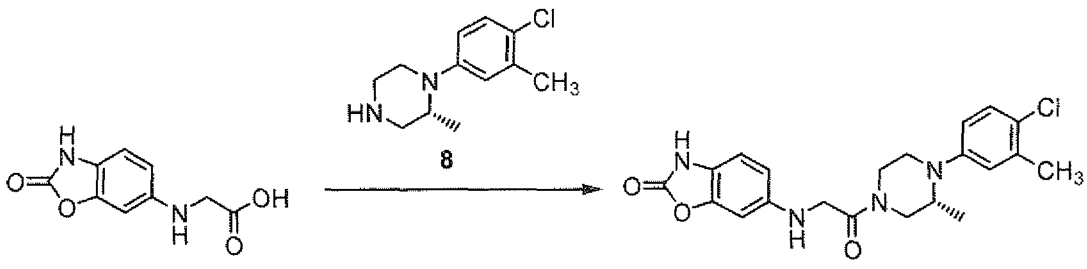

15

I-63

A compound 15 (168 mg, 0.81 mmol), and a compound 8 (240 mg, 0.81 mmol) were dissolved in DMF (5 ml), and the mixture was cooled in an ice bath. A solution of DEPC (0.135 ml, 0.89 mmol) in DMF (1 ml), and a solution of triethylamine (0.369 ml, 2.66 mmol) in DMF (2 ml) were added, sequentially, and the mixture was stirred at 0˚C for 30 minutes. A semi-saturated aqueous sodium bicarbonate solution (30 ml) and water (30 ml) were added, this was extracted with ethyl acetate, and the organic layer was washed with a saturated aqueous sodium bicarbonate solution, and a saturated aqueous sodium chloride solution. After drying with anhydrous magnesium sulfate, a solvent was distilled off under reduced pressure, and the resulting residue was recrystallized from methanol-water to obtain a compound (1-63) (245 mg, yield 73%). mp 112-114˚C
$^1$H-NMR (DMSO-$d_6$ / TMS) δppm: 0.84 and 0.95 (each d, J = 6.6Hz, 3H), 2.27 (s, 3H), 2.88-4.30 (m, 9H), 5.55-5.63 (m, 1H), 6.49 (dd, J = 8.1, 2.0Hz, 1H), 6.70 (d, J = 2.0Hz, 1H), 6.73-6.80 (m, 1H), 6.81 (d, J = 8.1Hz, 1H), 6.87-6.92 (m, 1H), 7.21 (d, J = 8.6Hz, 1H), 11.10 (br, 1H).

[Example 5]

Synthesis of compound (I-66)

a) Synthesis of compound 17

[Chemical formula 32]

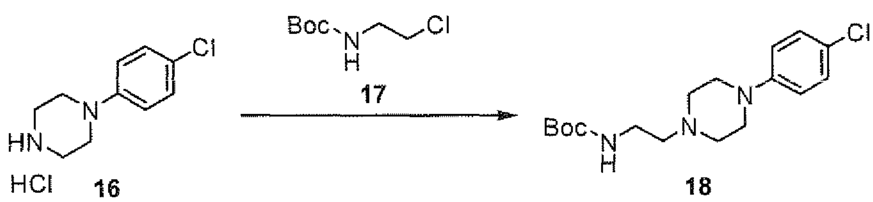

16

18

A commercially available compound 16 (7.00 g, 30.0 mmol), and a commercially available compound 17 (5.90 g, 33.0 mmol) were suspended in DMF (70 ml), sodium iodide (5.40 g, 36.0 mmol), and potassium carbonate (9.50 g, 69.0 mmol) were added, and the mixture was stirred at 80˚C for 11 hours. Insolubles were filtered off, and a solid was washed with ethyl acetate. Water was added to the filtrate and the washing solution, this was extracted with ethyl acetate, and the organic layer was washed with a 10% aqueous sodium thiosulfate solution (200 ml) and water (200 ml). After the organic layer was dried with anhydrous magnesium sulfate, a solvent was distilled off under reduced pressure, and the resulting residue was recrystallized from hexane-ethyl acetate to obtain a compound 18 (4.20 g, yield 42%).
$^1$H-NMR ($CDCl_3$ / TMS) δppm: 1.46 (s, 9H), 2.52 (t, J = 5.7Hz, 2H), 2.60 (t, J = 4.8Hz, 4H), 3.16 (t, J = 4.8Hz, 4H), 3.27 (t, J = 5.7Hz, 2H), 4.97 (brs, 1H), 6.83 (d, J = 9.1Hz, 2H). 7.20 (d, J = 9.1Hz, 2H).

b) Synthesis of compound 18

[Chemical formula 33]

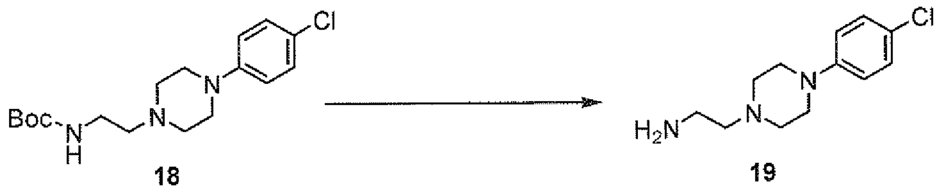

A compound 18 (4.20 g, 12.0 mmol) was dissolved in methanol (40 ml), 4 mol/L hydrogen chloride-ethyl acetate (12.0 ml, 48.0 mmol) was added, and the mixture was stirred at 60˚C for 4 hours. A solvent was distilled off under reduced pressure, and diethyl ether was added to the residue to crystallize. The crystal was filtered, washed with diethyl ether, and then dried to obtain a compound 19 trihydrochloride (4.20 g, yield 100%).
$^1$H-NMR (DMSO-$d_6$ / TMS) δppm: 3.06-3.95 (m, 12H), 7.04 (d, J = 9.1Hz, 2H), 7.30 (d, J = 9.1Hz, 2H), 8.53 (s, 3H), 11.53 (brs, 1H).

c) Synthesis of compound (I-66)

[Chemical formula 34]

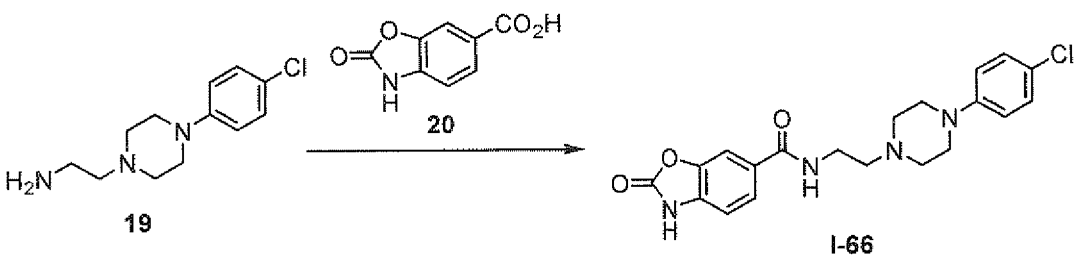

A compound 19 trihydrochloride (0.49 g, 1.40 mmol), and the known (J. Chem. Soc., 1921, viol.119, pp. 1425-1432) compound 20 (0.25 g, 1.40 mmol) were dissolved in DMF (10 ml), HOBt monohydrate (0.21 g, 1.50 mmol), DMAP (17 mg, 0.14 mmol), N-methylmorpholine (0.69 ml, 6.30 mmol), and EDC (0.30 g, 1.50 mmol) were added, and the mixture was stirred at room temperature for 3 hours. Water (50 ml) was added, and the precipitated crystal was filtered, washed with water, and then dried. The resulting solid was recrystallized from ethyl acetate-methanol-diethyl ether to obtain a compound (I-66) (0.50 g, yield 89%). mp 156-158˚C
$^1$H-NMR (DMSO-$d_6$ / TMS) δppm: 2.48-2.64 (m, 6H), 3.10-3.20 (m, 4H), 3.42 (q, J = 6.3Hz, 2H), 6.94 (d, J = 9.1Hz, 2H), 7.15 (d, J = 8.1Hz, 1H), 7.22 (d, J = 9.1Hz, 2H), 7.69-7.75 (m, 2H), 8.04 (t, J = 6.3Hz, 1H).

[Example 6]

Synthesis of compound (I-60)

a) Synthesis of compound 23

[Chemical formula 35]

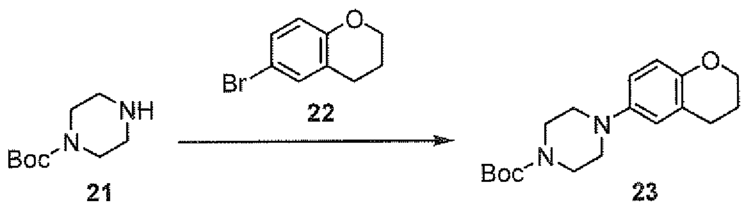

21 22 23

Under the nitrogen atmosphere, toluene (2.7 ml) was added to the known (Khimiya Geterotsiklicheskikh Soedinenii, 1984, No.8, pp. 1035-1038) compound 22 (533 mg, 2.50 mmol), a commercially available compound 21 (466 mg, 2.50 mmol) and sodium tert-butoxide (288 mg, 3.00 mmol) were added, and the system was degassed and replaced with nitrogen. Xantphos (43.4 mg, 0.075 mmol) and $Pd_2(dba)_3$ (22.9 mg, 0.025 mmol) were added to react them at 100˚C for 1 hour. The reaction solution was filtered using Celite and washed with toluene. The filtrate and the washing solution were combined, and concentrated under reduced pressure, and the resulting residue was purified by silica gel column chromatography (hexane-ethyl acetate) to obtain a compound 23 (710 mg, yield 89%).
$^1$H-NMR ($CDCl_3$ / TMS) δppm: 1.48 (s, 9H), 1.94-2.02 (m, 2H), 2.76 (t, J = 6.5Hz, 2H), 2.98 (t, J = 5.2Hz, 4H), 3.56 (t, J = 5.2Hz, 4H), 4.14 (t, J = 5.2Hz, 2H), 6.63 (s, 1H), 6.73 (s, 1H), 6.73 (s, 1H).

b) Synthesis of compound 24

[Chemical formula 36]

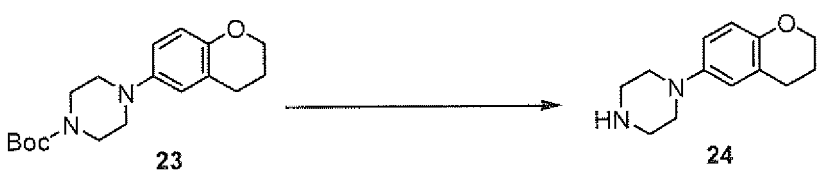

23 24

Under the nitrogen atmosphere, a compound 23 (705 mg, 2.21 mmol) was dissolved in methanol (3.5 ml), 4 mol/L hydrogen chloride-ethyl acetate (2.21 ml, 8.86 mmol) was added, and the mixture was stirred at 60˚C for 1 hour. A solvent was distilled off under reduced pressure, and the precipitated solid was filtered, washed with ethyl acetate, and then dried. A compound 24 dihydrochloride (634 mg, yield 98%) was obtained.
$^1$ H-NMR (DMSO-$d_6$ /TMS) δppm: 1.84-1.92 (m, 2H), 2.70 (t, J = 6.5Hz, 2H), 3.16-3.23 (m, 8H), 3.73 (br, 1H), 4.06 (t, J = 5.0Hz, 2H), 6.63-6.79 (m, 3H), 9.07 (br, 2H).

c) Synthesis of compound 25

[Chemical formula 37]

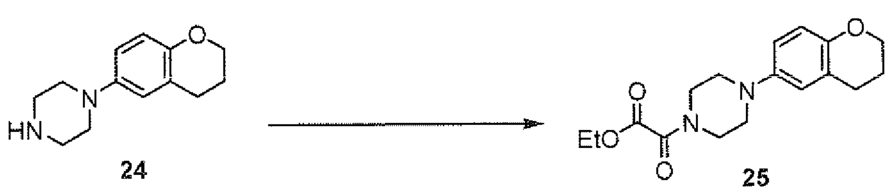

24 25

A compound 24 (315 mg, 1.08 mmol) was dissolved in toluene (1.6 ml), water (1.6 ml) and potassium carbonate (157 mg, 1.41 mmol) were added, ethyl chloroglyoxylate (0.157 ml, 1.41 mmol) was added dropwise, and the mixture was stirred at room temperature for 3 hours. Potassium carbonate (74.6 mg, 0.54 mmol), and ethyl chloroglyoxylate (0.024 ml, 0.22 mmol) were additionally added, and the mixture was stirred at room temperature for 16 hours. Water was added, this was extracted with ethyl acetate, and organic layer was dried with anhydrous sodium sulfate. A solvent was distilled off under reduced pressure, and the resulting residue was purified by silica gel column chromatography (hexane-ethyl acetate) to obtain a compound 25 (306 mg, yield 89%).
$^1$ H-NMR ($CDCl_3$ / TMS) δppm: 1.38 (t, J = 7.2Hz, 3H), 1.94-2.04 (m, 2H), 2.76 (t, J = 6.4Hz, 2H), 3.07 (t, J = 5.2Hz, 4H), 3.58 (t, J = 5.2Hz, 2H), 3.78 (t, J = 5.2Hz, 2H), 4.13 (t, J = 5.2Hz, 2H), 4.35 (q, J = 7.2Hz, 2H), 6.63 (s, 1H), 6.73 (s, 1H), 6.74 (s, 1H).

d) Synthesis of compound (I-60)

[Chemical formula 38]

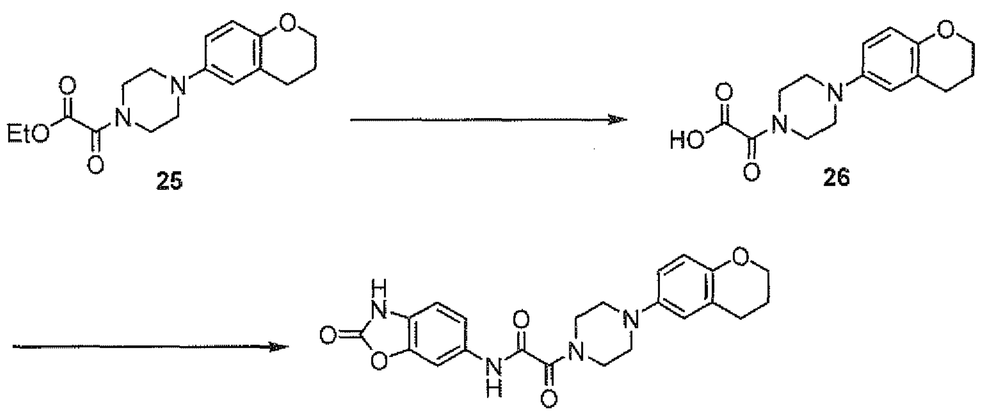

25

26

I-60

d-1) Synthesis of compound 26

A compound 25 (300 mg, 0.94 mmol) was dissolved in ethanol (3 ml), 2 mol/L sodium hydroxide (0.565 ml, 1.13 mmol) was added, and the mixture was stirred at room temperature for 30 minutes. After 2 mol/L hydrochloric acid (0.565 ml, 1.13 mmol) was added, the solvent was distilled off under reduced pressure to obtain a crude compound 26. This was used as it was in a reaction of a next step.

d-2) Synthesis of compound (I-60)

To the crude compound 26 were added DMF (6 ml), 6-amino-3H-benzoxazole-2-one (142 mg, 0.94 mmol), HOBt monohydrate (159 mg, 1.04 mmol), DMAP (11.5 mg, 0.094 mmol), and EDC (199 mg, 1.04 mmol), and the mixture was stirred at room temperature for 6 hours. A saturated aqueous sodium bicarbonate solution (10 ml) and water (20 ml) were added, and the precipitated crystal was filtered, washed with water, and then dried. The resulting solid was purified by silica gel column chromatography (chloroform-methanol), and further recrystallized from methanol-THF-water to obtain a compound (I-60) (313 mg, yield from compound 25 79%). mp 233-2344˚C
$^1$H-NMR (DMSO-$d_6$ / TMS) δppm: 1.84-1.91 (m, 2H), 2.70 (t, J = 6.6Hz, 2H), 3,00-3.05 (m, 4H), 3.62-3.69 (m, 4H), 4.05 (t, J = 5.0Hz, 2H), 6.62 (d, J = 8.7Hz, 1H), 6.67 (d, J = 2.9Hz, 1H), 6.73 (dd, J = 8.7, 2.9Hz, 1H), 7.07 (d, J = 8.4Hz, 1H), 7.37 (dd, J = 8.4, 1.8Hz, 1H), 7.71 (d, J = 1.8Hz, 1H), 10.85 (s, 1H), 11.61 (s, 1H).

[Example 7]

Synthesis of compound (I-67)

a) Synthesis of compound 29

[Chemical formula 39]

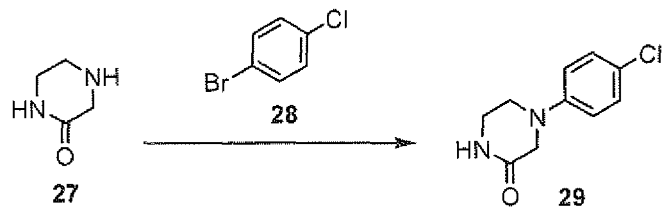

27 28 29

A commercially available compound 27 (1.00 g, 10.0 mmol), a commercially available compound 28 (2.11 g, 11.0 mmol), and sodium tert-butoxide (1.92 g, 20.0 mmol) were dissolved in DMF (15 ml), palladium acetate (112 mg, 0.50 mmol) and BINAP (623 mg, 1.00 mmol) were added to react at 1504˚C for 0.5 hours under Microwave irradiation. To the reaction solution was added water (100 ml), this was extracted with ethyl acetate, and the organic layer was washed with a saturated aqueous sodium chloride solution, and dried with anhydrous magnesium sulfate. A solvent was distilled off under reduced pressure, and the resulting residue was purified by silica gel column chromatography (chloroform-methanol) to obtain a compound 29 (0.62 g, yield 29%). $^1$H-NM ($CDCl_3$ / TMS) δppm: 3.41-3.46 (m, 2H), 3.48-3.55 (m, 2H), 3.85 (s, 2H), 6.55 (br, 1H), 6.86 (d, J = 8.9Hz, 2H), 7.25 (d, J = 8.9Hz, 2H).

b) Synthesis of compound 30

[Chemical formula 40]

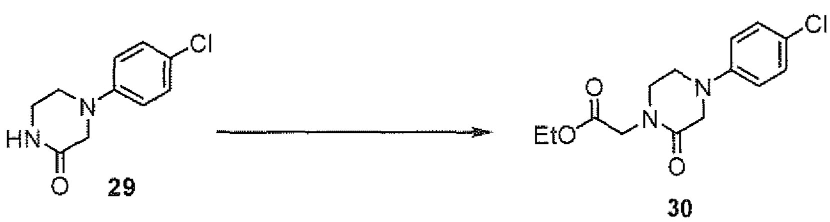

29 30

A compound 29 (211 mg, 1.00 mmol) was dissolved in THF (10 ml), 60% sodium hydride (80 mg, 2.00 mmol) was added under ice-cooling, and the mixture was stirred at 0˚C for 0.5 hours. Ethyl bromoacetate (0.121 ml, 1.10 mmol) was added, the mixture was stirred at room temperature for 1 hour, and water (15 ml) and a saturated aqueous sodium chloride solution (5 ml) were added to the reaction solution under ice-cooling, followed by extraction with ethyl acetate. The organic layer was washed with a saturated aqueous sodium chloride solution, and dried with anhydrous magnesium sulfate, a solvent was distilled off under reduced pressure, and the resulting residue was purified by silica gel column chromatography (hexane-ethyl acetate) to obtain a compound 30 (218 mg, yield 74%).

$^1$ H-NMR ($CDCl_3$ / TMS) δppm: 1.29 (t, J = 7.1Hz, 3H), 3.50-3.58 (m, 4H), 3.91 (s, 2H), 4.20 (s, 2H), 4.21 (q, J = 7.1Hz, 2H), 6.79 (d, J = 9.1Hz, 2H), 7.24 (d, J = 9.1Hz, 2H).

c) Synthesis of compound 31

[Chemical formula 41]

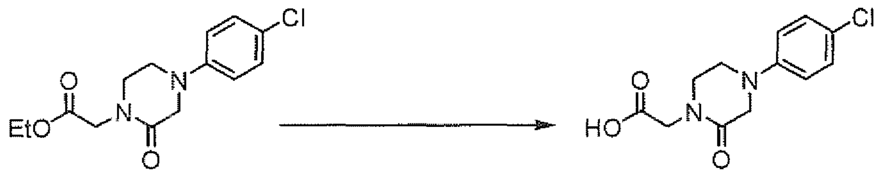

30 31

A compound 30 (210 mg, 0.71 mmol) was dissolved in methanol (5 ml), 2 mol/L sodium hydroxide (0.425 ml, 0.849 mmol) was added, and the mixture was stirred at room temperature for 1 hour. 2 mol/L sodium hydroxide (0.106 ml, 0.212 mmol) was further added, the mixture was further stirred at room temperature for 1 hour, a solvent was distilled off under reduced pressure, and water (5 ml) was added. The aqueous layer was washed with toluene (5 ml), and 2 mol/L hydrochloric acid (0.53 ml, 1.06 mmol) was then added, followed by extraction with ethyl acetate. The organic layer was dried with anhydrous magnesium sulfate, and a solvent was distilled off under reduced pressure to obtain a compound 31 (180 mg, yield 95%).
$^1$ H-NMR (DMSO-$d_6$ / TMS) δppm:3.49 (s, 4H), 3.81 (s, 2H), 4.08 (s, 2H), 6.96 (d, J = 9.1Hz, 2H), 7.25 (d, J = 9.1Hz, 2H), 12.78 (br, 1H).

d) Synthesis of compound (I-67)

[Chemical formula 42]

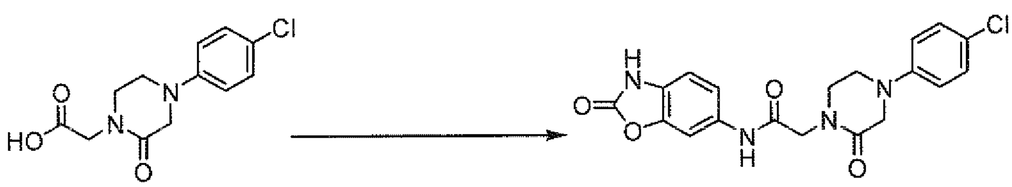

31 I-67

A compound 31 (180 mg, 0.67 mmol), and 6-amino-3H-benzoxazole-2-one (101 mg, 0.67 mmol) were dissolved in DMF (5 ml), EDC (154 mg, 0.804 mmol) was added, and the mixture was stirred at room temperature for 1 hour. Water (10 ml) was added, the precipitated solid was filtered, and the resulting solid was purified by silica gel column chromatography (chloroform-methanol) to obtain a compound (I-67) (60 mg, yield 22%). mp 223-225˚C
$^1$ H-NMR (DMSO-$d_6$ / TMS) δppm: 3.54 (brs, 4H), 3.84 (s, 2H), 4.21 (s, 2H), 6.98 (d, J = 9.1Hz, 2H), 7.04 (d, J = 8.1Hz, 1H), 7.22 (d, J = 8.1Hz, 1H), 7.26 (d, J = 9.1Hz, 2H), 7.67 (s, 1H), 10.17 (s, 1H), 11.55 (br, 1H).
According to the same manner, other compounds represented by the formula (I) were synthesized below. A structural formula and physical constant are shown below.
[Table 4]

| Compound No. | Structural formula | Melting Point |
|---|---|---|
| I-1 | 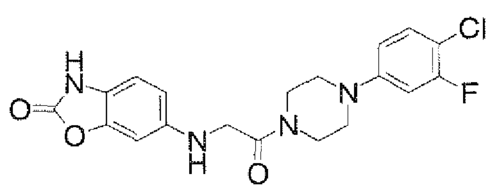 | 136-138 |

(continued)

| Compound No. | Structural formula | Melting Point |
|---|---|---|
| **I-2** | 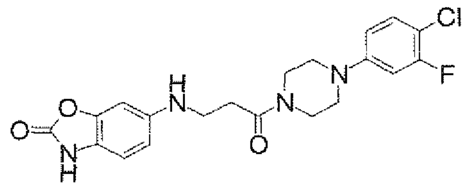 | **187-189** |
| **I-3** | 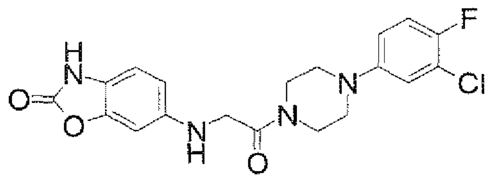 | **130-132** |
| **I-4** | 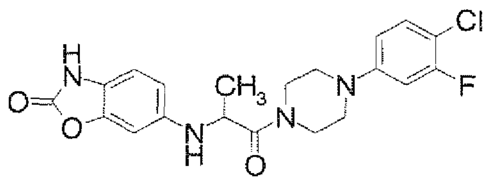 | **223-225** |
| **I-5** | 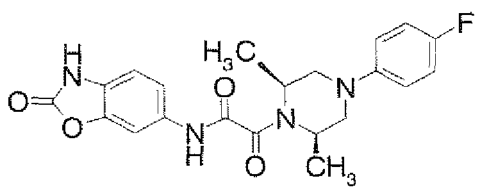 | **245-246** |
| **I-6** | 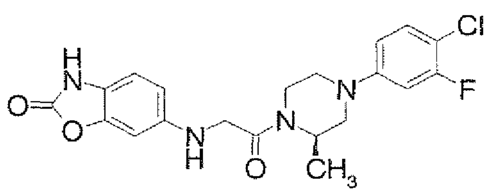 | **224-226** |
| **I-7** | 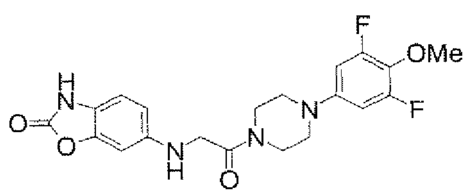 | **194-196** |

[Table 5]

| | | |
|---|---|---|
| **I-8** | 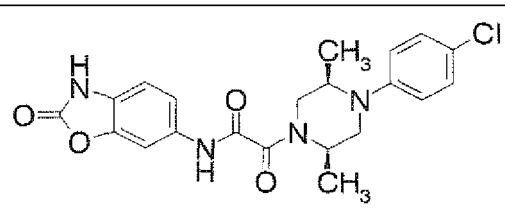 | **116-117** |

(continued)

| | | |
|---|---|---|
| I-9 | 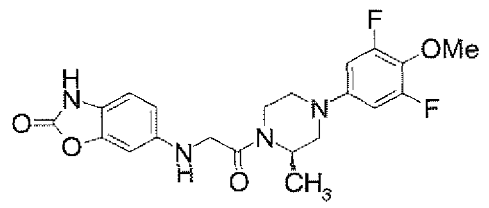 | 202-204 |
| I-10 | 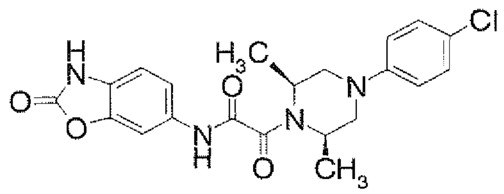 | 235-237 |
| I-11 | 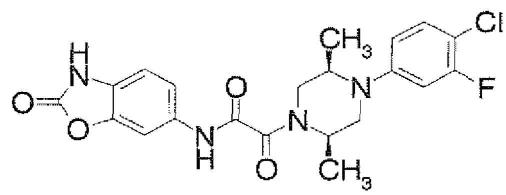 | 116-118 |
| I-12 | 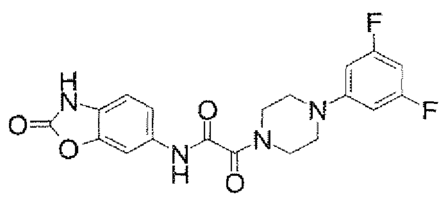 | 242-244 |
| I-13 | 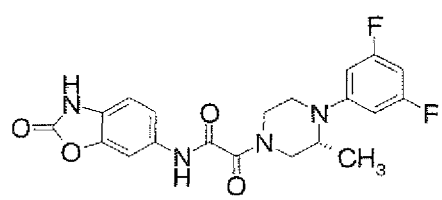 | 223-225 |
| I-14 | 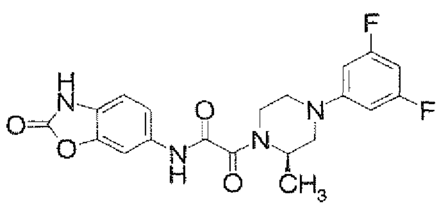 | 219-221 |

[Table 6]

| | | |
|---|---|---|
| I-15 | 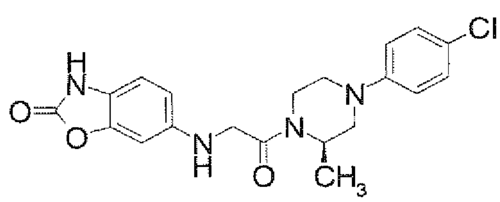 | 201-203 |

(continued)

| | | |
|---|---|---|
| I-16 | 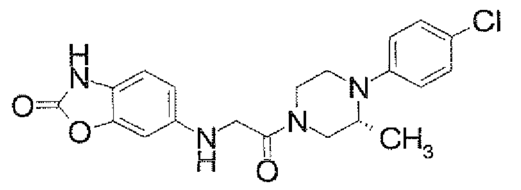 | 122-123 |
| I-17 | 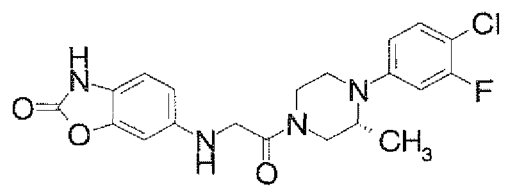 | 194-196 |
| I-18 | 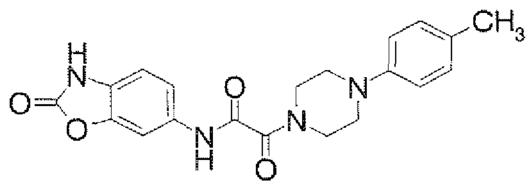 | 244-246 |
| I-19 | 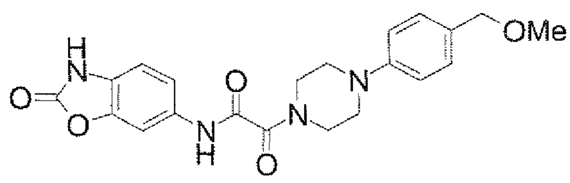 | 174-176 |
| I-20 | 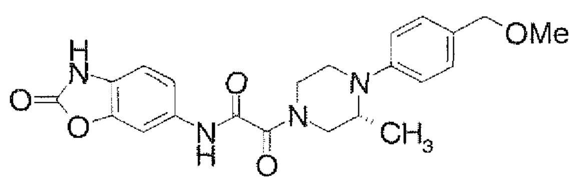 | 196-198 |
| I-21 | 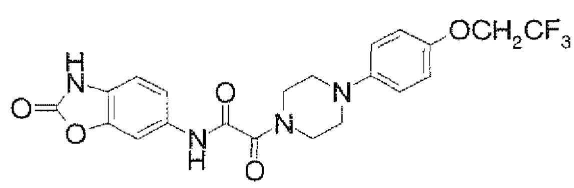 | 227-229 |

[Table 7]

| | | |
|---|---|---|
| I-22 | 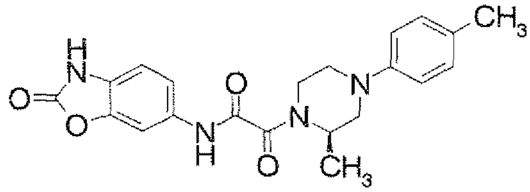 | 125-127 |

(continued)

| | | |
|---|---|---|
| I-23 | 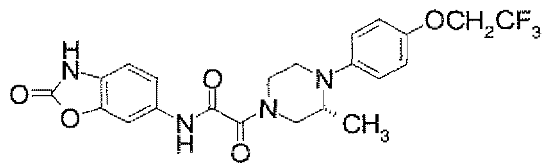 | 103-105 |
| I-24 | 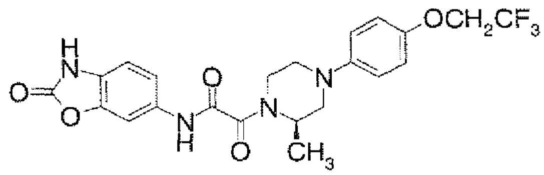 | 204-206 |
| I-25 | 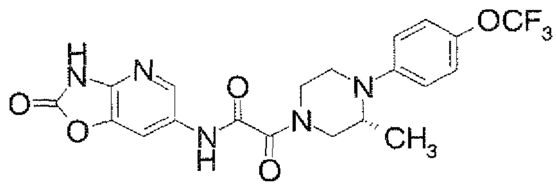 | 254-256 |
| I-26 | 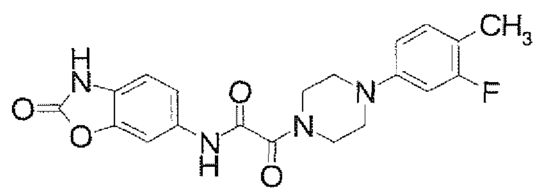 | 248-249 |
| I-27 | 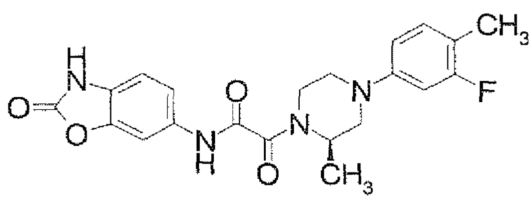 | 125 |
| I-28 | 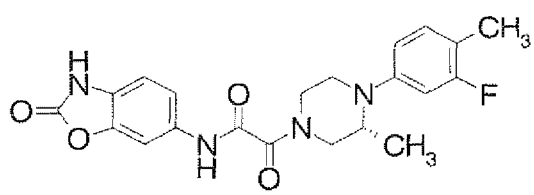 | 124 |

[Table 8]

| | | |
|---|---|---|
| I-29 | 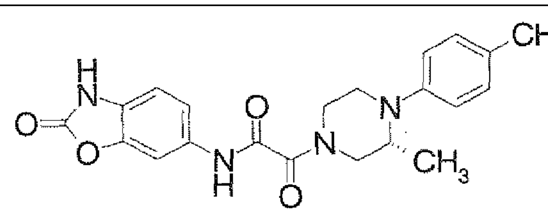 | 212-214 |

(continued)

| | | |
|---|---|---|
| I-30 | 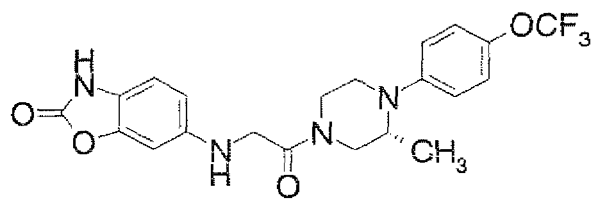 | 175-177 |
| I-31 | 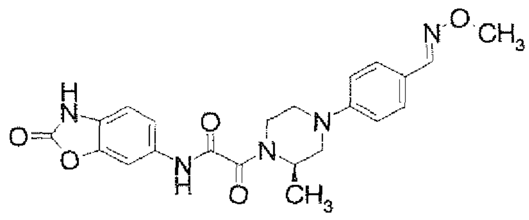 | 117-119 |
| I-32 | 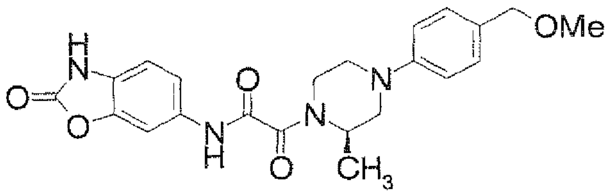 | 142-144 |
| I-33 | 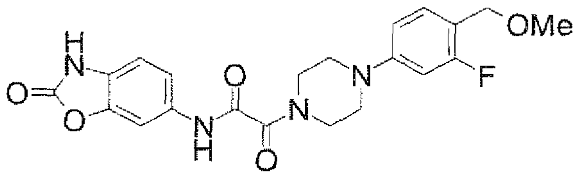 | 187-189 |
| I-34 | 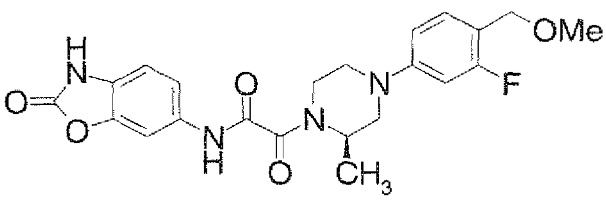 | 103-105 |

[Table 9]

| | | |
|---|---|---|
| I-35 | 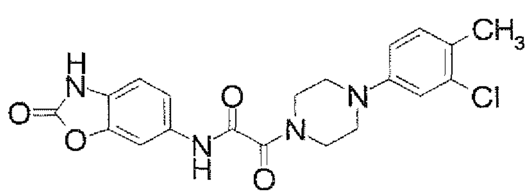 | 235-236 |
| I-36 | 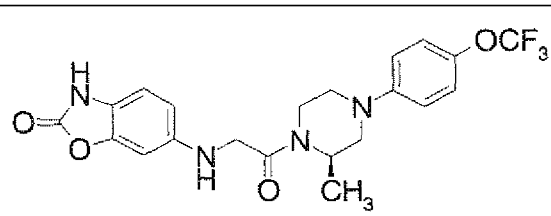 | 185-187 |

(continued)

| | | |
|---|---|---|
| I-37 | 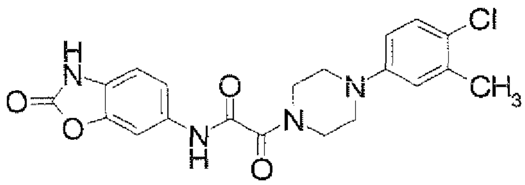 | 230-232 |
| I-38 | 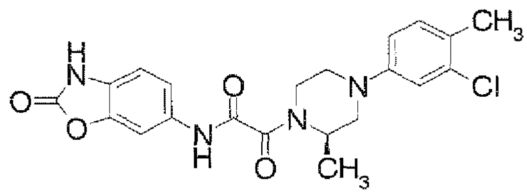 | 130 |
| I-39 | 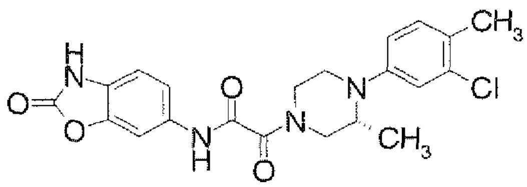 | 196-198 |
| I-40 | 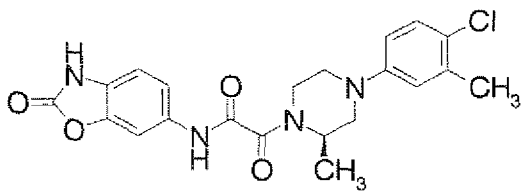 | 123-124 |
| I-41 | 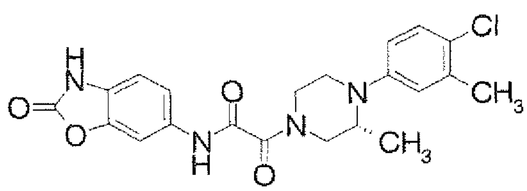 | 125-127 |

[Table 10]

| | | |
|---|---|---|
| I-42 | 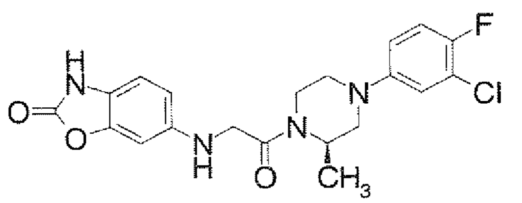 | 215-217 |
| I-43 | 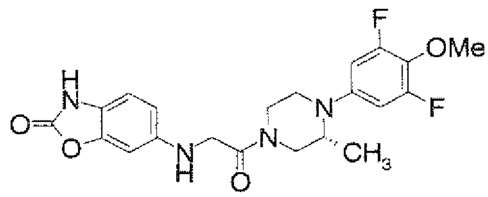 | 162-164 |

(continued)

| | | |
|---|---|---|
| I-44 | 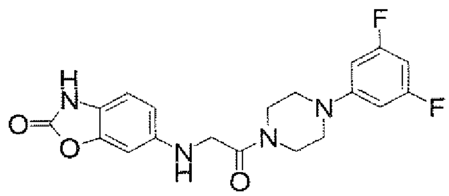 | 233-235 |
| I-45 | 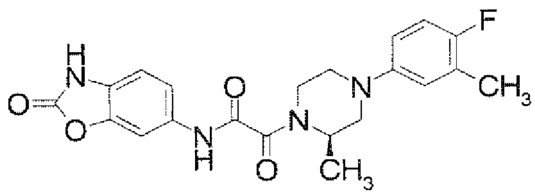 | 116-118 |
| I-46 | 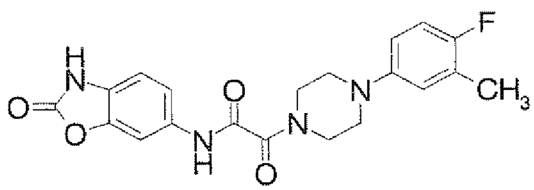 | 234-236 |
| I-47 | 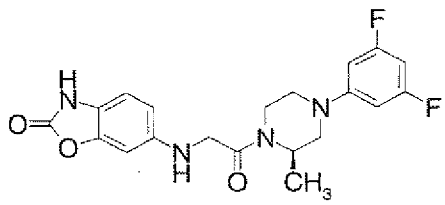 | 234-236 |
| I-48 | 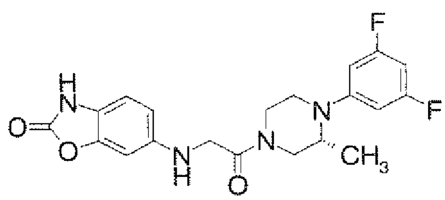 | 199-201 |

[Table 11]

| | | |
|---|---|---|
| I-49 | 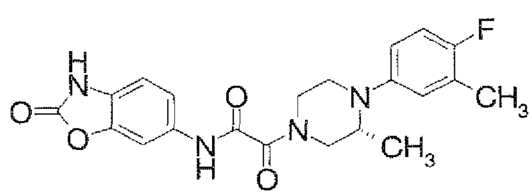 | 111-113 |
| I-50 | 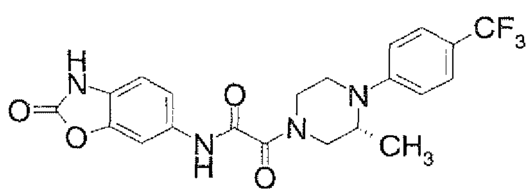 | 206-208 |

(continued)

| | | |
|---|---|---|
| I-51 | 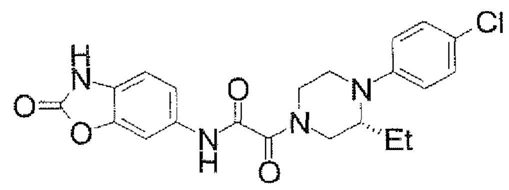 | 150-152 |
| I-52 | 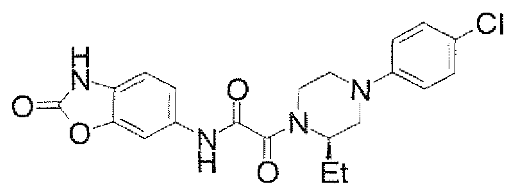 | 125-127 |
| I-53 | 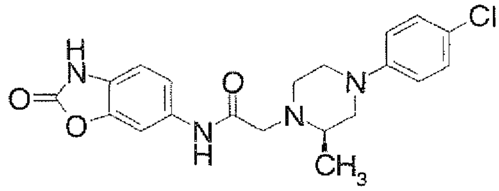 | 201-204 |
| I-54 | 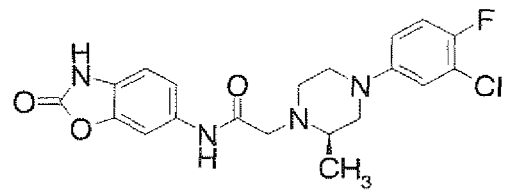 | 191-193 |
| I-55 | 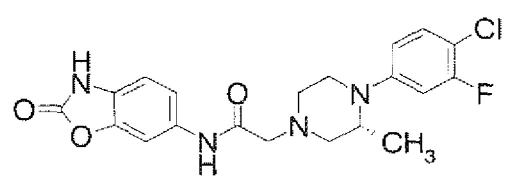 | 180-182 |

[Table 12]

| | | |
|---|---|---|
| I-56 | 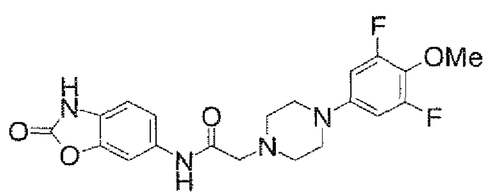 | 233-235 |
| I-57 | 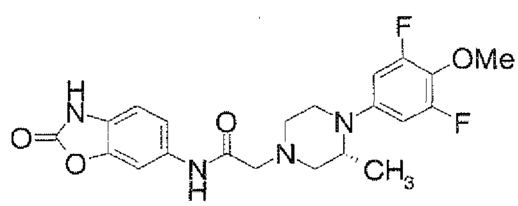 | 220-222 |

(continued)

| | | |
|---|---|---|
| I-58 | 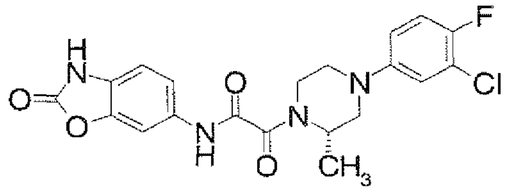 | 193-195 |
| I-59 | 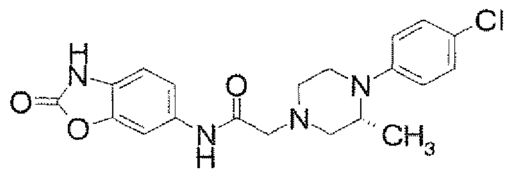 | 204-206 |
| I-60 | 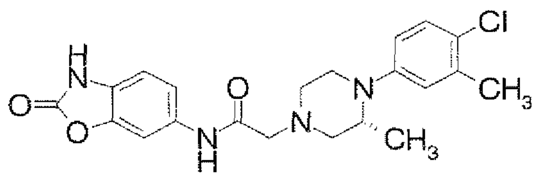 | 169-171 |
| I-61 | 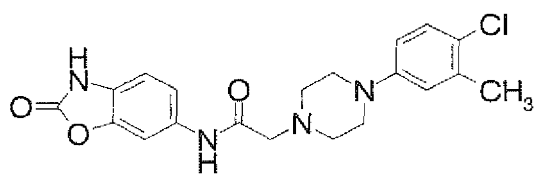 | 212-215 |
| I-62 | 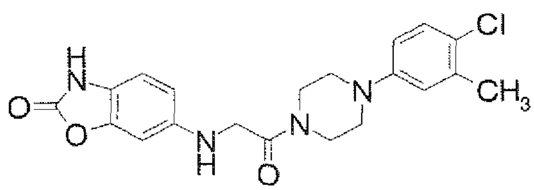 | 219-221 |

[Table 13]

| | | |
|---|---|---|
| I-63 | 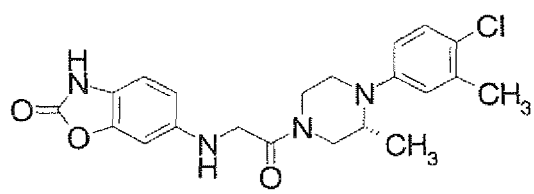 | 112-114 |
| I-64 | 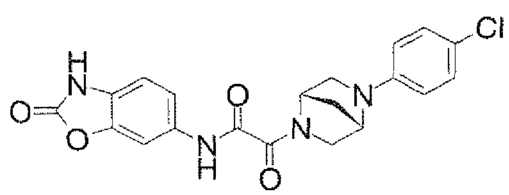 | >300 |

(continued)

| | | |
|---|---|---|
| I-65 | 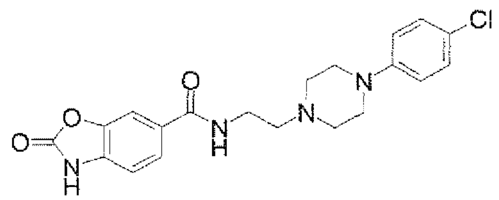 | 244-246 |
| I-66 | 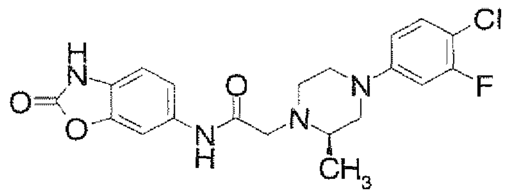 | 156-158 |
| I-67 | 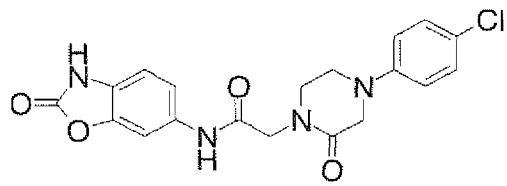 | 223-225 |
| I-68 | 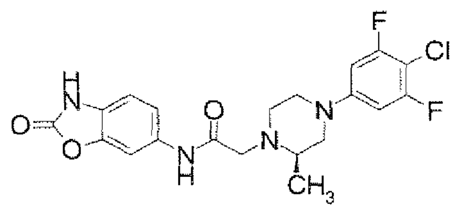 | 175-177 |
| I-69 | 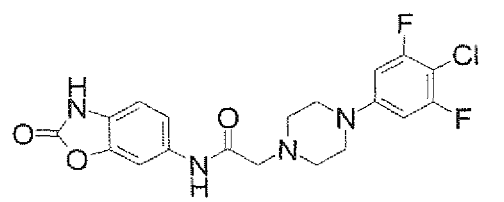 | 237-239 |

[Table 14]

| | | |
|---|---|---|
| I-70 | 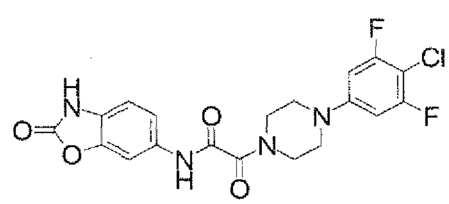 | 269-271 |
| I-71 | 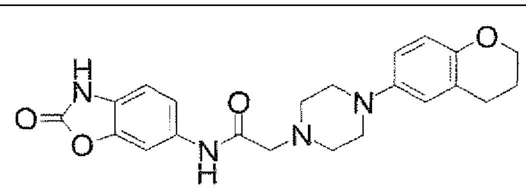 | 219-221 |

(continued)

| | | |
|---|---|---|
| **I-72** | 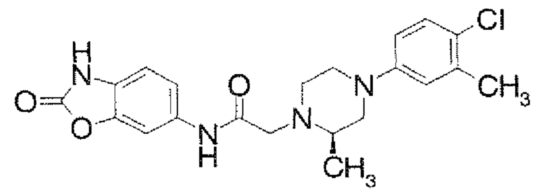 | **170-172** |
| **I-73** | 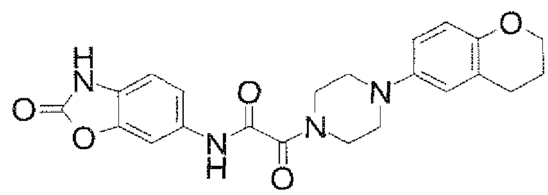 | **233-234** |
| **I-74** | 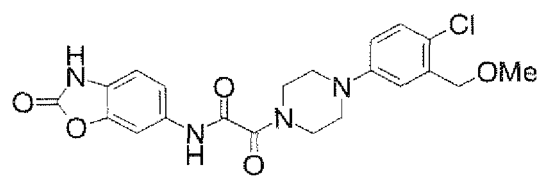 | **225-226** |
| **I-75** | 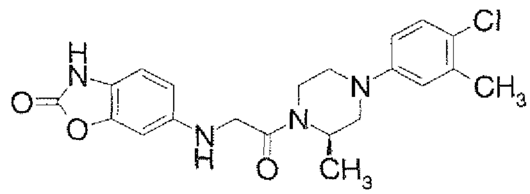 | **255-257** |
| **I-76** | 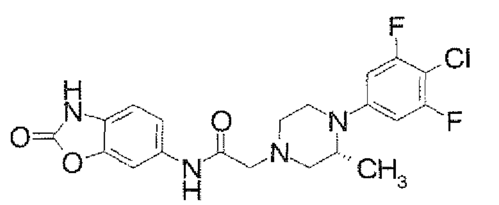 | **115-117** |

[Table 15]

| | | |
|---|---|---|
| **I-77** | 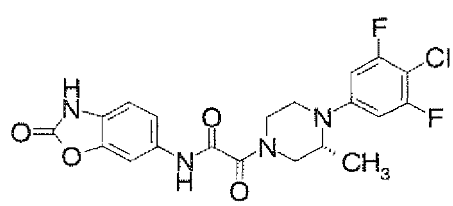 | **251-253** |
| **I-78** | 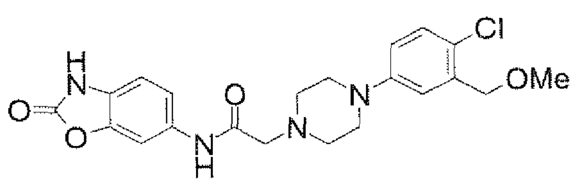 | **202-204** |

(continued)

| | | |
|---|---|---|
| **I-79** | 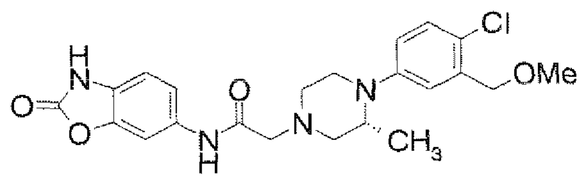 | **152-154** |
| **I-80** | 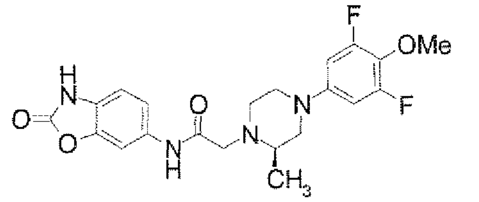 | |
| **I-81** | 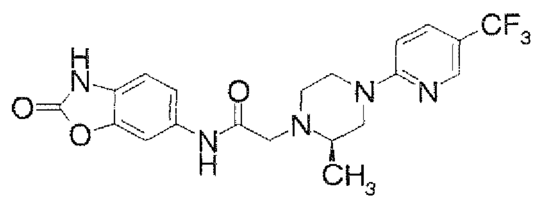 | |
| **I-82** | 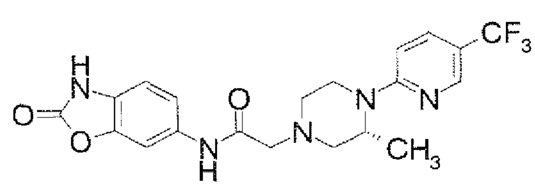 | |
| **P83** | 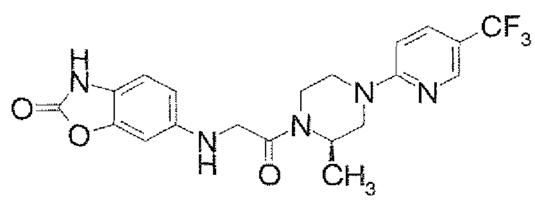 | |

[Table 16]

| | | |
|---|---|---|
| **I-84** | 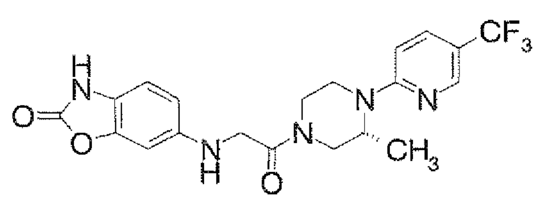 | |
| **I-85** | 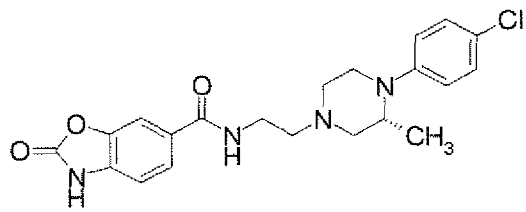 | |

(continued)

| | | |
|---|---|---|
| **I-86** | 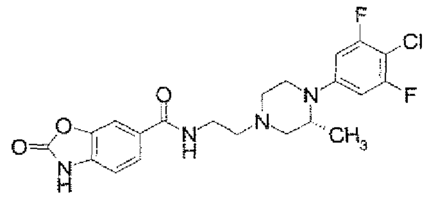 | |
| **I-87** | 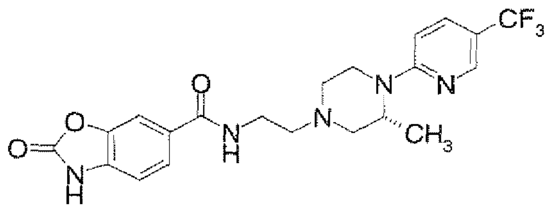 | |
| **I-88** | 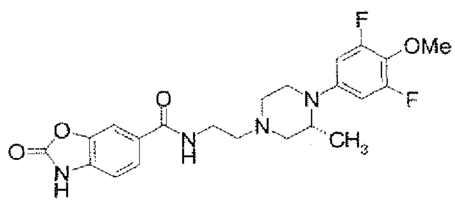 | |
| **I-89** | 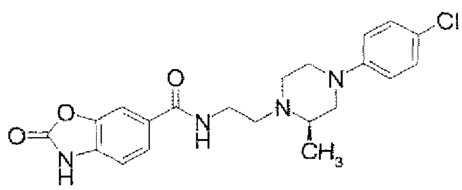 | |
| **I-90** | 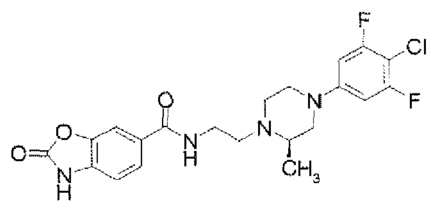 | |

[Table 17]

| | | |
|---|---|---|
| **I-91** | 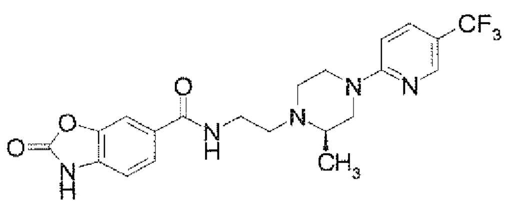 | |
| **I-92** | 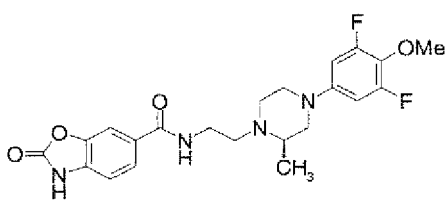 | |

(continued)

| | | |
|---|---|---|
| I-93 | 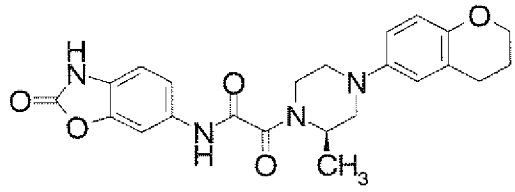 | |
| I-94 | 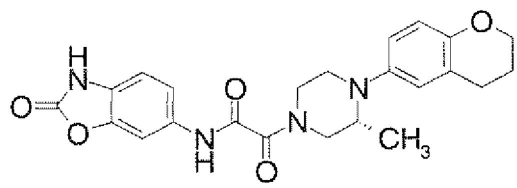 | |
| I-95 | 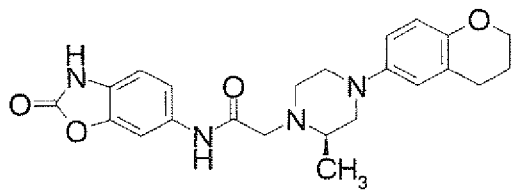 | |
| I-96 | 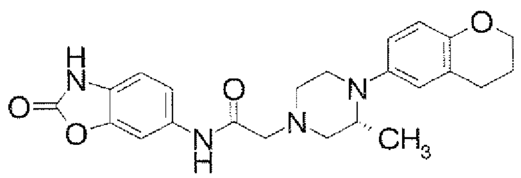 | |
| I-97 | 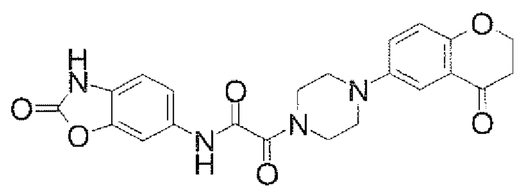 | |

[Table 18]

| | | |
|---|---|---|
| I-98 | 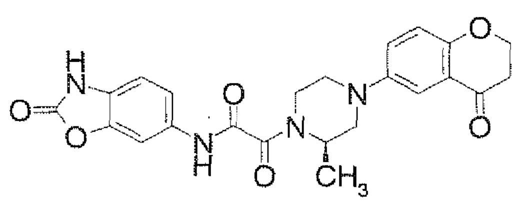 | |
| I-99 | 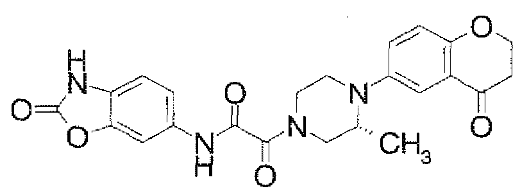 | |

(continued)

| | | |
|---|---|---|
| I-100 | 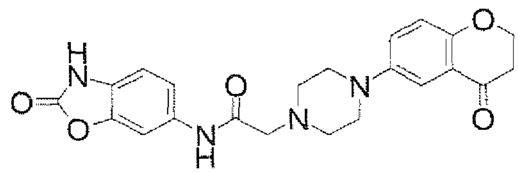 | |
| I-101 | 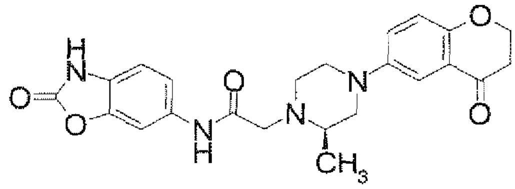 | |
| I-102 | 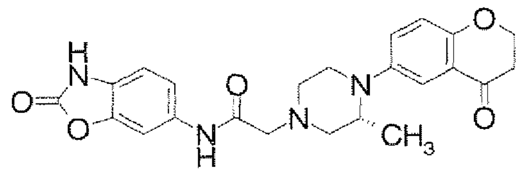 | |
| I-103 | 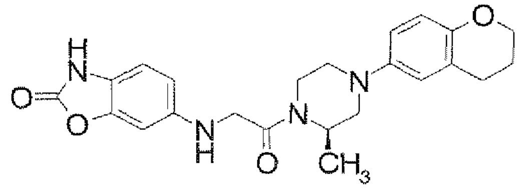 | |
| I-104 | 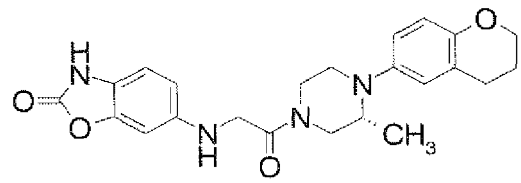 | |

[Table 19]

| | | |
|---|---|---|
| I-105 | 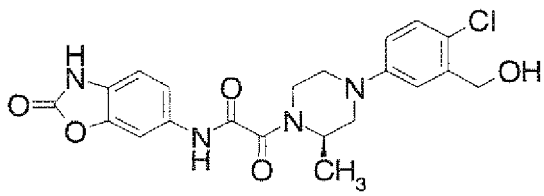 | |
| I-106 | 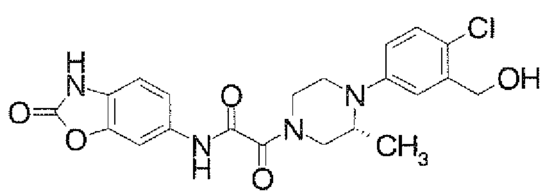 | |

(continued)

| | | |
|---|---|---|
| **I-107** | 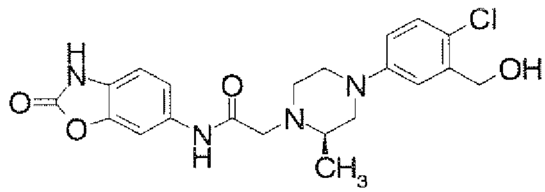 | |
| **I-108** | 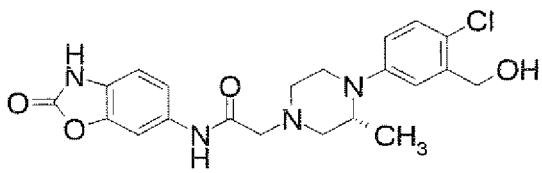 | |
| **I-109** | 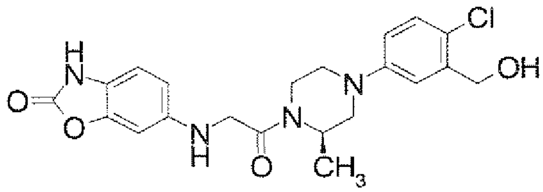 | |
| **I-110** | 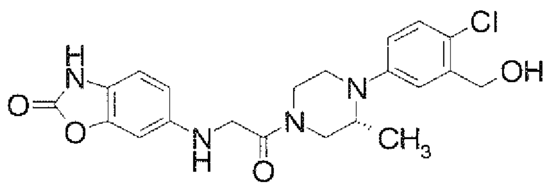 | |
| **I-111** | 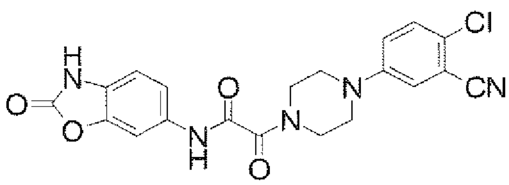 | |

[Table 20]

| | | |
|---|---|---|
| **I-112** | 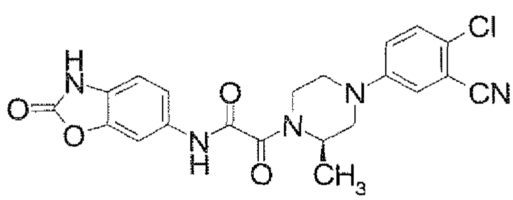 | |
| **I-113** | 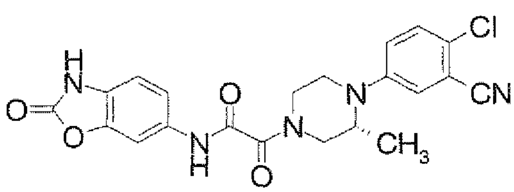 | |

(continued)

| | | |
|---|---|---|
| I-114 | 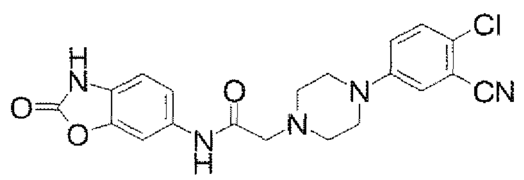 | |
| I-115 | 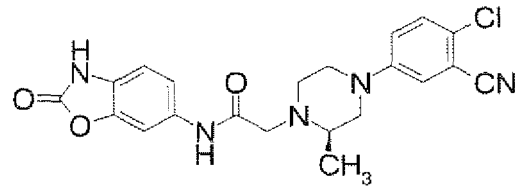 | |
| I-116 | 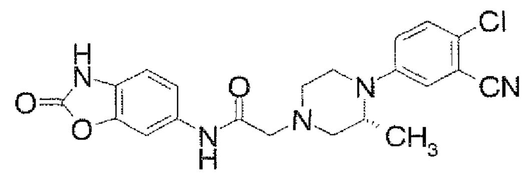 | |
| I-117 | 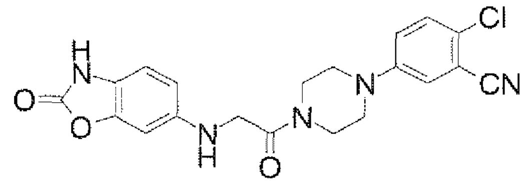 | |
| I-118 | 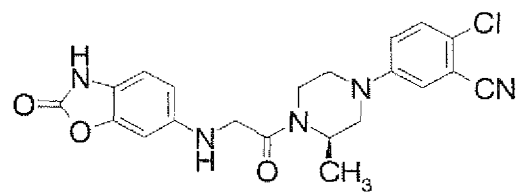 | |

[Table 21]

| | | |
|---|---|---|
| I-119 | 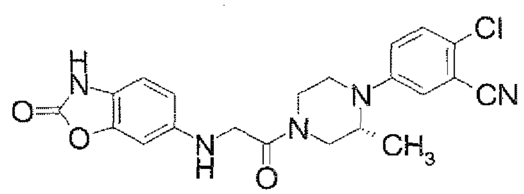 | |
| I-120 | 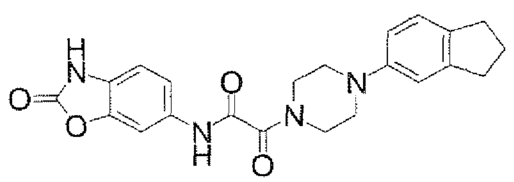 | |

(continued)

| | | |
|---|---|---|
| I-121 | 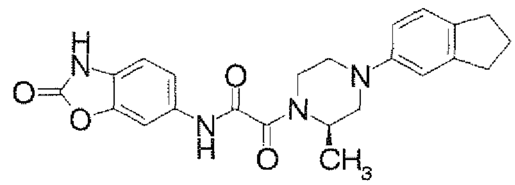 | |
| I-122 | 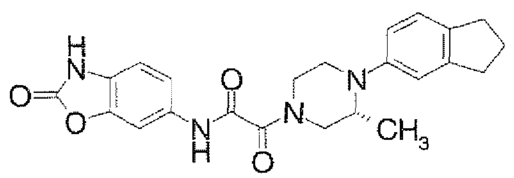 | |
| I-123 | 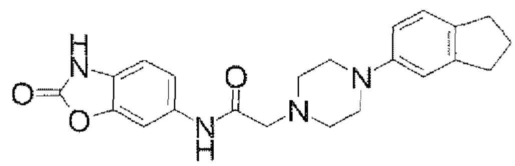 | |
| I-124 | 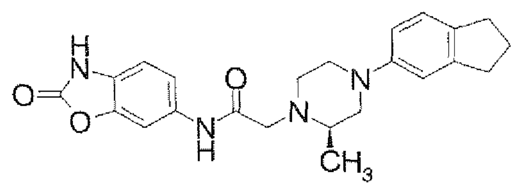 | |
| I-125 | 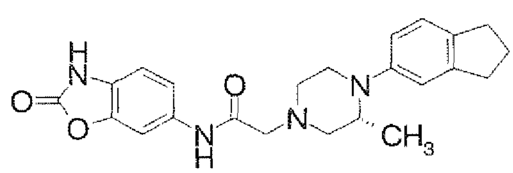 | |

[Table 22]

| | | |
|---|---|---|
| I-126 | 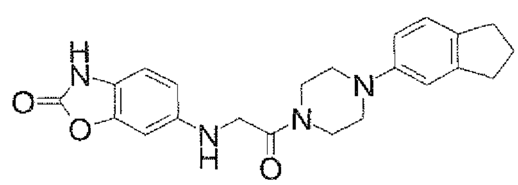 | |
| I-127 | 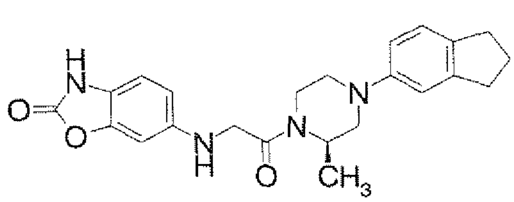 | |

(continued)

| | | |
|---|---|---|
| I-128 | 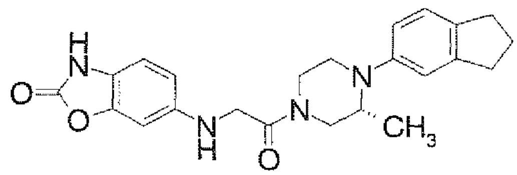 | |
| I-129 | 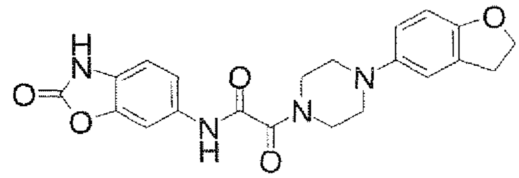 | |
| I-130 | 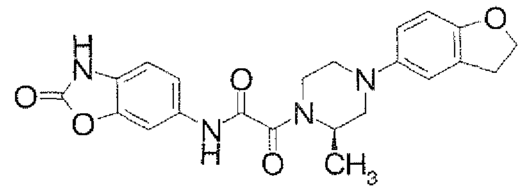 | |
| I-131 | 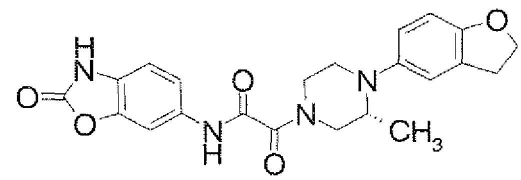 | |
| I-132 | 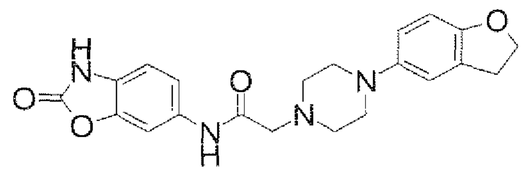 | |

[Table 23]

| | | |
|---|---|---|
| I-133 | 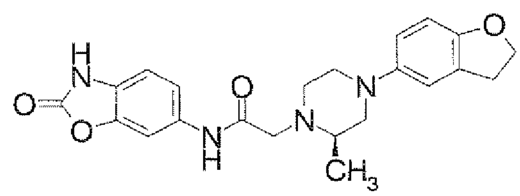 | |
| I-134 | 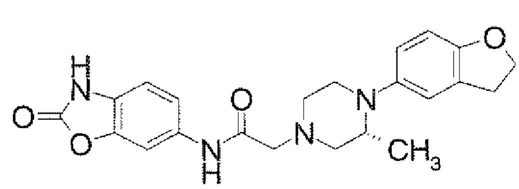 | |

(continued)

| | | |
|---|---|---|
| **I-135** | 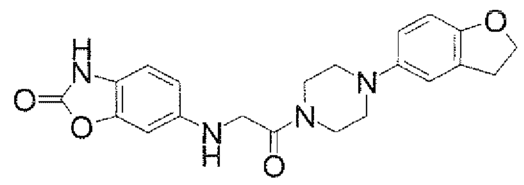 | |
| **I-136** | 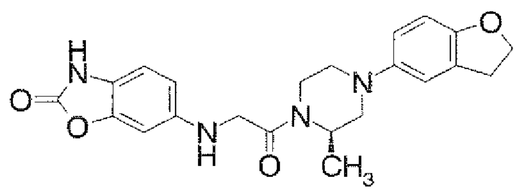 | |
| **I-137** | 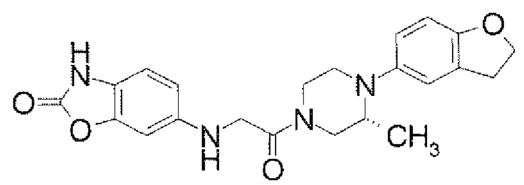 | |
| **I-138** | 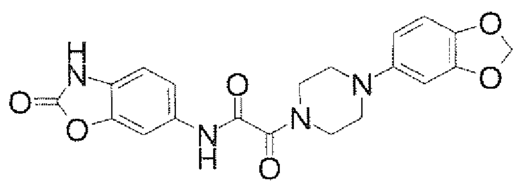 | |
| **I-139** | 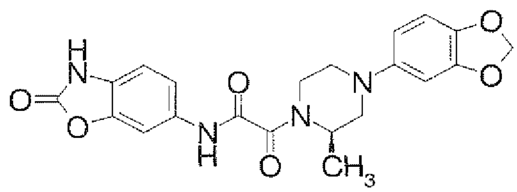 | |

[Table 24]

| | | |
|---|---|---|
| **I-140** | 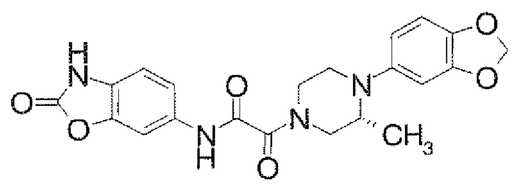 | |
| **I-141** | 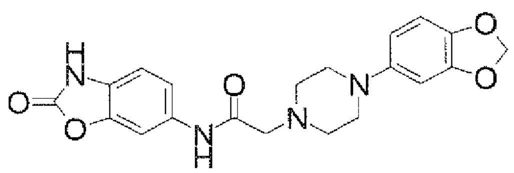 | |

(continued)

| | | |
|---|---|---|
| I-142 | 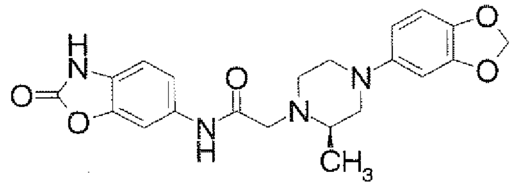 | |
| I-143 | 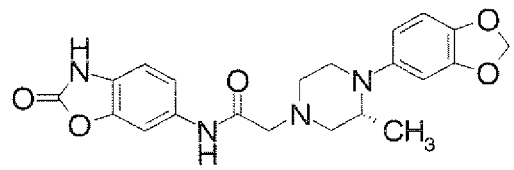 | |
| I-144 | 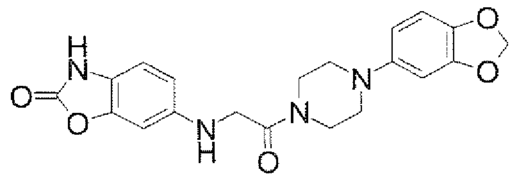 | |
| I-145 | 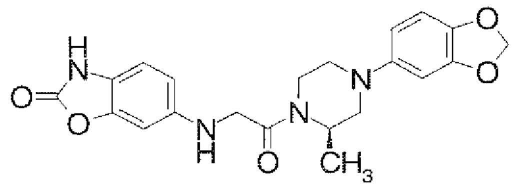 | |
| I-146 | 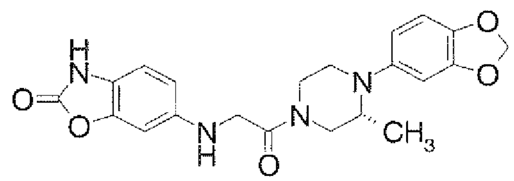 | |

[Table 25]

| | | |
|---|---|---|
| I-147 | 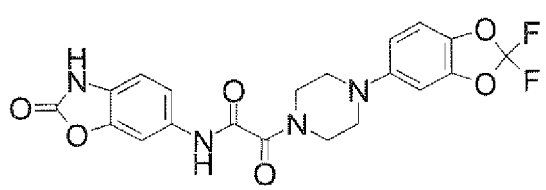 | |
| I-148 | 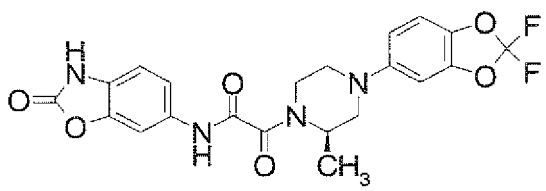 | |

(continued)

| | | |
|---|---|---|
| I-149 | 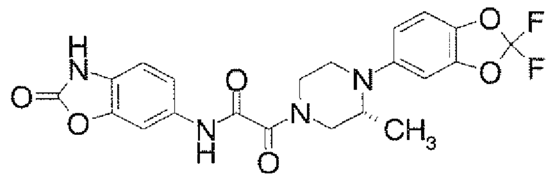 | |
| I-150 | 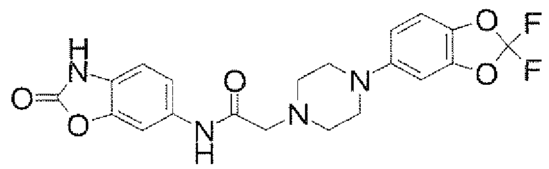 | |
| I-151 | 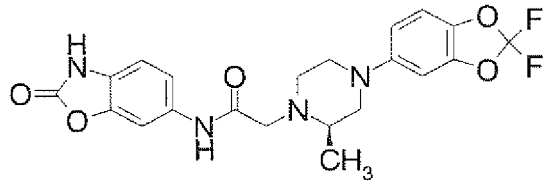 | |
| I-152 | 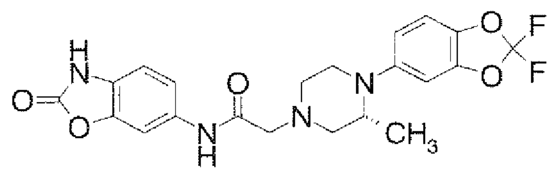 | |
| I-153 | 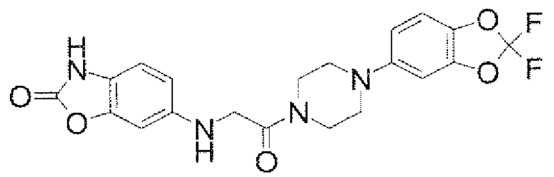 | |

[Table 26]

| | | |
|---|---|---|
| I-154 | 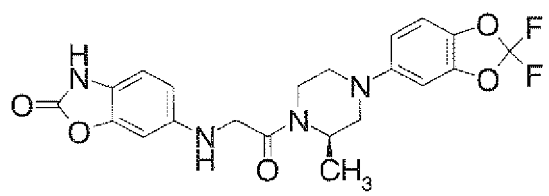 | |
| I-155 | 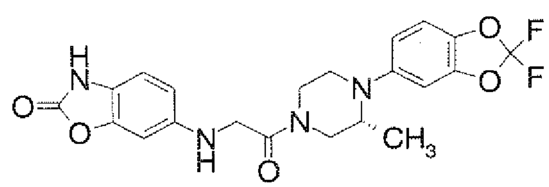 | |

(continued)

| | | |
|---|---|---|
| I-156 | 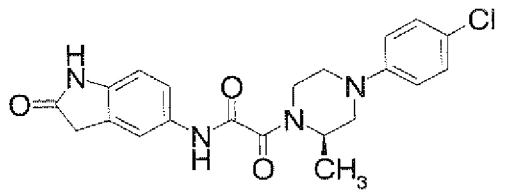 | |
| I-157 | 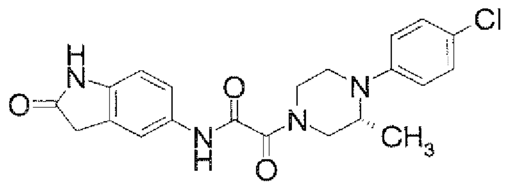 | |
| I-158 | 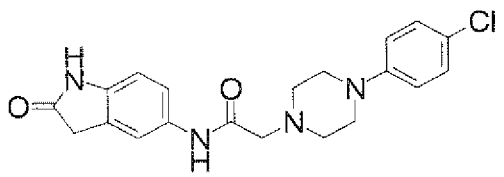 | |
| I-159 | 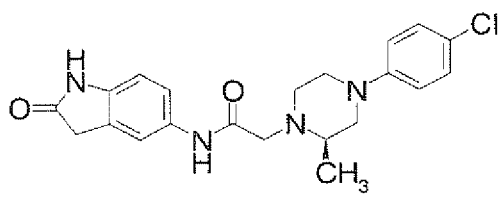 | |
| I-160 | 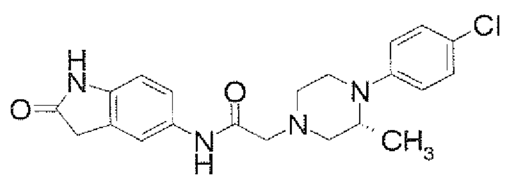 | |

[Table 27]

| | | |
|---|---|---|
| I-161 | 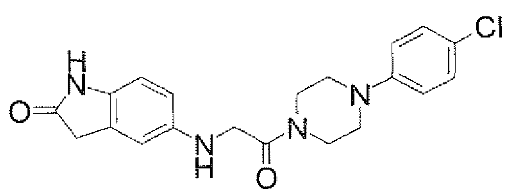 | |
| I-162 | 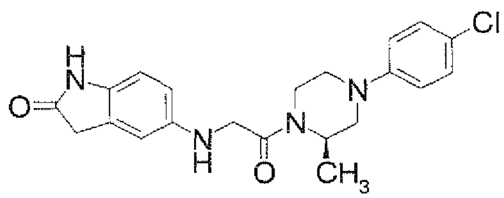 | |

(continued)

| | | |
|---|---|---|
| I-163 | 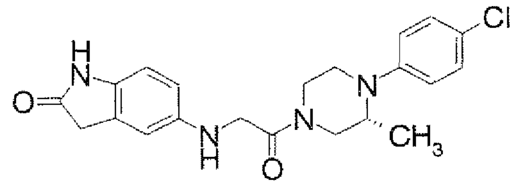 | |
| I-164 | 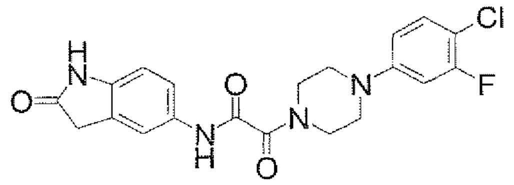 | |
| I-165 | 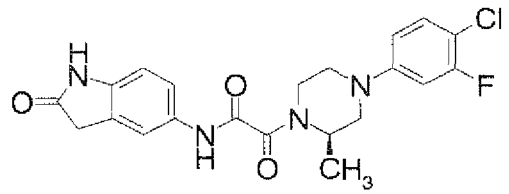 | |
| I-166 | 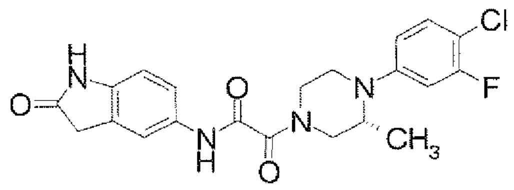 | |
| I-167 | 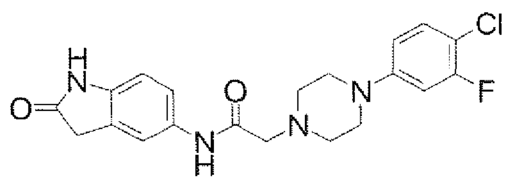 | |

[Table 28]

| | | |
|---|---|---|
| I-168 | 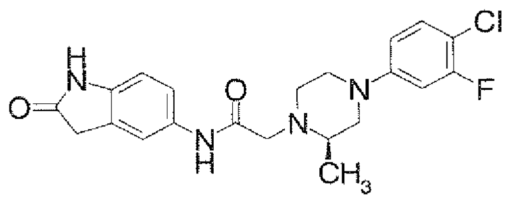 | |
| I-169 | 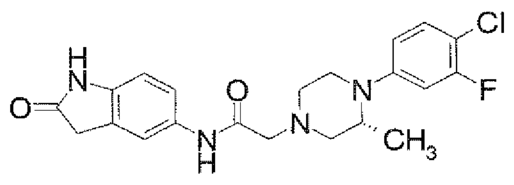 | |

(continued)

| | | |
|---|---|---|
| I-170 | 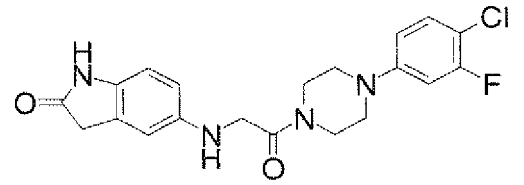 | |
| I-171 | 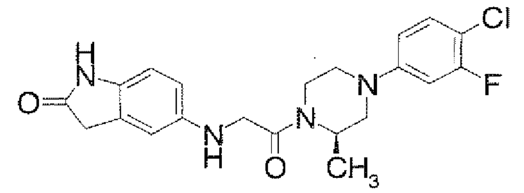 | |
| I-172 | 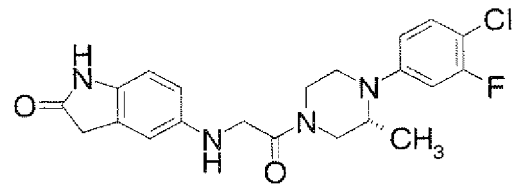 | |
| I-173 | 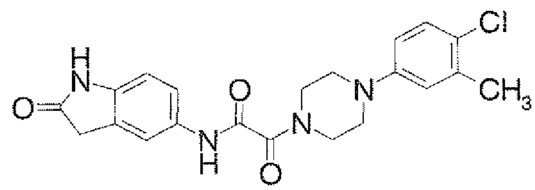 | |
| I-174 | 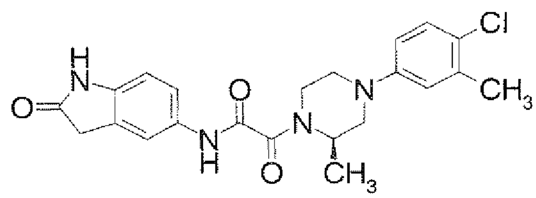 | |

[Table 29]

| | | |
|---|---|---|
| I-175 | 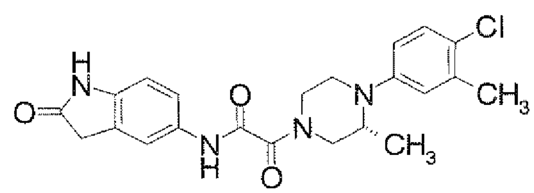 | |
| I-176 | 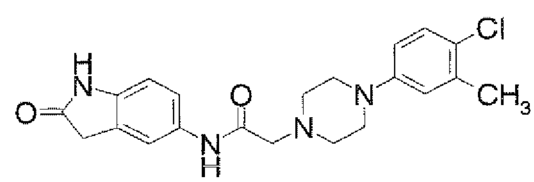 | |

(continued)

| | | |
|---|---|---|
| I-177 | 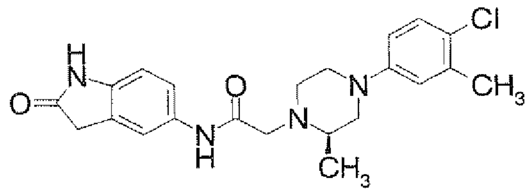 | |
| I-178 | 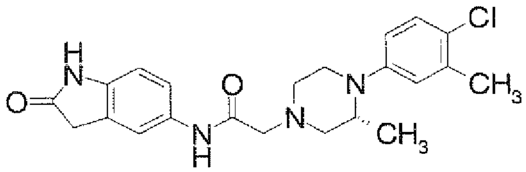 | |
| I-179 | 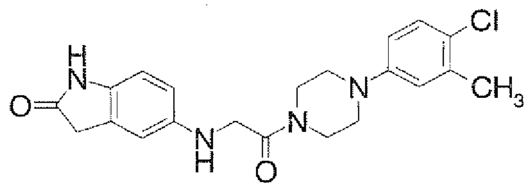 | |
| I-180 | 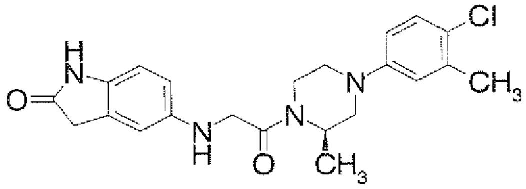 | |
| I-181 | 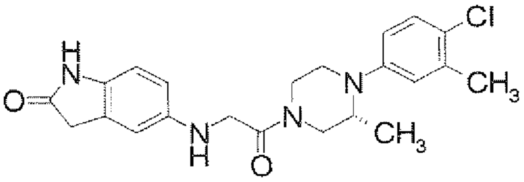 | |

[Table 30]

| | | |
|---|---|---|
| I-182 | 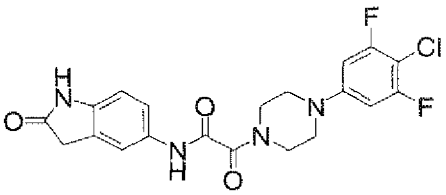 | |
| I-183 | 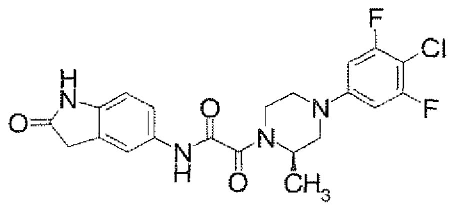 | |

(continued)

| | | |
|---|---|---|
| I-184 | 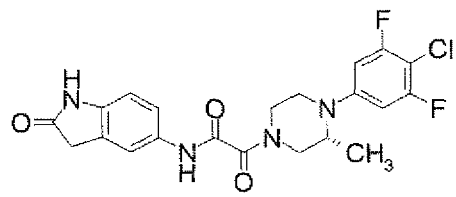 | |
| I-185 | 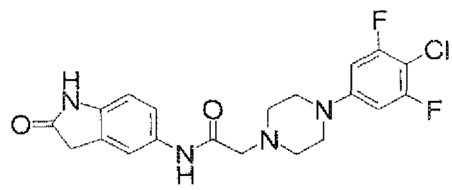 | |
| I-186 | 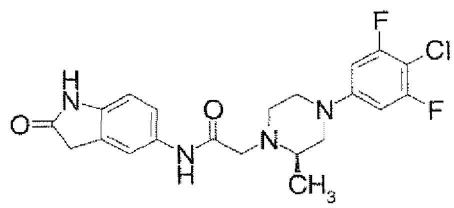 | |
| I-187 | 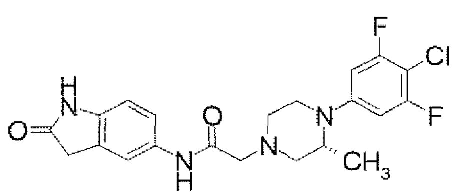 | |
| I-188 | 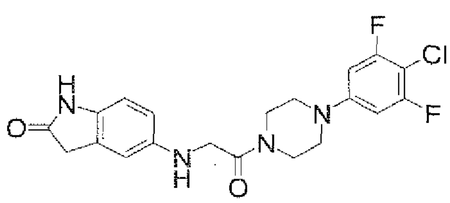 | |

[Table 31]

| | | |
|---|---|---|
| I-189 | 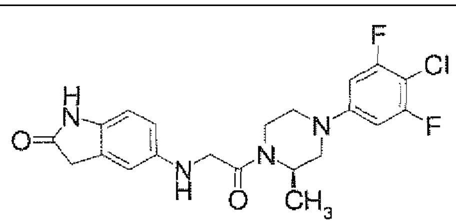 | |
| I-190 | 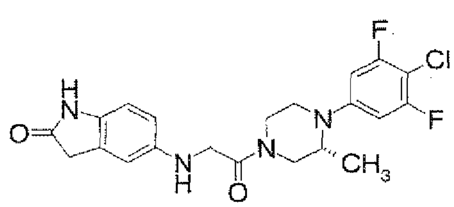 | |

(continued)

| | | |
|---|---|---|
| **I-191** | 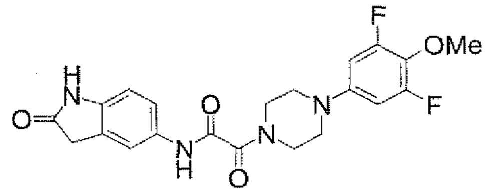 | |
| **I-192** | 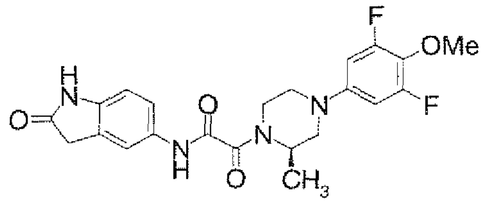 | |
| **I-193** | 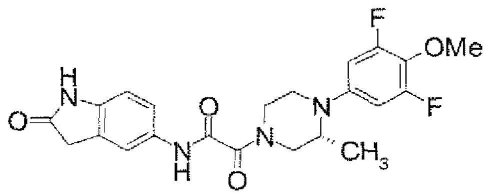 | |
| **I-194** | 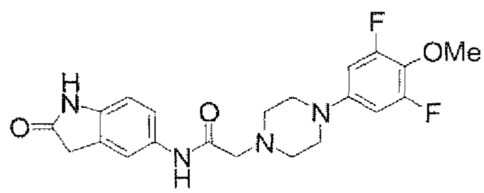 | |
| **I-195** | 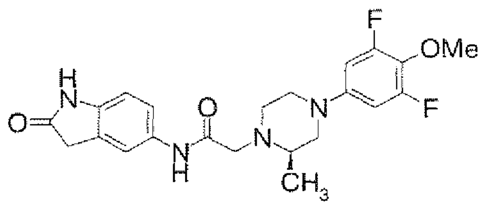 | |

[Table 32]

| | | |
|---|---|---|
| **I-196** | 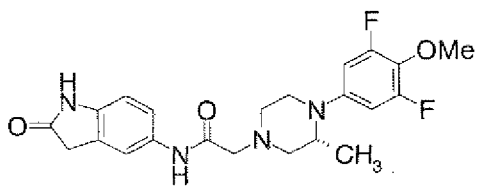 | |
| **I-197** | 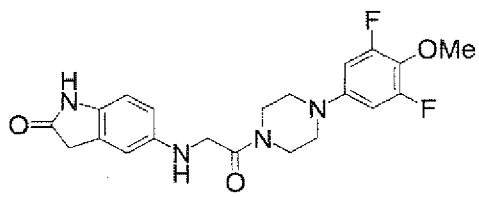 | |

(continued)

| | | |
|---|---|---|
| I-198 | 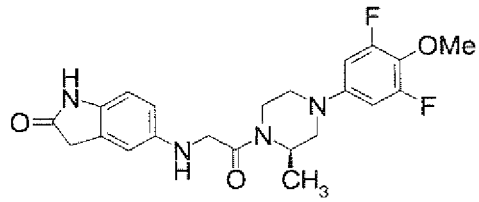 | |
| I-199 | 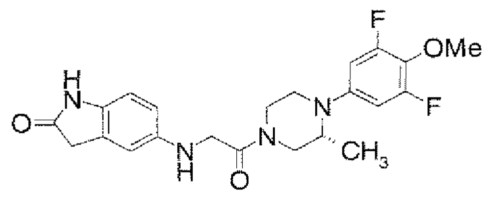 | |
| I-200 | 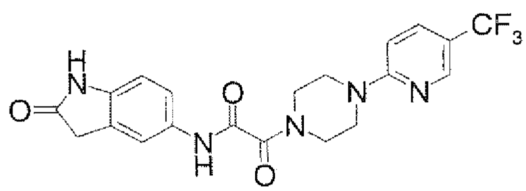 | |
| I-201 | 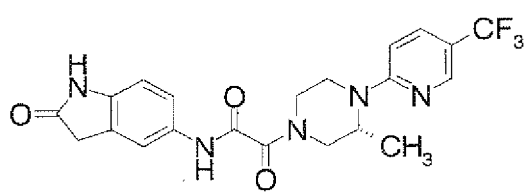 | |
| I-202 | 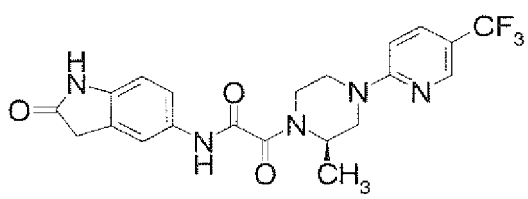 | |

[Table 33]

| | | |
|---|---|---|
| I-203 | 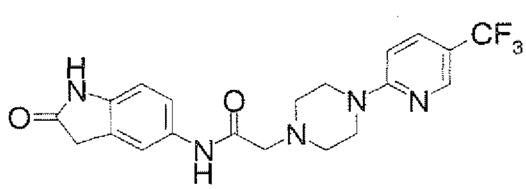 | |
| I-204 | 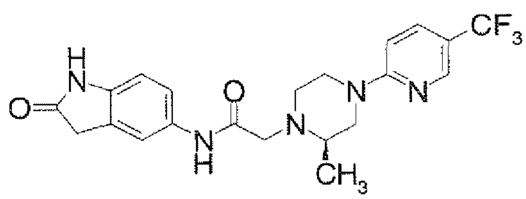 | |

(continued)

| | | |
|---|---|---|
| I-205 | 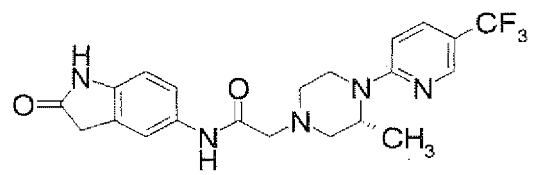 | |
| I-206 | 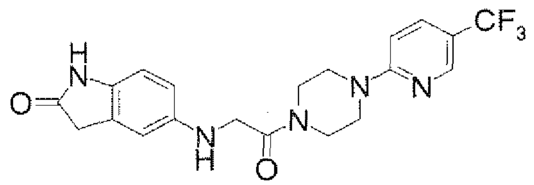 | |
| I-207 | 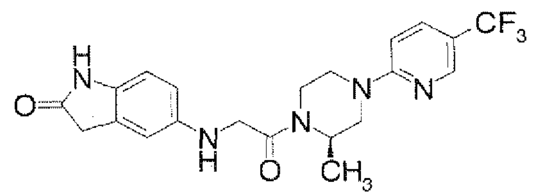 | |
| I-208 | 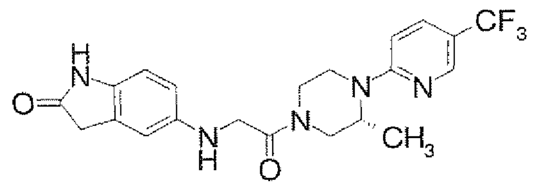 | |
| I-209 | 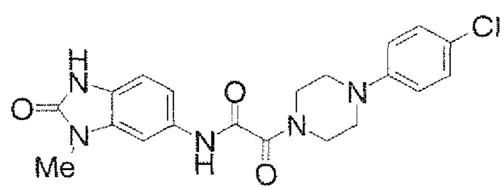 | |

[Table 34]

| | | |
|---|---|---|
| I-210 | 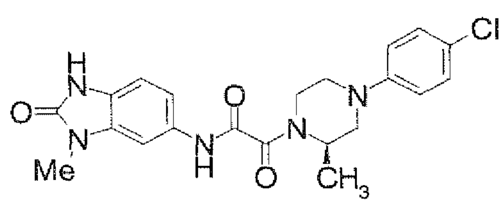 | |
| I-211 | 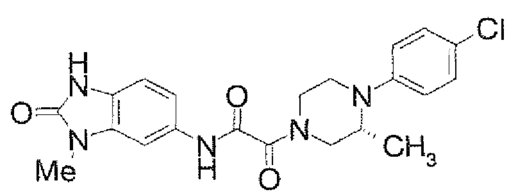 | |

(continued)

| | | |
|---|---|---|
| **I-212** | 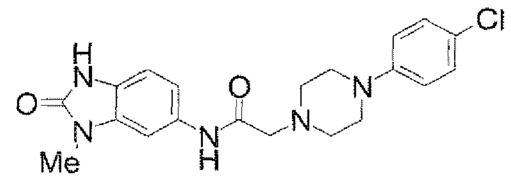 | |
| **I-213** | 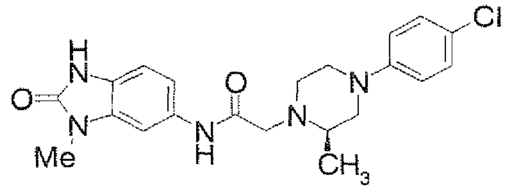 | |
| **I-214** | 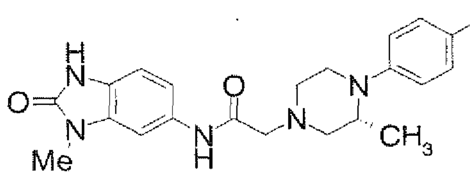 | |
| **I-215** | 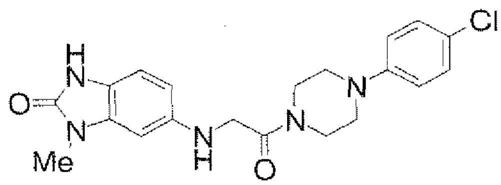 | |
| **I-216** | 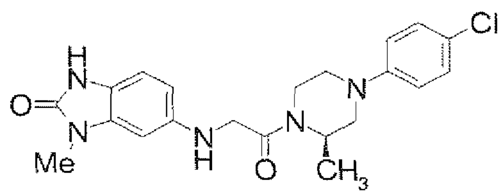 | |

[Table 35]

| | | |
|---|---|---|
| **I-217** | 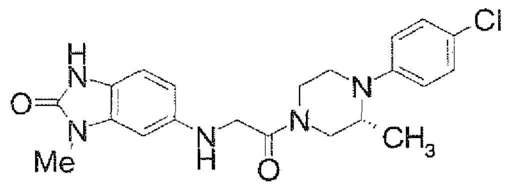 | |
| **I-218** | 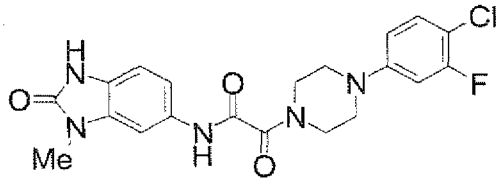 | |

(continued)

| | | |
|---|---|---|
| **I-219** | 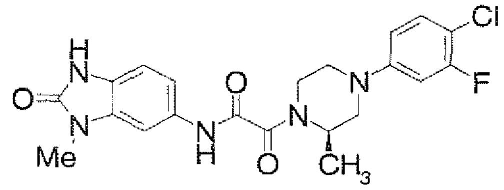 | |
| **I-220** | 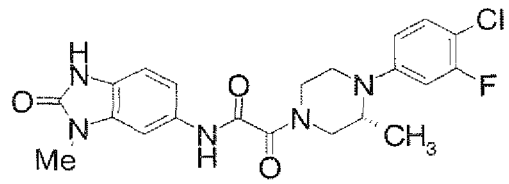 | |
| **I-221** | 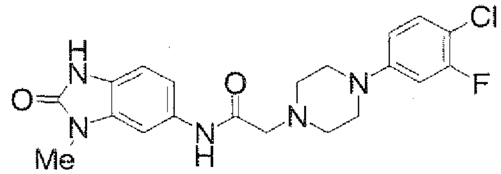 | |
| **I-222** | 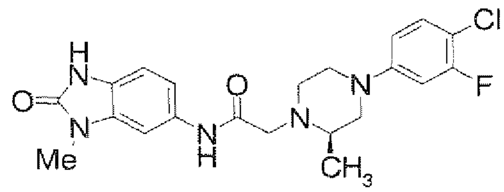 | |
| **I-223** | 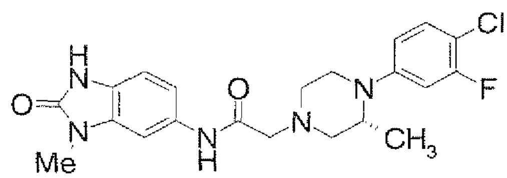 | |

[Table 36]

| | | |
|---|---|---|
| **I-224** | 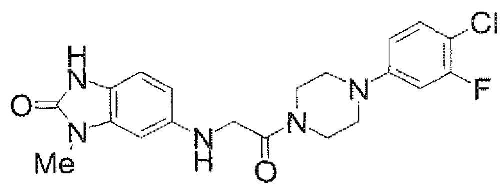 | |
| **I-225** | 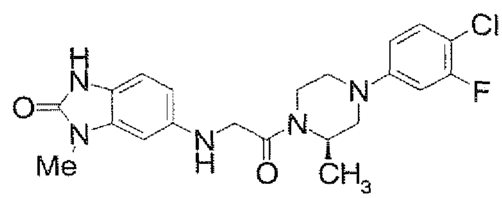 | |

(continued)

| | | |
|---|---|---|
| **I-226** | 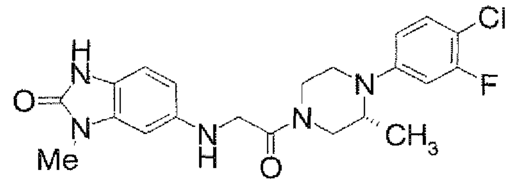 | |
| **I-227** | 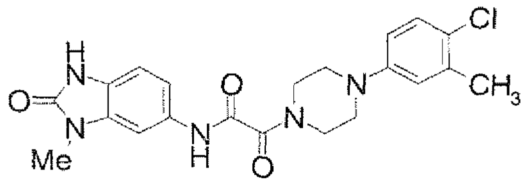 | |
| **I-228** | 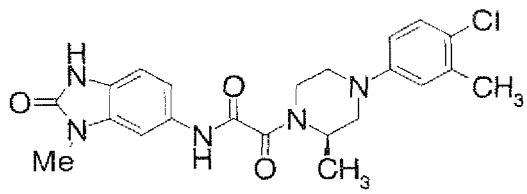 | |
| **I-229** | 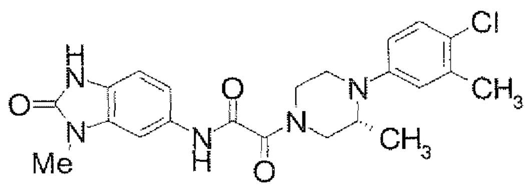 | |
| **I-230** | 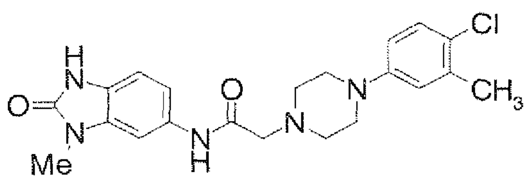 | |

[Table 37]

| | | |
|---|---|---|
| **I-231** | 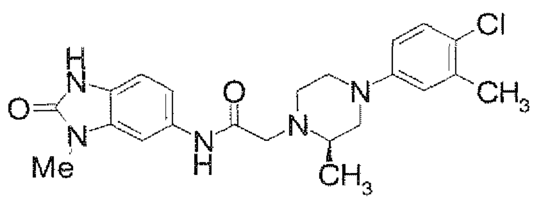 | |
| **I-232** | 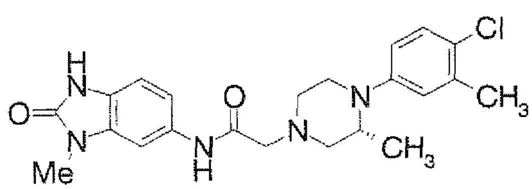 | |

(continued)

| | | |
|---|---|---|
| I-233 | 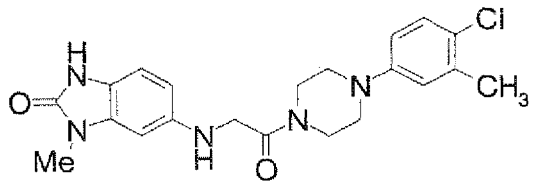 | |
| I-234 | 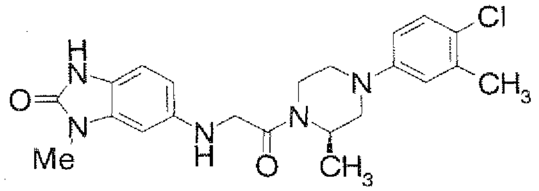 | |
| I-235 | 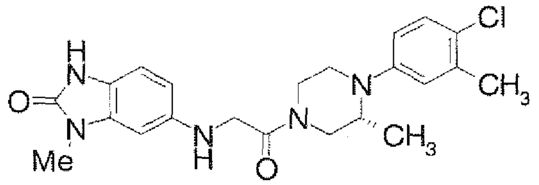 | |
| I-236 | 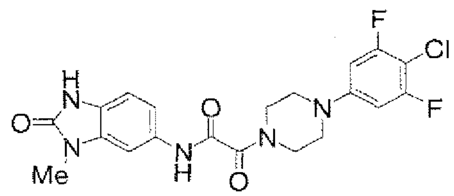 | |
| I-237 | 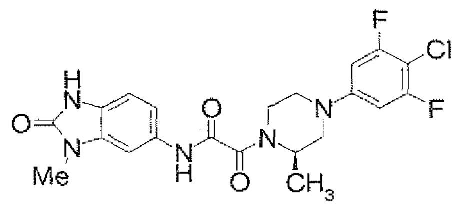 | |

[Table 38]

| | | |
|---|---|---|
| I-238 | 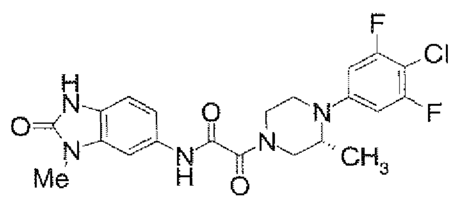 | |
| I-239 | 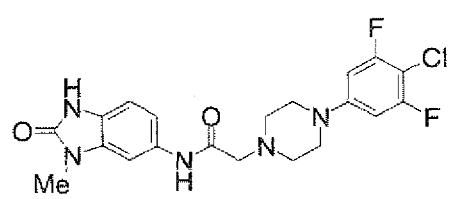 | |

(continued)

| | | |
|---|---|---|
| **I-240** | 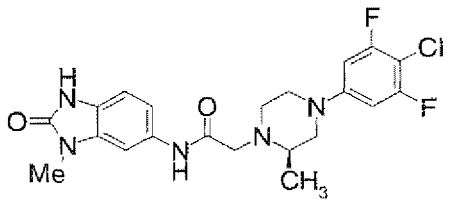 | |
| **I-241** | 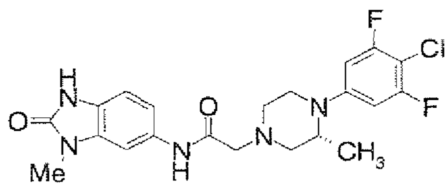 | |
| **I-242** | 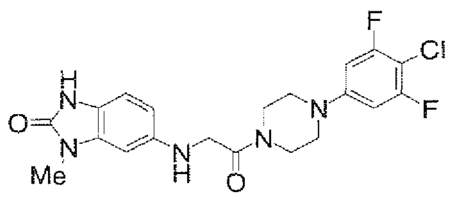 | |
| **I-243** | 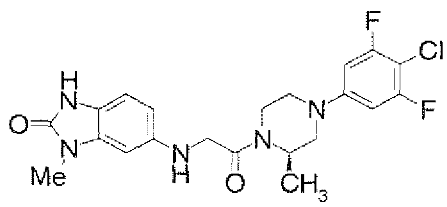 | |
| **I-244** | 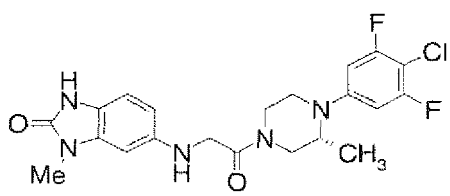 | |

[Table 39]

| | | |
|---|---|---|
| **I-245** | 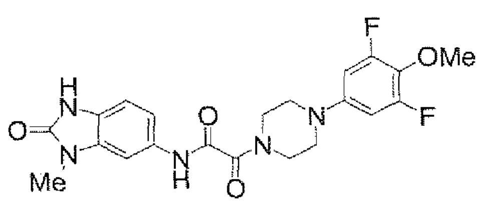 | |
| **I-246** | 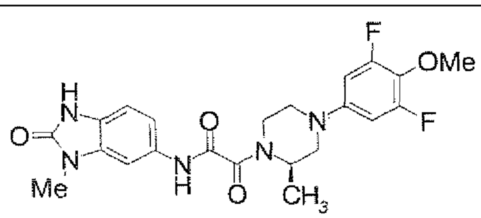 | |

(continued)

| | | |
|---|---|---|
| **I-247** | 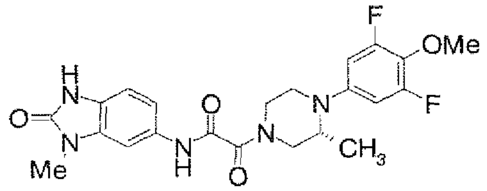 | |
| **I-248** | 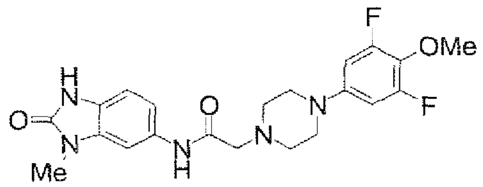 | |
| **I-249** | 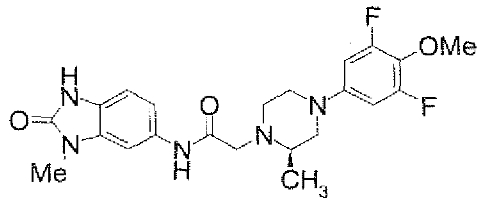 | |
| **I-250** | 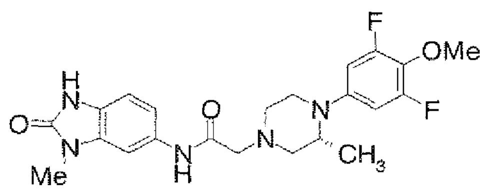 | |
| **I-251** | 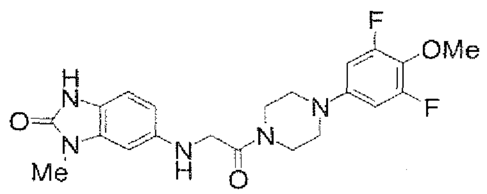 | |

[Table 40]

| | | |
|---|---|---|
| **I-252** | 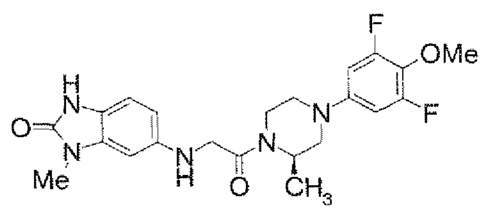 | |
| **I-253** | 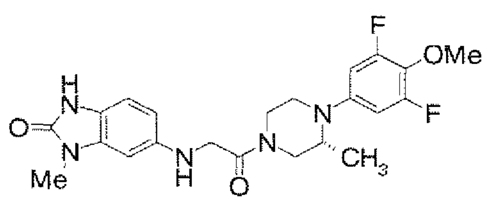 | |

(continued)

| | | |
|---|---|---|
| **I-264** | 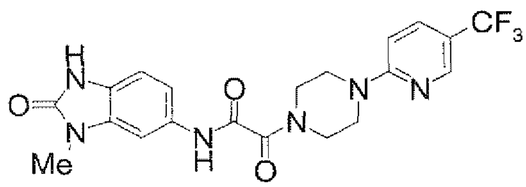 | |
| **I-255** | 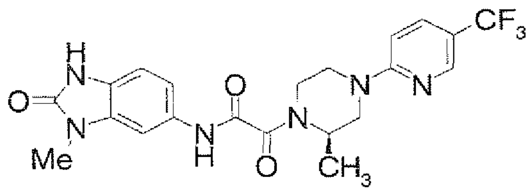 | |
| **I-256** | 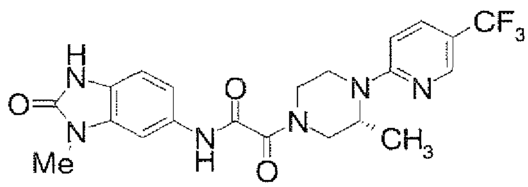 | |
| **I-257** | 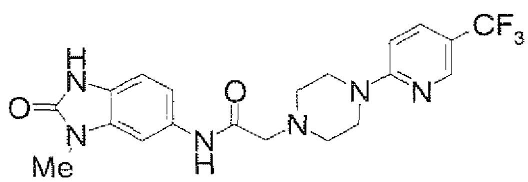 | |
| **I-258** | 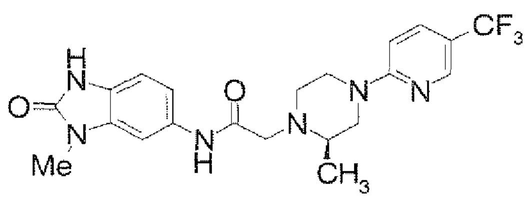 | |

[Table 41]

| | | |
|---|---|---|
| **I-259** | 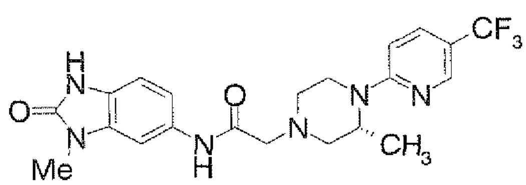 | |
| **I-260** | 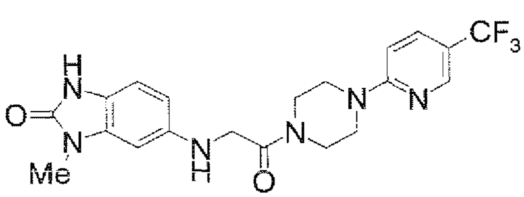 | |

(continued)

| | | |
|---|---|---|
| I-261 | 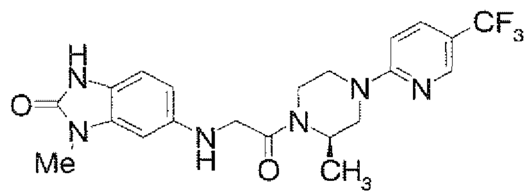 | |
| I-262 | 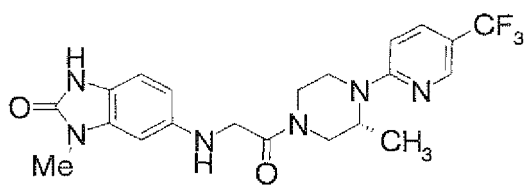 | |
| I-263 | 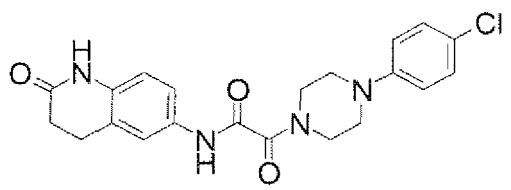 | |
| I-264 | 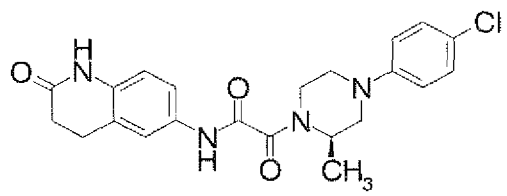 | |
| I-265 | 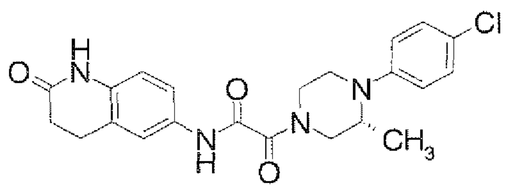 | |

[Table 42]

| | | |
|---|---|---|
| I-266 | 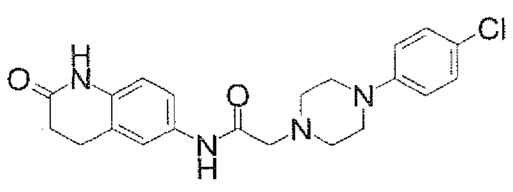 | |
| I-267 | 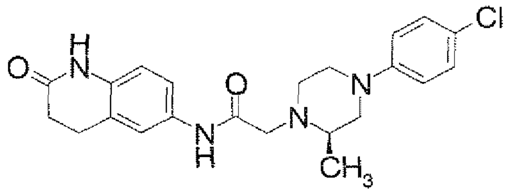 | |

(continued)

| | | |
|---|---|---|
| I-268 | 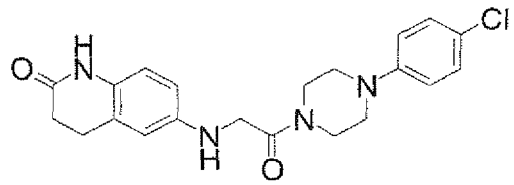 | |
| I-269 | 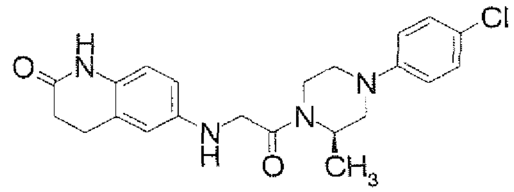 | |
| I-270 | 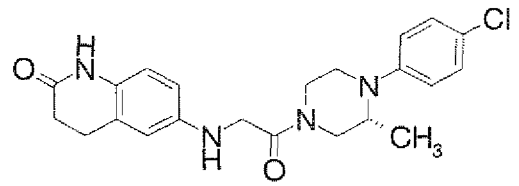 | |
| I-271 | 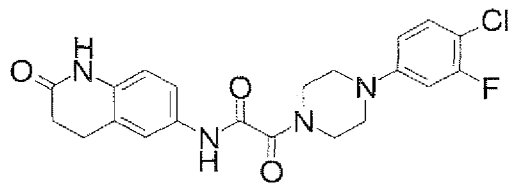 | |
| I-272 | 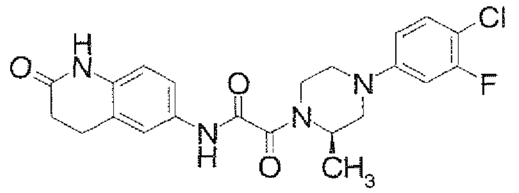 | |

[Table 43]

| | | |
|---|---|---|
| I-273 | 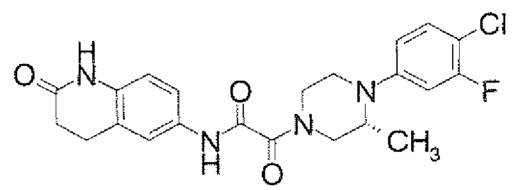 | |
| I-274 | 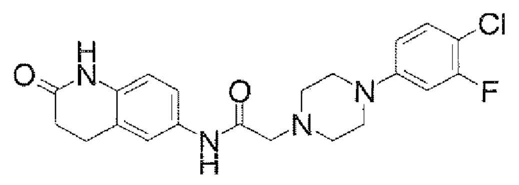 | |

(continued)

| | | |
|---|---|---|
| **I-275** | 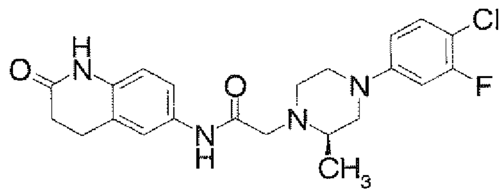 | |
| **I-276** | 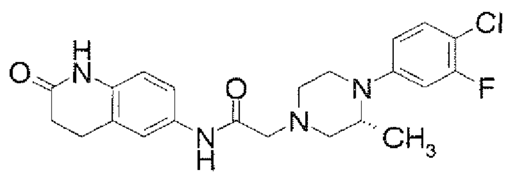 | |
| **I-277** | 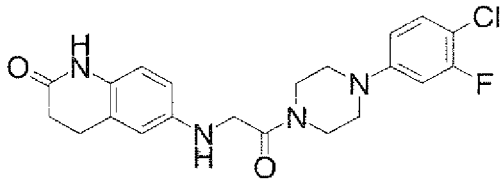 | |
| **I-278** | 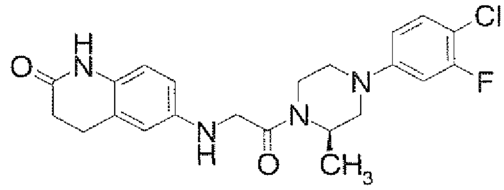 | |
| **I-279** | 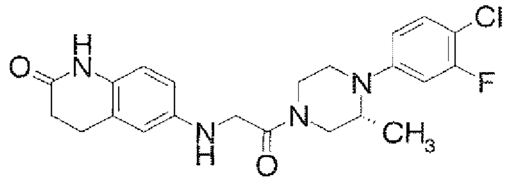 | |

[Table 44]

| | | |
|---|---|---|
| **I-280** | 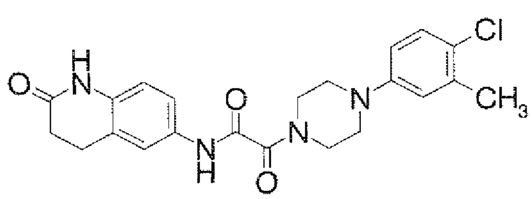 | |
| **I-281** | 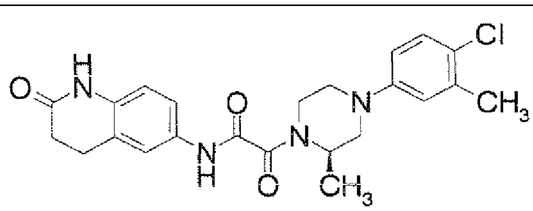 | |

(continued)

| | | |
|---|---|---|
| I-282 | 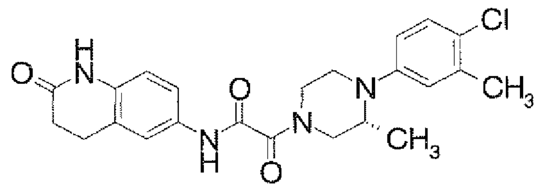 | |
| I-283 | 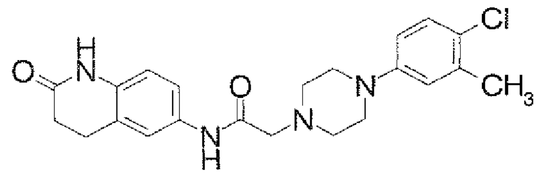 | |
| I-284 | 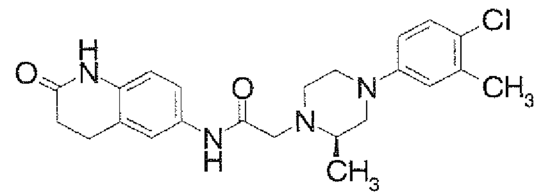 | |
| I-285 | 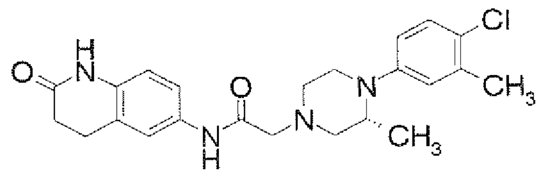 | |
| I-286 | 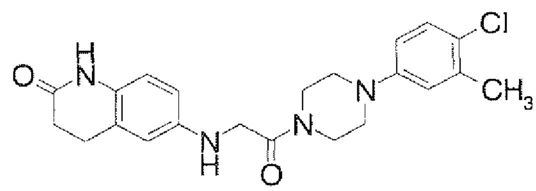 | |

[Table 45]

| | | |
|---|---|---|
| I-287 | 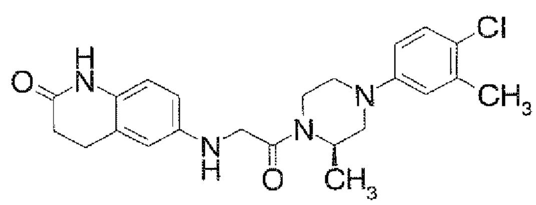 | |
| I-288 | 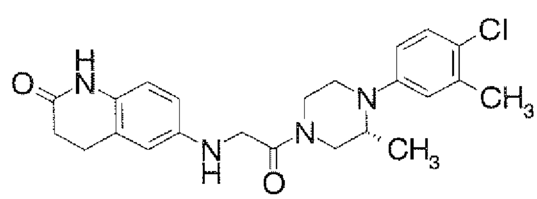 | |

(continued)

| | | |
|---|---|---|
| **I-289** | 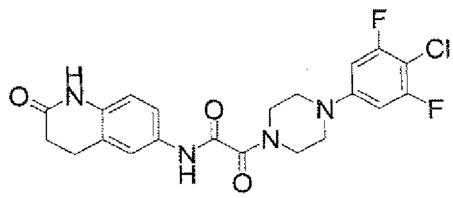 | |
| **I-290** | 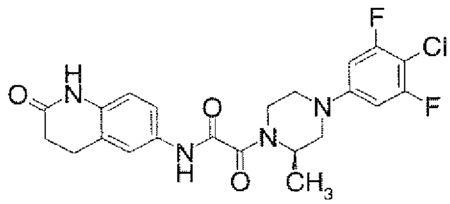 | |
| **I-291** | 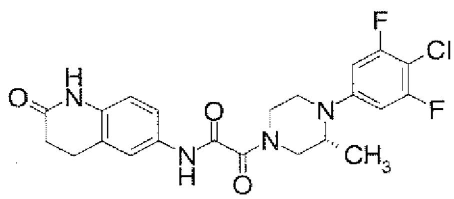 | |
| **I-292** | 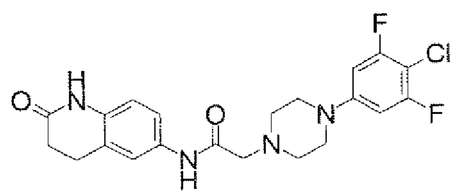 | |
| **I-293** | 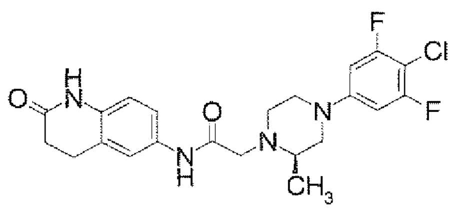 | |

[Table 46]

| | | |
|---|---|---|
| **I-294** | 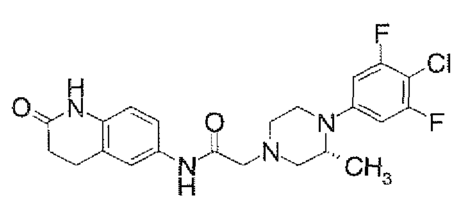 | |
| **I-295** | 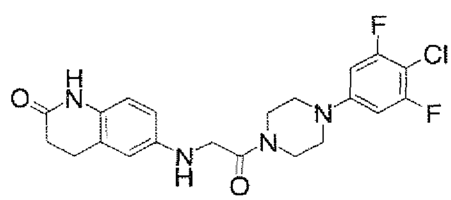 | |

(continued)

| | | |
|---|---|---|
| **I-296** | 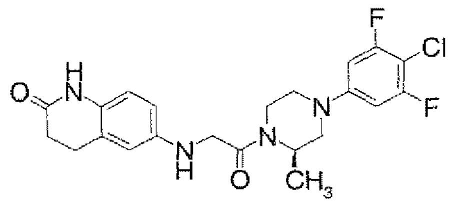 | |
| **I-297** | 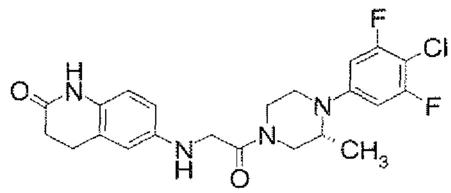 | |
| **I-298** | 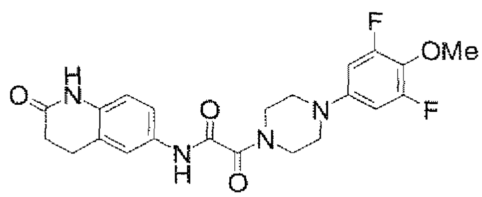 | |
| **I-299** | 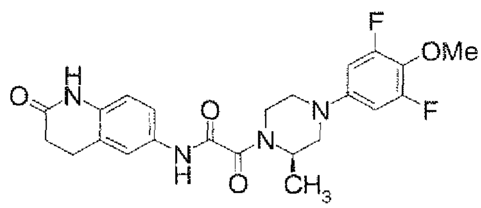 | |
| **I-300** | 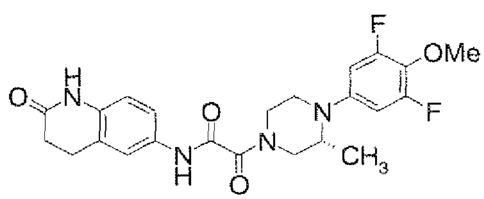 | |

[Table 47]

| | | |
|---|---|---|
| **I-301** | 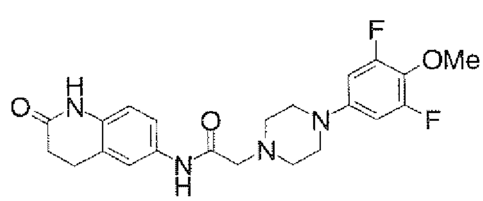 | |
| **I-302** | 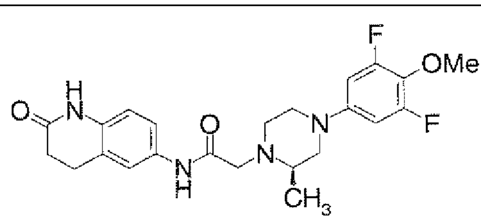 | |

(continued)

| | | |
|---|---|---|
| I-303 | 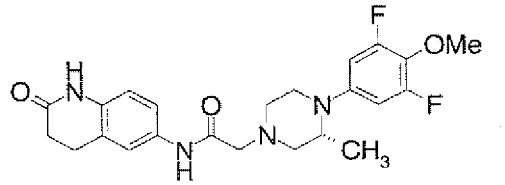 | |
| I-304 | 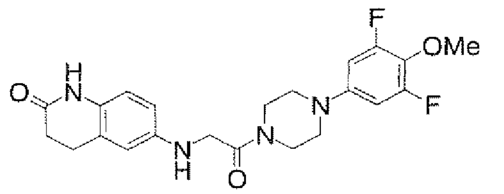 | |
| I-305 | 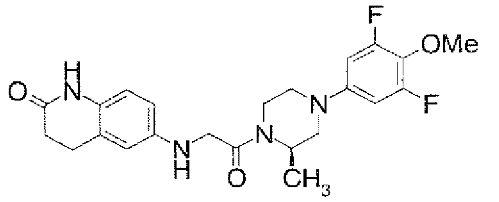 | |
| I-306 | 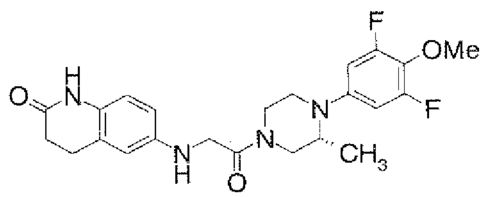 | |
| I-307 | 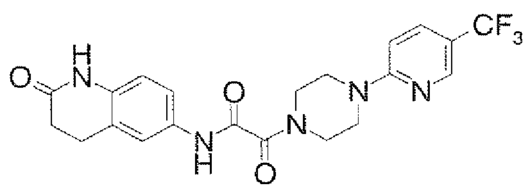 | |

[Table 48]

| | | |
|---|---|---|
| I-308 | 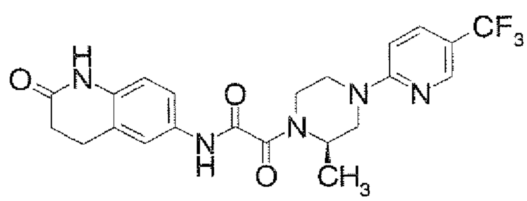 | |
| I-309 | 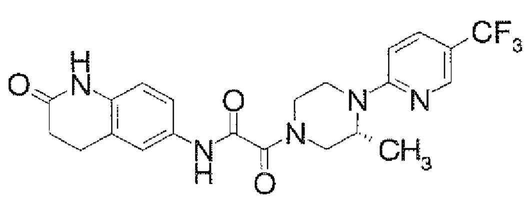 | |

(continued)

| | | |
|---|---|---|
| I-310 | 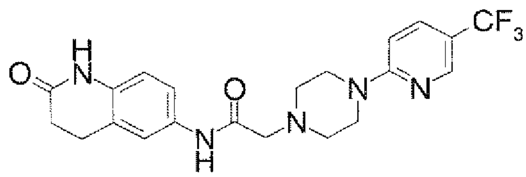 | |
| I-311 | 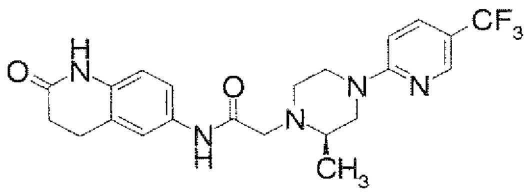 | |
| I-312 | 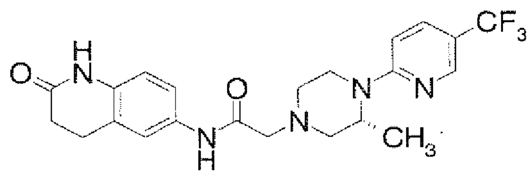 | |
| I-313 | 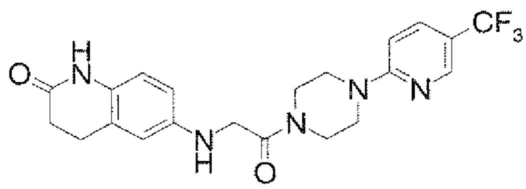 | |
| I-314 | 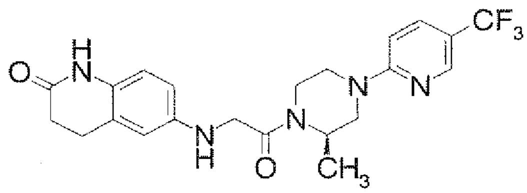 | |

[Table 49]

| | | |
|---|---|---|
| I-315 | 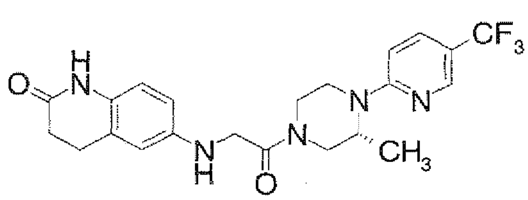 | |
| I-316 | 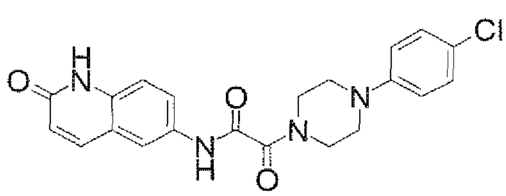 | |

(continued)

| | | |
|---|---|---|
| I-317 | 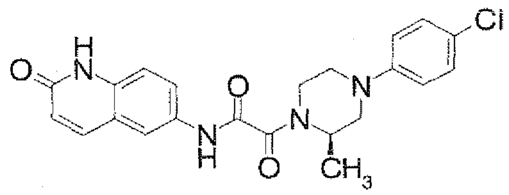 | |
| I-318 | 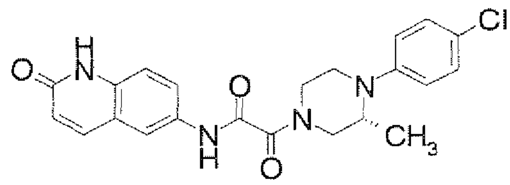 | |
| I-319 | 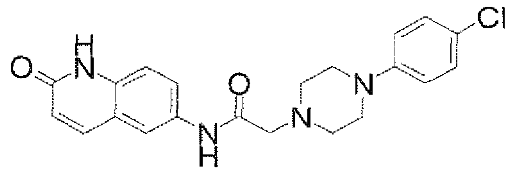 | |
| I-320 | 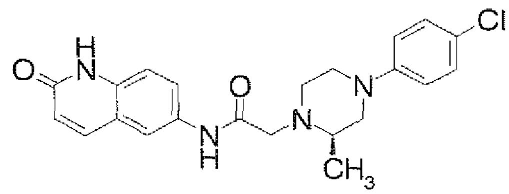 | |
| I-321 | 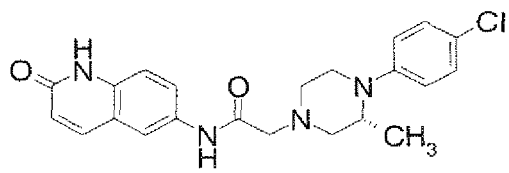 | |

[Table 50]

| | | |
|---|---|---|
| I-322 | 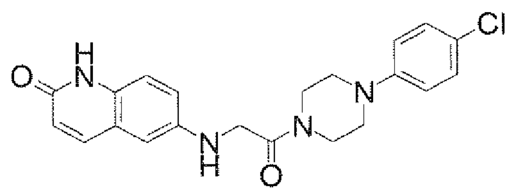 | |
| I-323 | 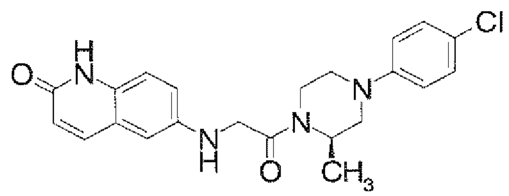 | |

(continued)

| | | |
|---|---|---|
| I-324 | 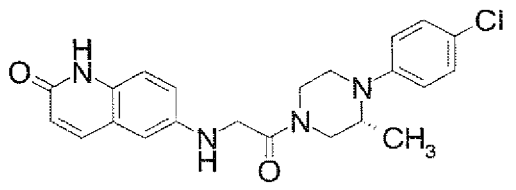 | |
| I-325 | 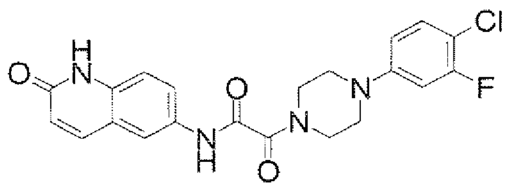 | |
| I-326 | 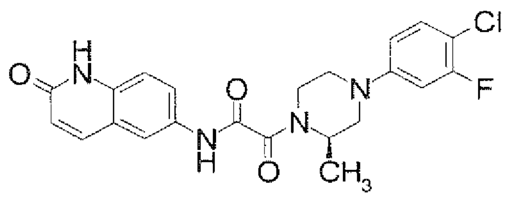 | |
| I-327 | 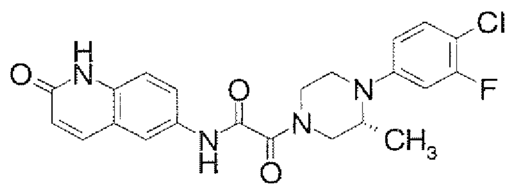 | |
| I-328 | 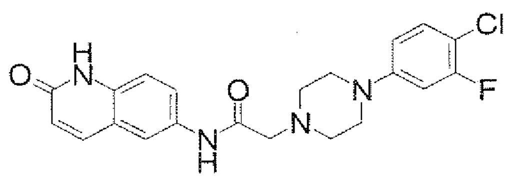 | |

[Table 51]

| | | |
|---|---|---|
| I-329 | 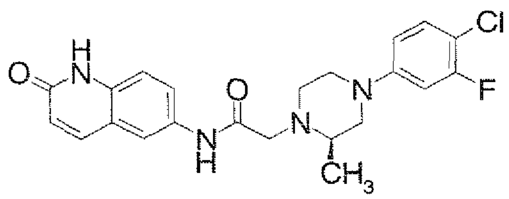 | |
| I-330 | 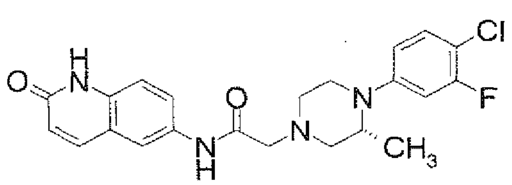 | |

(continued)

| | | |
|---|---|---|
| I-331 | 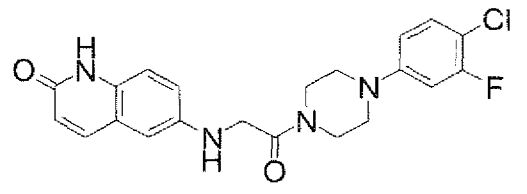 | |
| I-332 | 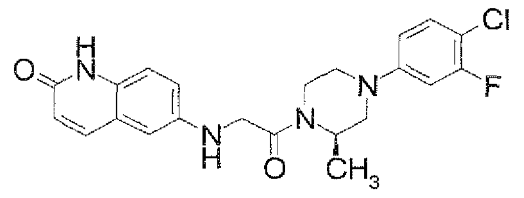 | |
| I-333 | 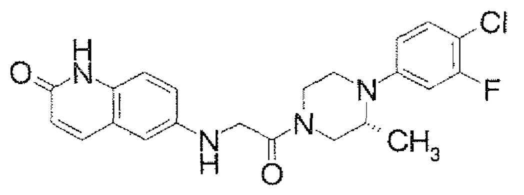 | |
| I-334 | 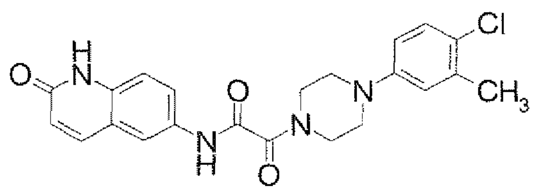 | |
| I-335 | 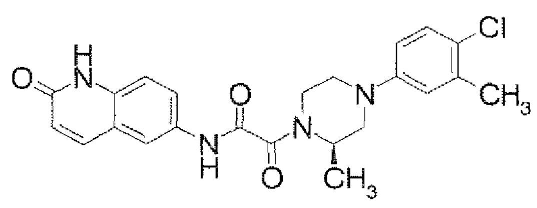 | |

[Table 52]

| | | |
|---|---|---|
| I-336 | 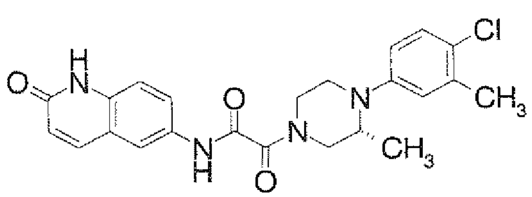 | |
| I-337 | 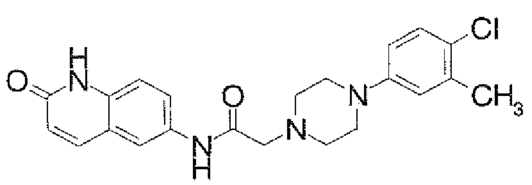 | |

(continued)

| | | |
|---|---|---|
| I-338 | 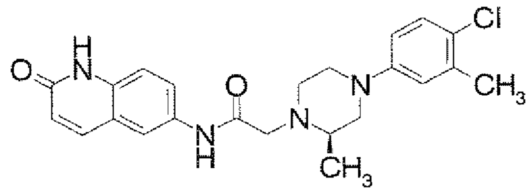 | |
| I-339 | 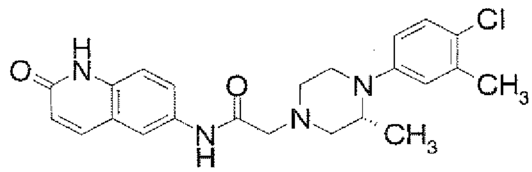 | |
| I-340 | 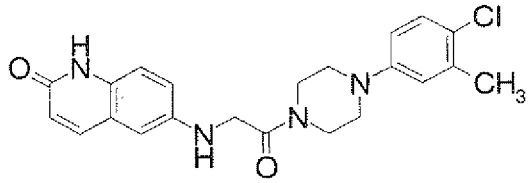 | |
| I-341 | 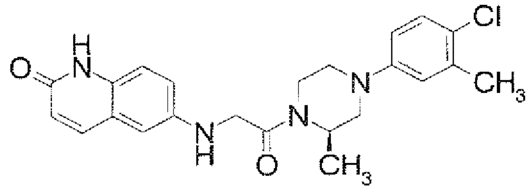 | |
| I-342 | 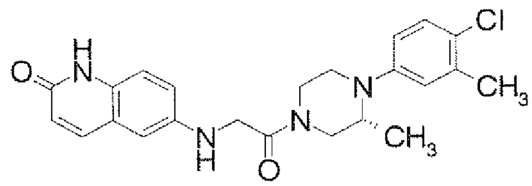 | |

[Table 53]

| | | |
|---|---|---|
| I-343 | 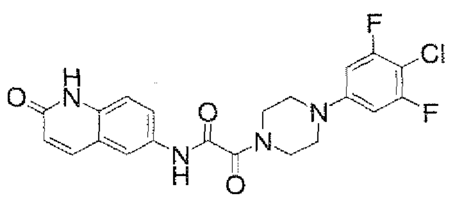 | |
| I-344 | 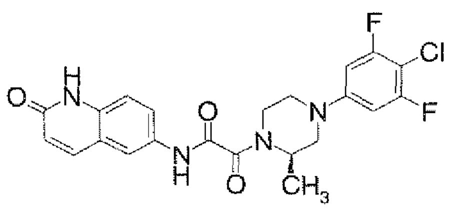 | |

(continued)

| | | |
|---|---|---|
| I-345 | 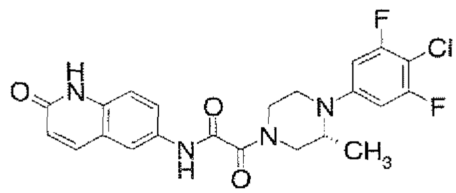 | |
| I-346 | 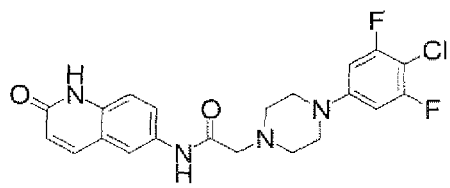 | |
| I-347 | 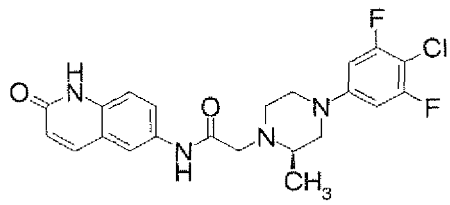 | |
| I-348 | 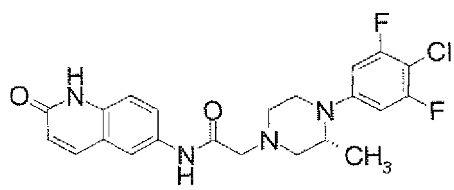 | |
| I-349 | 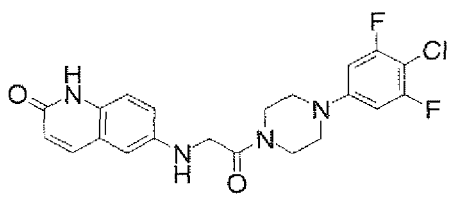 | |

[Table 54]

| | | |
|---|---|---|
| I-350 | 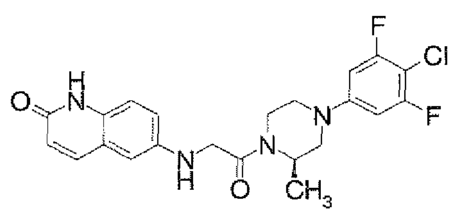 | |
| I-351 | 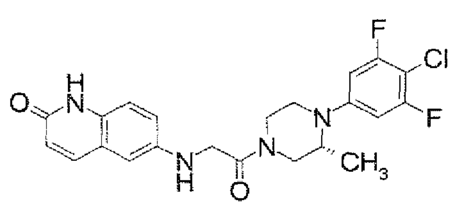 | |

(continued)

| | | |
|---|---|---|
| I-352 | 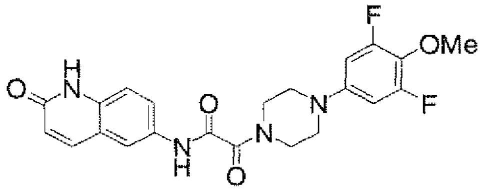 | |
| I-353 | 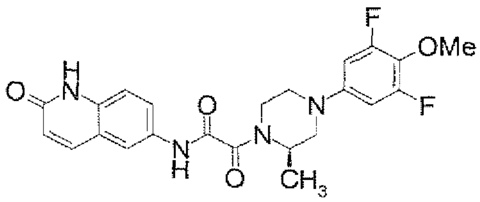 | |
| I-354 | 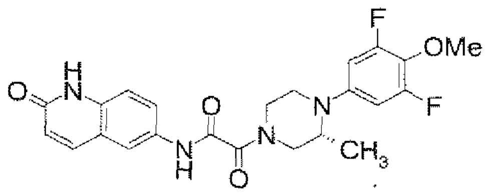 | |
| I-355 | 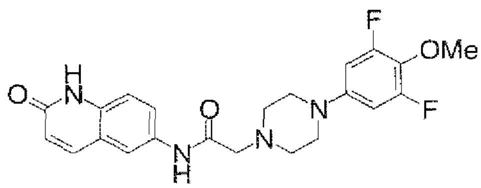 | |
| I-356 | 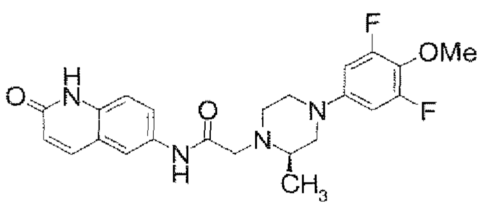 | |

[Table 55]

| | | |
|---|---|---|
| I-357 | 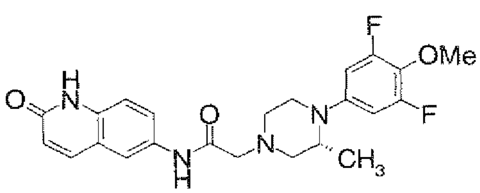 | |
| I-358 | 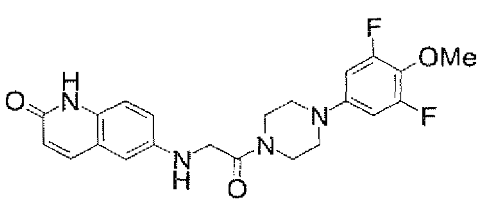 | |

(continued)

| | | |
|---|---|---|
| I-359 | 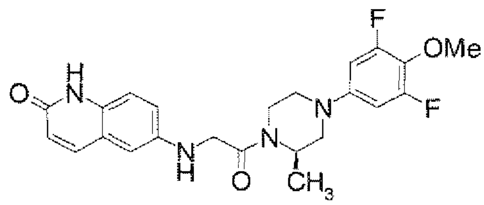 | |
| I-360 | 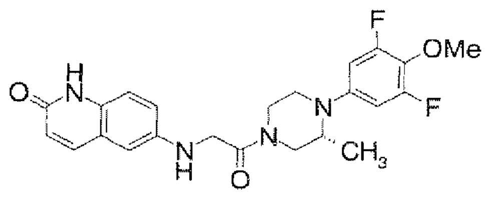 | |
| I-361 | 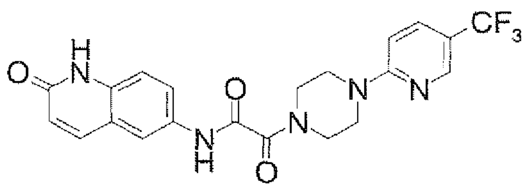 | |
| I-362 | 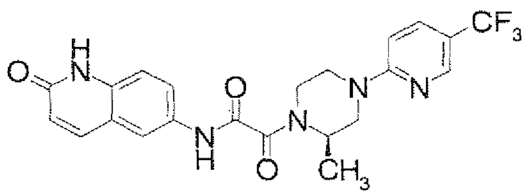 | |
| I-363 | 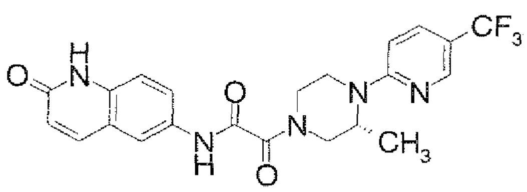 | |

[Table 56]

| | | |
|---|---|---|
| I-364 | 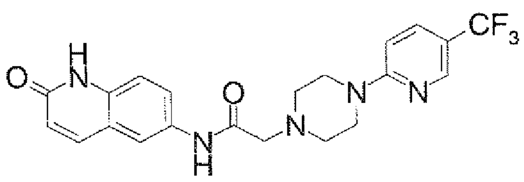 | |
| I-365 | 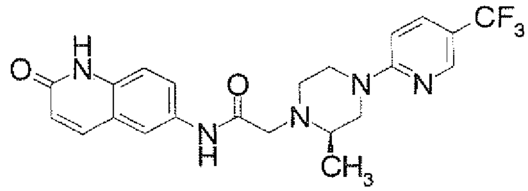 | |

(continued)

| | | |
|---|---|---|
| **I-366** | 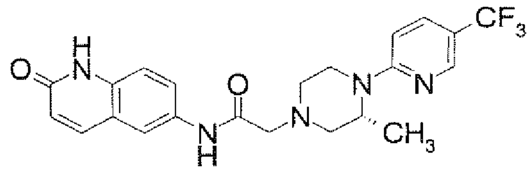 | |
| **I-367** | 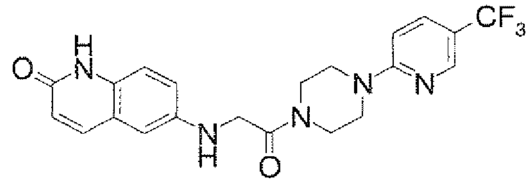 | |
| **I-368** | 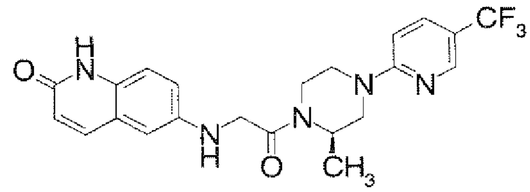 | |
| **I-369** | 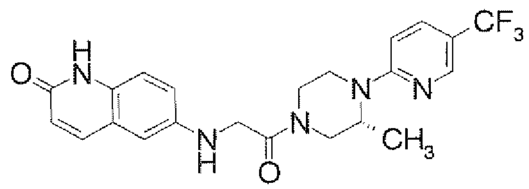 | |
| **I-370** | 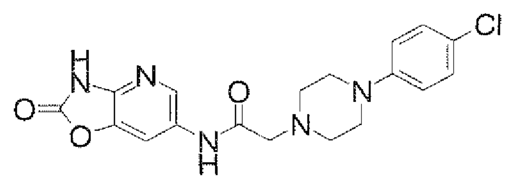 | |

[Table 57]

| | | |
|---|---|---|
| **I-371** | 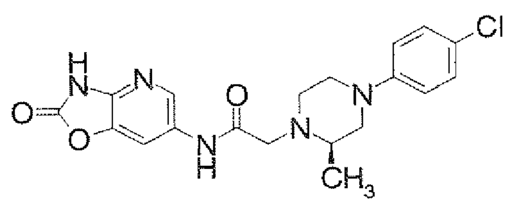 | |
| **I-372** | 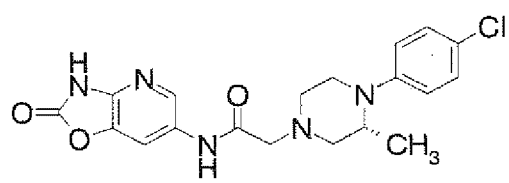 | |

(continued)

| | | |
|---|---|---|
| I-373 | 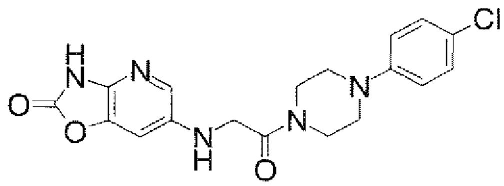 | |
| I-374 | 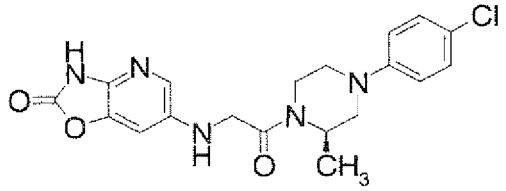 | |
| I-375 | 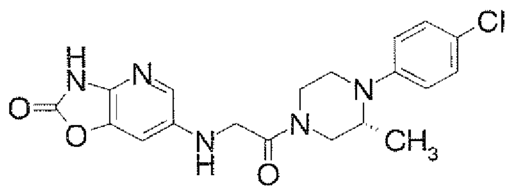 | |
| I-376 | 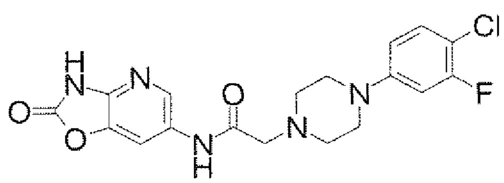 | |
| I-377 | 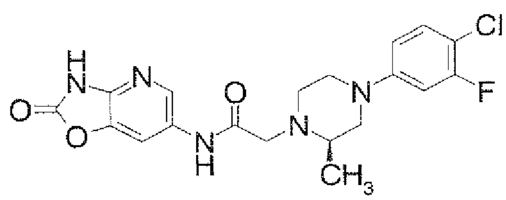 | |

[Table 58]

| | | |
|---|---|---|
| I-378 | 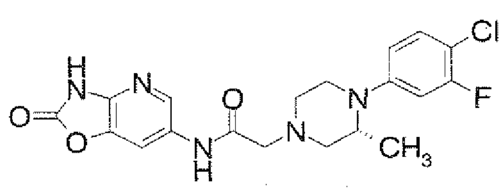 | |
| I-379 | 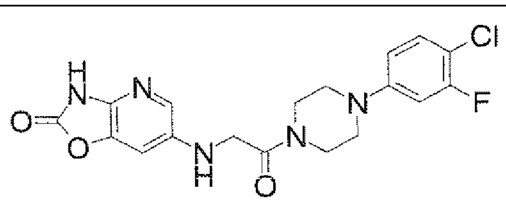 | |

(continued)

| | | |
|---|---|---|
| I-380 | 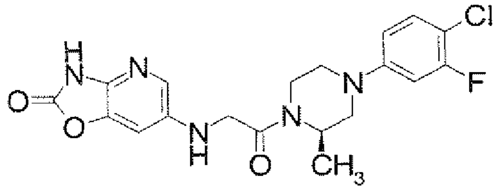 | |
| I-381 | 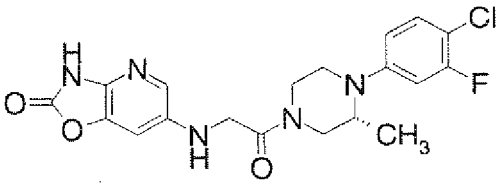 | |
| I-382 | 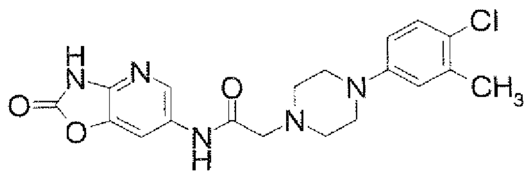 | |
| I-383 | 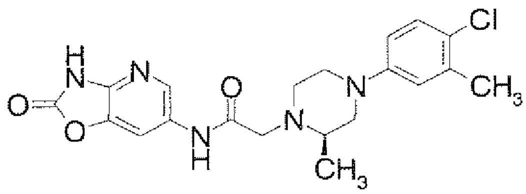 | |
| I-384 | 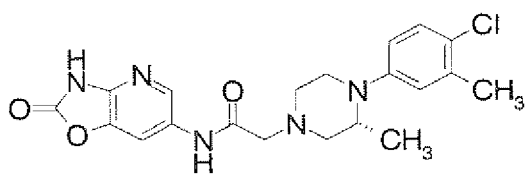 | |

[Table 59]

| | | |
|---|---|---|
| I-385 | 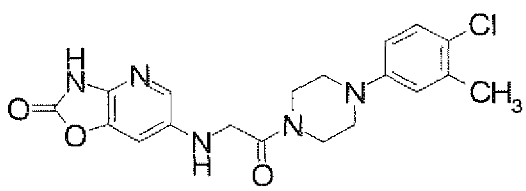 | |
| I-386 | 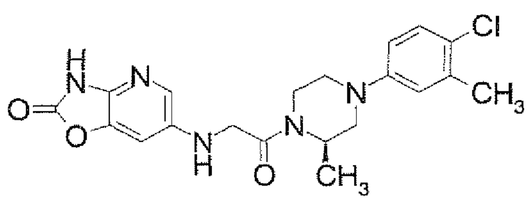 | |

(continued)

| | | |
|---|---|---|
| **I-387** | 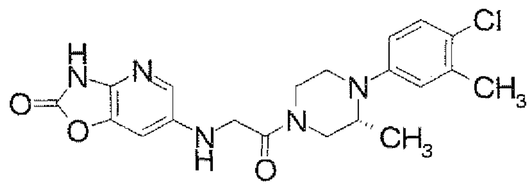 | |
| **I-388** | 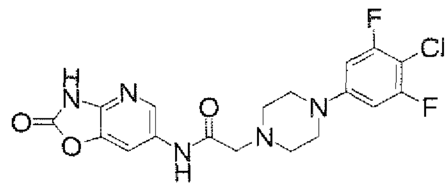 | |
| **I-389** | 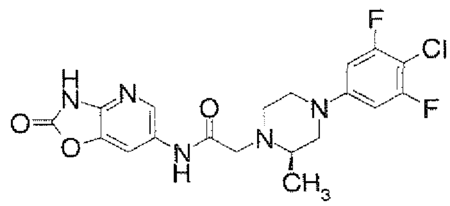 | |
| **I-390** | 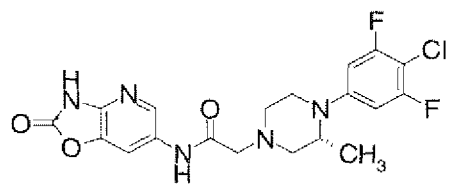 | |
| **I-391** | 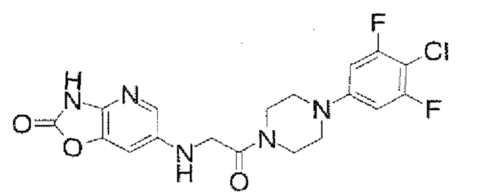 | |

[Table 60]

| | | |
|---|---|---|
| **I-392** | 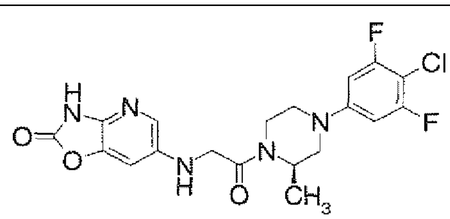 | |
| **I-393** | 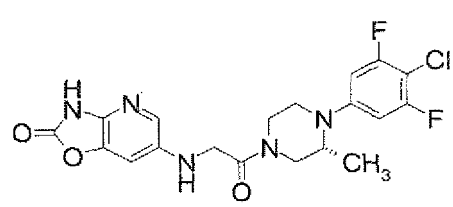 | |

(continued)

| | | |
|---|---|---|
| I-394 | 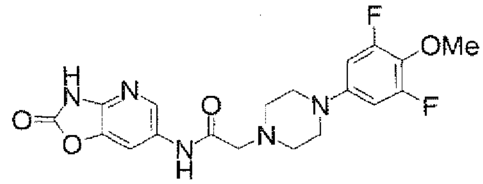 | |
| I-395 | 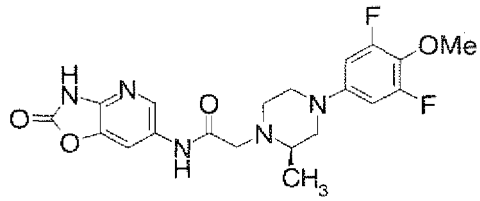 | |
| I-396 | 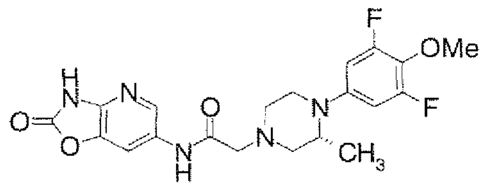 | |
| I-397 | 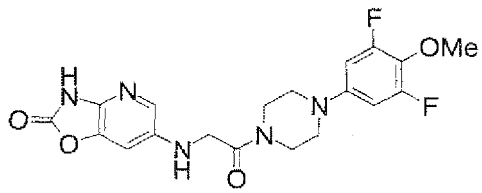 | |
| I-398 | 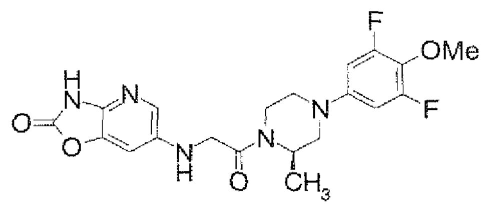 | |

[Table 61]

| | | |
|---|---|---|
| I-399 | 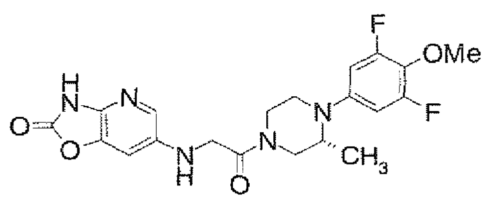 | |
| I-400 | 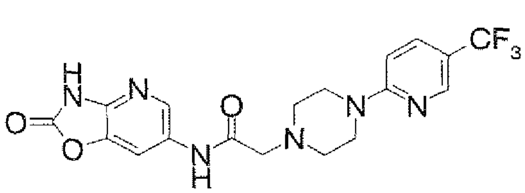 | |

(continued)

| | | |
|---|---|---|
| **I-401** | 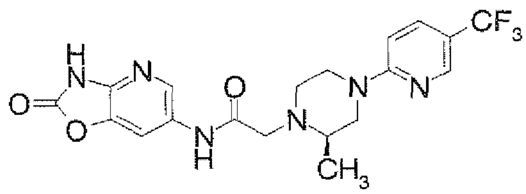 | |
| **I-402** | 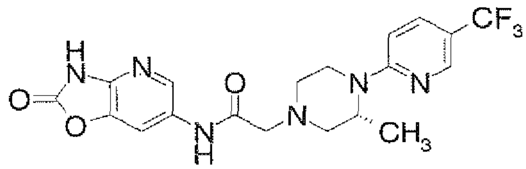 | |
| **I-403** | 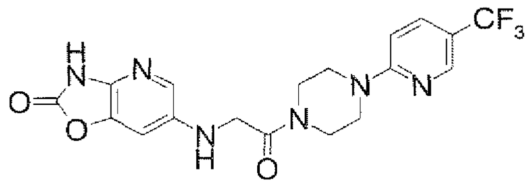 | |
| **I-404** | 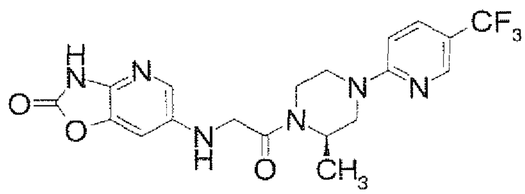 | |
| **I-405** | 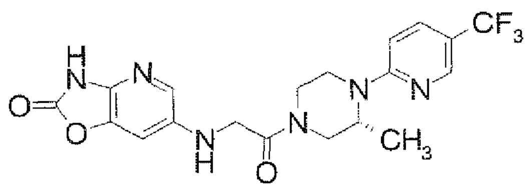 | |

[Table 62]

| | | |
|---|---|---|
| **I-406** | 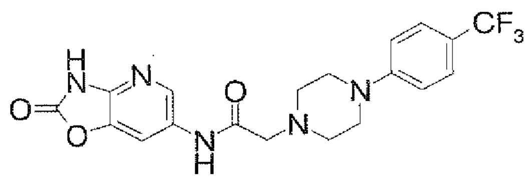 | |
| **I-407** | 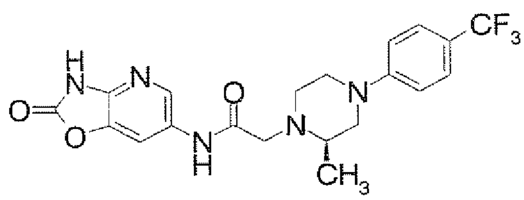 | |

(continued)

| | | |
|---|---|---|
| **I-408** | 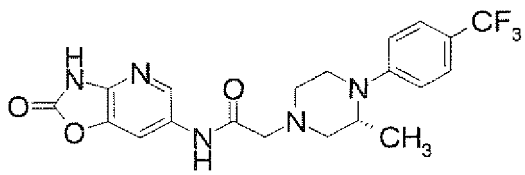 | |
| **I-409** | 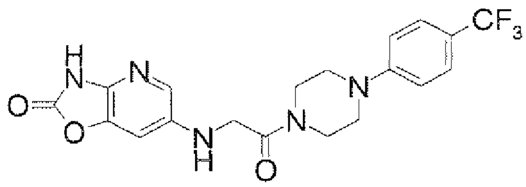 | |
| **I-410** | 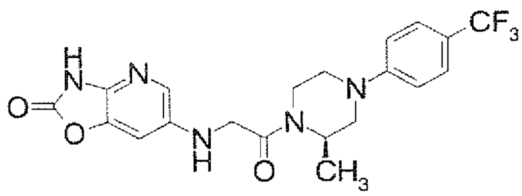 | |
| **I-411** | 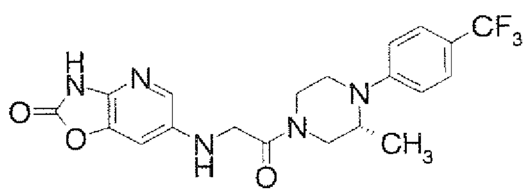 | |
| **I-412** | 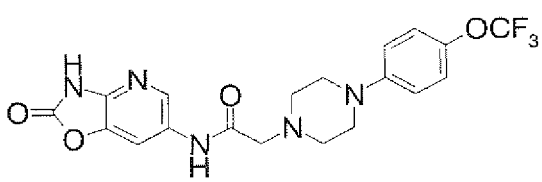 | |

[Table 63]

| | | |
|---|---|---|
| **I-413** | 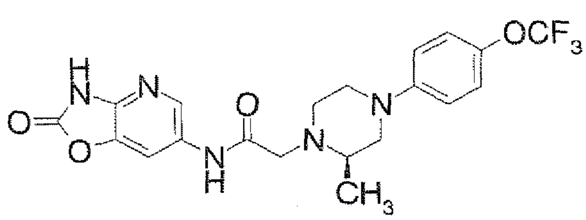 | |
| **I-414** | 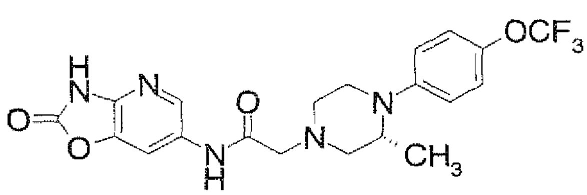 | |

(continued)

| | | |
|---|---|---|
| **I-415** | 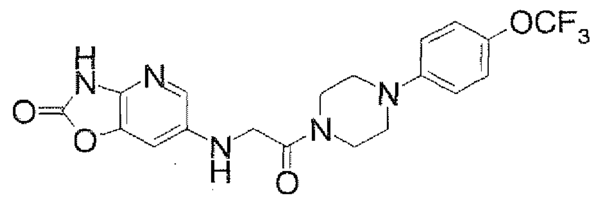 | |
| **I-416** | 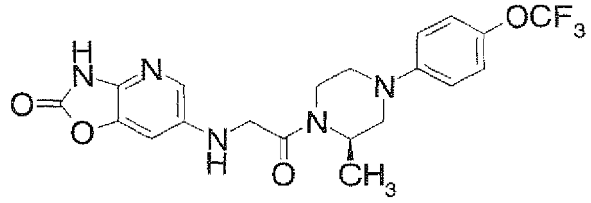 | |
| **I-417** | 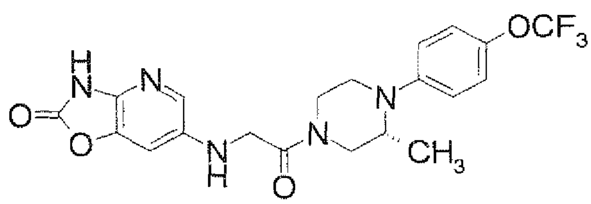 | |

Test Example 1 Experiment of binding to NMDA receptor (NR1/NR2B receptor)

Using Ifenprodil which is a NR1/NR2B subtype receptor-specific antagonist as a ligand, an experiment of receptor competition with a test compound was performed.

A male Slc:Wistar rat was used as an animal, a decapitated brain was isolated, and cerebral cortex was fractionated. The cerebral cortex was homogenized with a 20-fold amount of an ice-cooled 50 mM Tris / HCl buffer (pH 7.4), and centrifuged at 4˚C and 27,500 × g for 10 minutes. The resulting precipitate was suspended in the same buffer, and the suspension was centrifuged again. This procedure was repeated three times, and the resulting precipitate was suspended in a buffer, and stored at -80˚C. Immediately before an experiment, the frozen suspension was thawed at room temperature, and centrifuged at 4˚C and 27,500 × g for 10 minutes, and the resulting precipitate was suspended in a buffer. The suspension was further diluted 10-fold with a buffer, and this was used as a membrane specimen in an experiment.

In a binding experiment, to 470 μl of the membrane specimen were added 10 μl of different concentrations of a test compound, 10 μl of a labeled ligand [3H]-Ifenprodil and 10 μl of GBR-12909, and this was incubated at an ice temperature for 120 minutes. A concentration of [3H]-Ifenprodil of the labeled ligand was finally 5 nM, and a concentration of GBR-12909 was finally 3 μM. For measuring a total binding amount, DMSO as a solvent was used and, for measuring a non-specific binding amount, 100 μM of Ifenprodil was used. In addition, GBR-12909 was added in order to block binding of [3H]-Ifenprodil to a non-polyamine-sensitive site. After incubation, a bound body and a free body were separated using a Whatman GF/C filter (manufactured by Whatman), and the filter was washed with 2.5 ml of an ice-cooled buffer four times. The filter was immersed in a liquid scintillation (Clear-sol I, manufactured by Nacalai tesque) in a vial bottle, and radioactivity (dpm) was measured with a liquid scintillation counter. From a measured value, a binding inhibition rate (%) was obtained by the following equation, and a dose at which binding is inhibited 50% ($IC_{50}$) was calculated. An $IC_{50}$ value of a test substance is shown in Table 64. A formula of GBR-12909 (Vanoxerine) is shown below.

[Chemical formula 43]

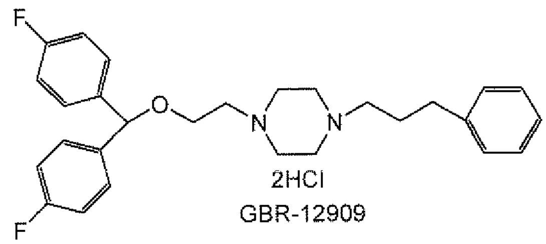

2HCl
GBR-12909

Binding inhibition rate (%) = 100 - [(binding amount in presence of test compound - non-specific amount)/(total binding amount - non-specific binding amount)] × 100

[Table 64]

| Compound No. | NR2B (IC50 μM) | Compound No. | NR2B (IC50 μM) |
|---|---|---|---|
| **I-1** | **0.149** | **I-37** | **0.029** |
| **I-6** | **0.026** | **I-38** | **0.041** |
| **I-9** | **0.031** | **I-39** | **0.080** |
| **I-13** | **0.114** | **I-40** | **0.040** |
| **I-15** | **0.039** | **I-41** | **0.067** |
| **I-16** | **0.072** | **I-43** | **0.065** |
| **I-17** | **0.024** | **I-50** | **0.064** |
| **I-18** | **0.107** | **I-51** | **0.090** |
| **I-22** | **0.085** | **I-52** | **0.081** |
| **I-24** | **0.105** | **I-53** | **0.134** |
| **I-26** | **0.014** | **I-55** | **0.033** |
| **I-27** | **0.035** | **I-56** | **0.044** |
| **I-28** | **0.068** | **I-57** | **0.056** |
| **I-29** | **0.129** | **I-59** | **0.042** |
| **I-33** | **0.059** | **I-60** | **0.080** |
| **I-34** | **0.099** | **I-65** | **0.026** |
| **I-35** | **0.020** | **I-66** | **0.042** |
| **I-36** | **0.071** | | |

From the above results, it was revealed that the present compound exhibits strong binding property on the NR1/NR2B subtype receptor. Test Example 2 Expression of NMDA receptor and measurement of Ca ion flow in amount A complementary DNA (cDNA) of a mouse NMDA receptor subunit was transiently introduced into HEK293 cells and, after one day from introduction, change in a glutamic acid/glycine-induced intracellular Ca amount was measured using

a Ca ion reactive fluorescent dye.

HEK293 cells were cultured and passaged using a modified Dulbecco's Eagle medium (DMEM, low glucose).

20,000/well of HEK293 cells were seeded on a 96-will plate, a NR1 subunit and a NR2B subunit of a NMDA receptor incorporated into pcDNA 3.1 plasmid were transiently introduced into cells, and co-expression of subunits was performed. An introduction amount of a DNA was such that the NR1 subunit was 0.025 μg, and the NR2B subunit was 0.075 μg per well. For cells after introduction, cell death was inhibited using 50 μM of a NMDA receptor antagonist MK-801.

For adjusting a test compound and washing cells, Krebs Ringer Hepes buffer (KRH, Ca: 5 mM) was used.

One day after introduction, the NMDA receptor antagonist MK-801 was washed off using the KRH buffer, and a Ca ion indicative fluorescent dye Fluo-3/AM was made to be taken into cells. Flow in of a Ca ion was induced with glutamic acid 20 μM/glycine 2 μM. Change in a fluorescence amount due to intracellular Ca ion flow in was measured at excitation 480 nm using a fluorescence imaging system FDSS3000.

Usually, if the test compound exhibits antagonism of the NMDA receptor, flow in of a Ca ion into cells is reduced, and a fluorescence amount is reduced.

From a measured value of the test compound, a Ca ion flow in inhibitory rate (%) was obtained by the following equation, and a dose at which flow in is 50% inhibited ($IC_{50}$) was calculated. An $IC_{50}$ value of the test substance is shown in Table 65.

Ca ion flow in inhibitory rate (%) = 100 - [(fluorescence amount in presence of test compound - background fluorescence amount)/(total fluorescence amount - background fluorescence amount)] × 100

[Table 65]

| Compound No. | Ca2+ IC50 (μM) | Compound No. | Ca2+ IC50 (μM) |
|---|---|---|---|
| **I-1** | **0.049** | **I-33** | **0.026** |
| **I-6** | **0.020** | **I-34** | **0.032** |
| **I-7** | **0.089** | **I-35** | **0.009** |
| **I-9** | **0.007** | **I-37** | **0.009** |
| **I-12** | **0.108** | **I-38** | **0.007** |
| **I-13** | **0.014** | **I-39** | **0.009** |
| **I-14** | **0.048** | **I-40** | **0.014** |
| **I-15** | **0.005** | **I-41** | **0.005** |
| **I-16** | **0.012** | **I-43** | **0.021** |
| **I-17** | **0.003** | **I-45** | **0.057** |
| **I-18** | **0.048** | **I-46** | **0.083** |
| **I-20** | **0.041** | **I-49** | **0.019** |
| **I-21** | **0.047** | **I-50** | **0.005** |
| **I-22** | **0.022** | **I-51** | **0.005** |
| **I-23** | **0.022** | **I-52** | **0.008** |
| **I-24** | **0.019** | **I-53** | **0.033** |
| **I-25** | **0.009** | **I-54** | **0.052** |
| **I-26** | **0.007** | **I-55** | **0.004** |
| **I-27** | **0.002** | **I-56** | **0.012** |

(continued)

| Compound No. | Ca2+ IC50 (μM) | Compound No. | Ca2+ IC50 (μM) |
|---|---|---|---|
| **I-28** | **0.003** | **I-57** | **0.004** |
| **I-29** | **0.009** | **I-59** | **0.006** |
| **I-30** | **0.031** | **I-60** | **0.010** |
| **I-31** | **0.106** | **I-65** | **0.010** |
| **I-32** | **0.105** | **I-66** | **0.003** |

From the above results, it was revealed that the present compound exhibited NMDA receptor antagonism. Test Example 3 Method of confirming analgesic activity by mouse formalin test

A pain act of a mouse with formalin is classified into two phases with time, and a mouse exhibits a pain act called licking and biting acts. At a first phase, during 5 minutes immediately after formalin administration, an acute pain is manifested and, at a second phase, during 20 minutes from 10 to 30 minutes of administration, an inflammatory pain is manifested. In the experiment, a ddY line male mouse (5-week-old) was used. A formalin (2%) solution was subcutaneously administered to a mouse into a right hind leg. The test compound having NMDA receptor antagonism was dissolved in a 0.5% methylcellulose solution, and a different concentration (50 mg/kg or 30mg/kg) was orally administered 60 minutes before formalin administration. During 30 minutes after formalin administration, a pain act time was measured. When an analgesic effect is recognized in the test compound, a pain act time is shortened. A measurement time was substituted into the following equation, and an analgesic rate (%) was calculated.

$$\text{Analgesic rate (\%)} = (1 - \text{pain act time in presence of test compound / pain act time in absence of test compound}) \times 100$$

[Industrial applicability]

The present compound exhibits specific antagonism for a glutamic acid receptor of a central nervous cell, particularly, a NR1/NR2B receptor which is one kind of NMDA receptors, and is useful as an analgesic and/or a neuroprotecting agent having little side effect on motor function (paresis), and mental symptom (mental fragmentation).

## Claims

**1.** A compound represented by the formula (I):

[Chemical formula 1]

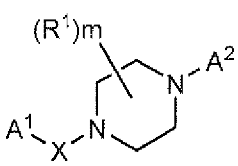

(I)

wherein

$R^1$ is each independently C1-C3 alkyl, halo C1-C3 alkyl, C1-C3 alkoxy, halo C1-C3 alkoxy, hydroxyl, or amino,

two of $R^1$ may be bound to the same carbon atom to form oxo, or

$R^1$ may be bound to different two carbon atoms which are not adjacent to form $-(CH_2)_r-$,

m is an integer of 0 to4,

r is an integer of 1 or 2,

X is $-N(R^4)-C(=O)-C(=O)-$, $-N(R^4)-(CR^5R^6)_p-C(=O)-$, $-N(R^4)-C(=O)-(CR^7R^8)_q-$ or $-C(=O)-N(R^4)-(CR^7R^8)_q-$,
p and q are each independently an integer of 1 to 3,
$R^4$, $R^5$, $R^6$, $R^7$ and $R^8$ are each independently a hydrogen atom or lower alkyl,
$A^1$ is a group represented by the formula:

[Chemical formula 2]

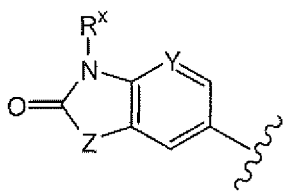
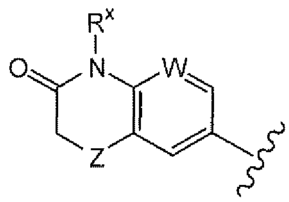
or
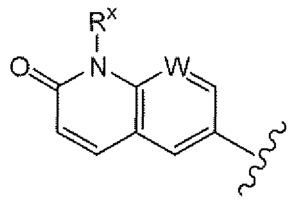

wherein Y and W are each independently CH or N,
Z is an oxygen atom, a sulfur atom, $CH_2$ or $N(-CH_3)$,
$R^X$ is a hydrogen atom, optionally substituted lower alkyl, acyl, lower alkyloxycarbonyl, optionally substituted aralkyloxycarbonyl, lower alkylsulfonyl, arylsulfonyl optionally substituted with lower alkyl, or carbamoyl optionally substituted with lower alkyl,
carbon atoms constituting a ring in the group may be substituted with halogen,
$A^2$ is a group represented by the formula:

[Chemical formula 3]

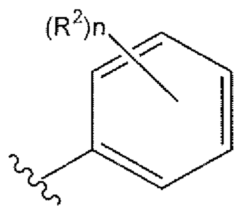
or
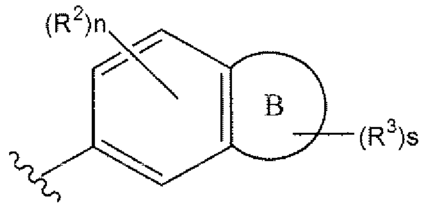

wherein,
ring B is a non-aromatic carbocycle, a non-aromatic heterocycle, an aromatic carbocycle, or an aromatic heterocycle,
$R^2$ and $R^3$ are each independently halogen; cyano; hydroxyl; acyl; acylamino; amino optionally substituted with lower alkyl; optionally substituted lower alkyl; lower alkyloxy; lower alkylsulfonyl; aryl optionally substituted with halogen and/or lower alkyl; heteroaryl optionally substituted with halogen and/or lower alkyl; or aralkyl optionally substituted with halogen and/or lower alkyl; or two of $R^3$ may be substituted at the same carbon atom to form oxo,
n and s are each independently an integer of 0 to 3, provided that when W is CH, X is not $-N(R^4)-C(=O)-(CR^7R^8)_2-$,
or a pharmaceutically acceptable salt thereof, or a solvate thereof.

2. The compound according to claim 1, wherein two of $R^1$ is bound to the same carbon atom to form oxo, or $R^1$ is bound to different two carbon atoms which are not adjacent to form $-CH_2-$ or $-(CH_2)_2-$, or a pharmaceutically acceptable salt thereof, or a solvate thereof.

3. The compound according to claim 1 or 2, wherein $A^2$ is a group represented by the formula:

[Chemical formula 4]

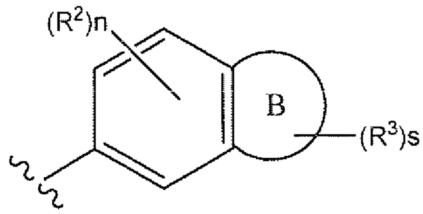

wherein
ring B is a non-aromatic carbocycle, or a non-aromatic heterocycle,
$R^2$, $R^3$, n and s are as defined in claim 1, or a pharmaceutically acceptable salt thereof, or a solvate thereof.

**4.** The compound according to claim 1 or 2, wherein $A^2$ is a group represented by the formula:

[Chemical formula 5]

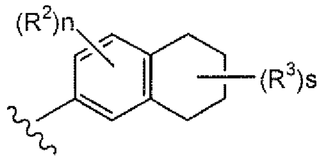

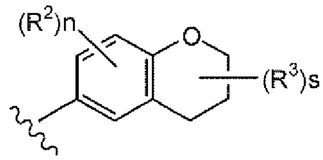

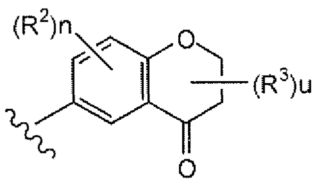

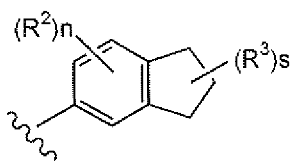

or

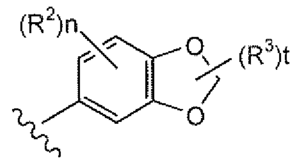

wherein
$R^2$ and $R^3$ are each independently halogen, cyano, hydroxy, acyl, acylamino, amino optionally substituted with lower alkyl, optionally substituted lower alkyl, lower alkyloxy, lower alkylsulfonyl, aryl optionally substituted with halogen and/or lower alkyl, heteroaryl optionally substituted with halogen and/or lower alkyl, or aralkyl optionally substituted with halogen and/or lower alkyl,
n and s are each independently an integer of 0 to 3, t is an integer of 0 to 2, and u is an integer of 0 or 1,
or a pharmaceutically acceptable salt thereof, or a solvate thereof.

**5.** The compound according to claim 1 or 2, wherein $A^2$ is a group represented by the formula:

[Chemical formula 6]

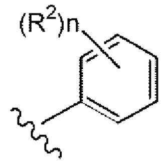

wherein $R^2$ and n are as defined in claim 1,
or a pharmaceutically acceptable salt thereof, or a solvate thereof.

**6.** The compound according to any one of claims 1 to 5, wherein $A^1$ is a group represented by the formula:

[Chemical formula 7]

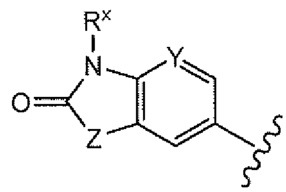

wherein Y, Z and $R^X$ are as defined in claim 1, and carbon atoms constituting a ring may be substituted with halogen,
or a pharmaceutically acceptable salt thereof, or a solvate thereof.

**7.** The compound according to any one of claims 1 to 5, wherein $A^1$ is a group represented by the formula:

[Chemical formula 8]

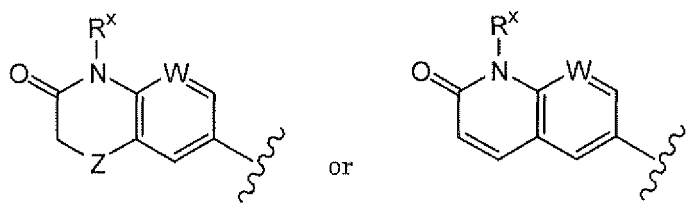

wherein W, Z and $R^X$ are as defined in claim 1, and carbon atoms constituting a ring in the group may be substituted with halogen,
or a pharmaceutically acceptable salt thereof, or a solvate thereof.

**8.** The compound according to any one of claims 1 to 7, wherein Z is an oxygen atom, or a pharmaceutically acceptable salt thereof, or a solvate thereof.

**9.** The compound according to any one of claims 1 to 8, wherein both ofY and W are CH, and $R^X$ is a hydrogen atom, or a pharmaceutically acceptable salt thereof, or a solvate thereof.

**10.** The compound according to any one of claims 1 to 9, wherein X is

- NH-C(=O)-C(=O)-, -NHCH$_2$C(=O)-, -NH-C(=O)-CH$_2$-, -NH-CH(Me)-C(=O)-,
- NH-C(=O)-CH(Me)-, -C(=O)-NH-(CH$_3$)$_2$- or -C(=O)-NH-(CH$_2$)$_3$- wherein Me is methyl, or a pharmaceutically acceptable salt thereof, or a solvate thereof.

**11.** The compound according to any one of claims 1, or 3 to 10, wherein m is 0 or 1, or a pharmaceutically acceptable salt thereof, or a solvate thereof.

**12.** The compound according to any one of claims 1, or 3 to 11, wherein $R^1$ is methyl, or a pharmaceutically acceptable salt thereof, or a solvate thereof.

**13.** A pharmaceutical composition containing the compound as defined in any one of claims 1 to 12, or a pharmaceutically acceptable salt thereof, or a solvate thereof.

**14.** The pharmaceutical composition according to claim 13, which has NMDA receptor antagonism.

**15.** The pharmaceutical composition according to claim 14, which has NR1/NR2B receptor antagonism.

**16.** A method of alleviating a pain, or a method of treating migraine, cerebral stroke, head trauma, Alzheimer's disease, Parkinson's disease, tinnitus, epilepsia, Huntington's disease, a motor disorder or alcohol dependency, comprising administering the compound as defined in any one of claims 1 to 12, or a pharmaceutically acceptable salt, or a solvate thereof.

**17.** Use of the compound as defined in any one of claims 1 to 12, for manufacturing an analgesic, or a therapeutic agent for migraine, cerebral stroke, head trauma, Alzheimer's disease, Parkinson's disease, tinnitus, epilepsia, Huntington's disease, a motor disorder or alcohol dependency.

**18.** The compound as defined in any one of claims 1 to 12 for use as an analgesic, or in therapy of migraine, cerebral stroke, head trauma, Alzheimer's disease, Parkinson's disease, tinnitus, epilepsia, Huntington's disease, a motor disorder or alcohol dependency.

## INTERNATIONAL SEARCH REPORT

International application No.
PCT/JP2008/064731

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D209/34, A61K31/496, A61P9/10, A61P25/02, A61P25/04, A61P25/08, A61P25/14, A61P25/16, A61P25/28, A61P43/00, C07D215/22, C07D235/26, C07D263/58, C07D401/12, C07D401/14, C07D403/12, C07D413/12, C07D413/14,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2007/099828 A1 (Shionogi & Co., Ltd.), 07 September, 2007 (07.09.07), Full text (Family: none) | 1-15,17-18 |
| X<br>A | RN 926544-95-8 REGISTRY<br>ED Entered STN: 15 Mar 2007<br>CN 1-Piperazineacetamide,N-(2,3-dihydro-2-oxo-1Hbenzimidazol-5-yl)-4-(2-nitrophenyl)-(CA INDEX NAME)<br>MF C19 H20 N6 O4<br>SR Chemical Library<br>Supplier: UkrOrgSynthesis<br>retrieval date 05 September, 2008 (05.09.08)<br>URL http://stnweb-japan.cas.org/ | 1,5-6,10-13, 18<br>2-4,7-9, 14-15,17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 September, 2008 (08.09.08) | 22 September, 2008 (22.09.08) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2008/064731

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | RN 924093-64-1 REGISTRY<br>ED Entered STN: 01 Mar 2007<br>CN 1-Piperazineacetamide, N-(2,3-dihydro-2-oxo-1Hbenzimidazol-5-yl)-4-(2-hydroxyphenyl)- (CA INDEX NAME)<br>MF C19 H21 N5 O3<br>SR Chemical Library<br>Supplier: Aurora Fine Chemicals<br>retrieval date 05 September, 2008 (05.09.08)<br>URL http://stnweb-japan.cas.org/ | 1,5-6,10-13, 18<br>2-4,7-9, 14-15,17 |
| X<br><br>A | RN 924018-18-8 REGISTRY<br>ED Entered STN: 01 Mar 2007<br>CN 1-Piperazineacetamide, N-(2,3-dihydro-2-oxo-1Hbenzimidazol-5-yl)-4-(4-methoxyphenyl)- (CA INDEX NAME)<br>MF C20 H23 N5 O3<br>SR Chemical Library<br>Supplier: Aurora Fine Chemicals<br>retrieval date 05 September, 2008 (05.09.08)<br>URL http://stnweb-japan.cas.org/ | 1,5-6,10-13, 18<br>2-4,7-9, 14-15,17 |
| X<br><br>A | RN 874972-49-3 REGISTRY<br>ED Entered STN: 23 Feb 2006<br>CN 1-Piperazineacetamide, N-(2,3-dihydro-2-oxo-1Hbenzimidazol-5-yl)-4-phenyl- (CA INDEX NAME)<br>MF C19 H21 N5 O2<br>SR Chemical Library<br>Supplier: Enamine<br>retrieval date 05 September, 2008 (05.09.08)<br>URL http://stnweb-japan.cas.org/ | 1,5-6,10-13, 18<br>2-4,7-9, 14-15,17 |
| X<br><br>A | RN 830344-42-8 REGISTRY<br>ED Entered STN: 14 Feb 2005<br>CN 1-Piperazineacetamide,4-(5-chloro-2-methylphenyl)-N-(2,3-dihydro-2-oxo-1H-benzimidazol-5-yl)- (CA INDEX NAME)<br>MF C20 H22 Cl N5 O2<br>SR Chemical Library<br>Supplier: ChemBridge Corporation<br>retrieval date 05 September, 2008 (05.09.08)<br>URL http://stnweb-japan.cas.org/ | 1,5-6,10-13, 18<br>2-4,7-9, 14-15,17 |
| A | JP 2005-519953 A (Merck Patent GmbH), 07 July, 2005 (07.07.05),<br>& WO 2003/076420 A1 & EP 1485363 A1<br>& US 2005/124627 A1 & CN 1642927 A | 1-15,17-18 |

Form PCT/ISA/210 (continuation of second sheet) (April 2007)

**INTERNATIONAL SEARCH REPORT**

International application No.
PCT/JP2008/064731

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-515968 A (Richter Gedeon Vegyészeti Gyár Rt), 02 June, 2005 (02.06.05), & WO 2003/010159 A1 & EP 1409477 A1 & US 2004/157886 A1 & KR 2004041152 A & CN 1556805 A | 1-15,17-18 |
| A | JP 56-49364 A (Otsuka Pharmaceutical Co., Ltd.), 02 May, 1981 (02.05.81), (Family: none) | 1-15,17-18 |

Form PCT/ISA/210 (continuation of second sheet) (April 2007)

**INTERNATIONAL SEARCH REPORT**

International application No.
PCT/JP2008/064731

**Box No. II Observations where certain claims were found unsearchable (Continuation of item 2 of first sheet)**

This international search report has not been established in respect of certain claims under Article 17(2)(a) for the following reasons:

1. [X] Claims Nos.: 16
because they relate to subject matter not required to be searched by this Authority, namely:
Claim 16 pertains to a method for treatment of a human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (April 2007)

INTERNATIONAL SEARCH REPORT

International application No.
PCT/JP2008/064731

Continuation of A. CLASSIFICATION OF SUBJECT MATTER
(International Patent Classification (IPC))

*C07D209/34*(2006.01)i, *A61K31/496*(2006.01)i, *A61P9/10*(2006.01)i, *A61P25/02*(2006.01)i, *A61P25/04*(2006.01)i, *A61P25/08*(2006.01)i, *A61P25/14*(2006.01)i, *A61P25/16*(2006.01)i, *A61P25/28*(2006.01)i, *A61P43/00*(2006.01)i, *C07D215/22*(2006.01)i, *C07D235/26*(2006.01)i, *C07D263/58*(2006.01)i, *C07D401/12*(2006.01)i, *C07D401/14*(2006.01)i, *C07D403/12*(2006.01)i, *C07D413/12*(2006.01)i, *C07D413/14*(2006.01)i, *C07D487/08*(2006.01)i, *C07D498/04*(2006.01)i

(According to International Patent Classification (IPC) or to both national classification and IPC)

Continuation of B. FIELDS SEARCHED
Minimum documentation searched (International Patent Classification (IPC))

C07D487/08, C07D498/04

Minimum documentation searched (classification system followed by classification symbols)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02068409 A
- WO 02080928 A
- WO 0240466 A
- JP 11147872 A
- WO 2003076420 A
- WO 2003010159 A
- WO 2006010968 A
- WO 2006010964 A
- WO 2003053366 A
- WO 2002051806 A
- WO 8600899 A
- CH 460016
- CH 460017
- US 3538089 A
- JP 56049363 A
- JP 56049364 A
- US 20050256144 A

### Non-patent literature cited in the description

- *Journal of Heterocyclic Chemistry,* 1995, vol. 32 (1), 1-11
- *Bulletin of the Korean Chemical Society,* 2004, vol. 25 (9), 1326-1330
- *Acta Facultatis Rerum Naturalium Universtatis Comennianae Chimia,* 1985, vol. 33, 137-146
- *Bulletin of the Korean Chemical Society,* 2005, vol. 26 (11), 1757-1760
- **T. W. Green et al.** Protective Groups in Organic Synthesis. John Wiley & Sons, 1991
- *J. Chem. Soc.,* 1921, vol. 119, 1425-1432
- *Khimiya Geterotsiklicheskikh Soedinenii,* 1984, 1035-1038